(19) Europäisches Patentamt
European Patent Office
Office européen des brevets

(11) EP 1 673 092 B1

(12) **EUROPEAN PATENT SPECIFICATION**

(45) Date of publication and mention of the grant of the patent:
**15.08.2007 Bulletin 2007/33**

(21) Application number: **04790649.0**

(22) Date of filing: **18.10.2004**

(51) Int Cl.:
***A61K 31/519*** (2006.01) ***A61K 31/551*** (2006.01)
***C07D 475/08*** (2006.01) ***A61P 37/00*** (2006.01)
***A61P 37/06*** (2006.01) ***A61P 9/00*** (2006.01)
***A61P 25/00*** (2006.01) ***A61P 37/08*** (2006.01)
***A61P 29/00*** (2006.01) ***A61P 31/12*** (2006.01)
***A61P 35/00*** (2006.01)

(86) International application number:
**PCT/EP2004/011836**

(87) International publication number:
**WO 2005/039587 (06.05.2005 Gazette 2005/18)**

(54) **HETEROCYCLE-SUBSTITUTED PTERIDINE DERIVATIVES AND THEIR USE IN THERAPY**

HETEROCYCLUS-SUBSTITUIERTE PTERIDIN-DERIVATE UND IHRE VERWENDUNG IN DER THERAPIE

DERIVES DE PTERIDINE SUBSTITUES PAR DES HETEROCYCLES ET LEUR UTILISATION A DES FINS THERAPEUTIQUES

(84) Designated Contracting States:
**AT BE BG CH CY CZ DE DK EE ES FI FR GB GR HU IE IT LI LU MC NL PL PT RO SE SI SK TR**

(30) Priority: **17.10.2003 GB 0324324**
**22.04.2004 GB 0408955**

(43) Date of publication of application:
**28.06.2006 Bulletin 2006/26**

(73) Proprietor: **4 AZA IP NV**
**3000 Leuven (BE)**

(72) Inventors:
- **WAER, Mark, Jozef, Albert**
  **B-3001 Heverlee (BE)**
- **HERDEWIJN, Piet, André, Maurits, Maria**
  **B-3111 Rotselaar/Wezemaal (BE)**
- **DE JONGHE, Steven, Cesar, Alfons**
  **B-1030 Brussel (BE)**
- **MARCHAND, Arnaud, Didier, Marie**
  **B-3001 Heverlee (BE)**
- **GAO, Ling-Jie**
  **B-3001 Heverlee (BE)**

(74) Representative: **Bird, Ariane et al**
**Bird Goën & Co**
**Klein Dalenstraat 42A**
**3020 Winksele (BE)**

(56) References cited:

| | |
|---|---|
| **EP-A- 0 185 259** | **EP-A- 0 362 645** |
| **EP-A- 0 574 906** | **EP-A- 1 479 682** |
| **WO-A-00/39129** | **WO-A-01/21619** |
| **WO-A-02/32507** | **US-A- 2 940 972** |

- **FROEHLICH, LOTHAR G. ET AL: "Inhibition of Neuronal Nitric Oxide Synthase by 4-Amino Pteridine Derivatives: Structure-Activity Relationship of Antagonists of (6R)-5,6,7,8-Tetrahydrobiopterin Cofactor" JOURNAL OF MEDICINAL CHEMISTRY , 42(20), 4108-4121 CODEN: JMCMAR; ISSN: 0022-2623, 1999, XP002313877**
- **MERZ KARL-HEINZ ET AL: "Synthesis of 7-benzylamino-6-chloro-2-piperazino-4-pyrr olidinopterid ine and novel derivatives free of positional isomers. Potent inhibitors of cAMP-specific phosphodiesterase and of malignant tumor cell growth" JOURNAL OF MEDICINAL CHEMISTRY, vol. 41, no. 24, 19 November 1998 (1998-11-19), pages 4733-4743, XP002313878 ISSN: 0022-2623**
- **WEINSTOCK J ET AL: "PTERIDINES. XII. STRUCTURE-ACTIVITY RELATIONSHIPS OF SOME PTERIDINE DIURETICS" JOURNAL OF MEDICINAL CHEMISTRY, AMERICAN CHEMICAL SOCIETY. WASHINGTON, US, vol. 11, no. 3, 1968, pages 573-579, XP002908101 ISSN: 0022-2623**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- Y. LANDRY, J.-P. GIES: "Pharmacologie. Des cibles vers l'indication thérapeutique. Cours et exercices." 2003, DUNOD , PARIS , XP002313503 page 177, paragraphs 2,3
- NICOLAUS B J R: "Symbiotic Approach to Drug Design" DECISION MAKING IN DRUG RESEARCH, XX, XX, 1983, pages 173-186, XP002197412

## Description

FIELD OF THE INVENTION

The invention relates to a class of novel pteridines. The invention further relates to pharmaceutical compositions including a broad class of pteridines especially for the prevention and/or the treatment of pathologic conditions such as immune and auto-immune disorders, organ and cells transplant rejections, cell proliferative disorders, cardiovascular disorders, allergic conditions, disorders of the central nervous system and viral diseases.

The invention further relates to combined pharmaceutical preparations comprising one or more such pteridines and one or more known immunosuppressant drugs or antineoplastic drugs or anti-viral drugs.

The pharmaceutical composition can be used for the prevention and/or treatment of pathologic conditions such as immune and autoimmune disorders, organ and cells transplant rejections, cell proliferative disorders, cardiovascular disorders, disorders of the central nervous system, allergic conditions and viral diseases, for the treatment of side effects of various chemotherapeutic drugs and/or of irradiation in cancer therapy, for the treatment of septic shock, as well as toxic side effects, disorders and diseases related to or resulting from the exposure of patients to abnormally high levels of tumor necrosis factor-alpha (hereinafter referred as TNF-$\alpha$) in general, and particularly following the administration of TNF-$\alpha$ in cancer treatment in humans, for the treatment of radiotherapy-induced or chemotherapy-induced disorders such as mucositis, secondary myelodysplastic syndromes and radiation-induced graft-versus-host disease, for the prevention and/or the treatment of injuries in cancer patients such as apoptosis, radiation necrosis and nephrotoxicity following the administration of certain chemotherapeutic drugs such as cisplatin in cancer treatment, and for the treatment of cachexia.

BACKGROUND OF THE INVENTION

Several 2,4-diaminopteridine derivatives being substituted in the 6-position and/or the 7-position of the pteridine ring (according to standard atom numbering for said ring) are known in the art, e.g. from various sources of literature including Swiss Patent No. 231,852; British Patent No. 763,044; United States Patents No. 2,512,572; No. 2,581,889; No. 2,665,275; No. 2,667,486; No. 2,940,972; No. 3,081,230 and No. 5,047,405. Some of these substituted 2,4-diaminopteridine derivatives were disclosed in relationship with various medical uses, such as bacterial growth inhibitors, antineoplastic agents, anti-schistosomiasis activity, coronary dilating activity, diuretic and hypotensive activity, and anti-amnesic activity. In particular, U.S. Patent No. 2,940,972 and EP-A-362,645 disclose very specific 2,4-diaminopteridine derivatives being substituted by piperidinyl, morpholinyl or pyrrolidinyl in the 7-position of the pteridine ring.

EP-A-185,259 discloses tri- and tetrasubstituted pteridines wherein the substituent in position 2 of the pteridine ring is N-formylpiperazino; the substituent in position 4 of the pteridine ring is selected from the group consisting of dialkylamino, phenylalkylamino, N-alkyl-phenylalkylamino, pyrrolidino, piperidino, (thio)morpholino, 1-oxidothiomorpholino and 1-oxidothioazolidino; the substituent in position 6 of the pteridine ring is selected from the group consisting of hydrogen, alkyl and phenyl; and the substituent in position 7 of the pteridine ring is selected from the group consisting of (di)alkylamino, phenylalkylamino, N-alkyl-phenylalkylamino, piperidino, (thio)morpholino and 1-oxidothiomorpholino. These com-pounds are suggested for the prophylaxy of thromboembolic disease and arteriosclerosis and for the treatment of tumor growth.

EP-A-574,906 discloses 2,7-diaminopteridines having a *tert*-butoxycarbonylpiperazinyl group in position 4 or in position 6 of the pteridine ring, such compounds being useful for lipid peroxidation inhibition.

Merz et al. in J. Medicinal Chem. (1998) 41:4733-4743 discloses 2-N-acetylpiperazino-4-pyrrolidino-6-chloro-7-benzylaminopteridine being useful for inhibiting cAMP phosphodiesterase and malignant tumor cell growth.

WO 02/32507 discloses a series of 7-aminopteridines wherein the substituent in position 2 of the pteridine ring may be, among others, SR wherein R is alkyl, cycloalkyl, alkenyl or alkynyl; the substituent in position 4 of the pteridine ring may be, among others, $NR^2R^3$ wherein $R^2$ and $R^3$ are each independently selected from the group consisting of hydrogen, alkyl, cycloalkyl, alkenyl and alkynyl, the latter four groups being optionally substituted; and the substituent in position 6 of the pteridine ring is selected from the group consisting of hydrogen, alkyl and phenyl. These compounds are suggested as modulators of chemokine receptors being useful e.g. for the treatment of asthma, rhinitis, rheumatoid arthritis and the like.

Nevertheless, there still is a need in the art for specific and highly therapeutically active compounds, such as drugs for treating immune and autoimmune disorders, organ and cells transplant rejections, cell proliferative disorders, cardiovascular disorders, disorders of the central nervous system, allergic conditions and viral diseases. In particular, there is a need in the art to provide immunosuppressive compounds, antineoplastic drugs and anti-viral drugs which are active in a minor dose in order to replace existing drugs having significant side effects and to decrease treatment costs.

Currently used immunosuppressive drugs include antiproliferative agents, such as methotrexate (a 2,4-diaminopteridine derivative disclosed by U.S. Patent No. 2,512,572), azathioprine, and cyclophosphamide. Since these drugs

affect mitosis and cell division, they have severe toxic effects on normal cells with high turn-over rate such as bone marrow cells and the gastrointestinal tract lining. Accordingly, marrow depression and liver damage are common side effects of these antiproliferative drugs.

Anti-inflammatory compounds used to induce immunosuppression include adrenocortical steroids such as dexamethasone and prednisolone. The common side effects observed with the use of these compounds are frequent infections, abnormal metabolism, hypertension, and diabetes.

Other immunosuppressive compounds currently used to inhibit lymphocyte activation and subsequent proliferation include cyclosporine, tacrolimus and rapamycin. Cyclosporine and its relatives are among the most commonly used immunosuppressant drugs. Cyclosporine is typically used for preventing or treating organ rejection in kidney, liver, heart, pancreas, bone marrow, and heart-lung transplants, as well as for the treatment of autoimmune and inflammatory diseases such as Crohn's disease, aplastic anemia, multiple-sclerosis, myasthenia gravis, uveitis, biliary cirrhosis, etc. However, cyclosporines suffer from a small therapeutic dose window and severe toxic effects including nephrotoxicity, hepatotoxicity, hypertension, hirsutism, cancer, and neurotoxicity.

Additionally, monoclonal antibodies with immunosuppressant properties, such as OKT3, have been used to prevent and/or treat graft rejection. Introduction of such monoclonal antibodies into a patient, as with many biological materials, induces several side-effects, such as dyspnea. Within the context of many life-threatening diseases, organ transplantation is considered a standard treatment and, in many cases, the only alternative to death. The immune response to foreign cell surface antigens on the graft, encoded by the major histocompatibility complex (hereinafter referred as MHC) and present on all cells, generally precludes successful transplantation of tissues and organs unless the transplant tissues come from a compatible donor and the normal immune response is suppressed. Other than identical twins, the best compatibility and thus, long term rates of engraftment, are achieved using MHC identical sibling donors or MHC identical unrelated cadaver donors. However, such ideal matches are difficult to achieve. Further, with the increasing need of donor organs an increasing shortage of transplanted organs currently exists. Accordingly, xenotransplantation has emerged as an area of intensive study, but faces many hurdles with regard to rejection within the recipient organism.

The host response to an organ allograft involves a complex series of cellular interactions among T and B lymphocytes as well as macrophages or dendritic cells that recognize and are activated by foreign antigen. Co-stimulatory factors, primarily cytokines, and specific cell-cell interactions, provided by activated accessory cells such as macrophages or dendritic cells are essential for T-cell proliferation. These macrophages and dendritic cells either directly adhere to T-cells through specific adhesion proteins or secrete cytokines that stimulate T-cells, such as IL-12 and IL-15. Accessory cell-derived co-stimulatory signals stimulate activation of interleukin-2 (IL-2) gene transcription and expression of high affinity IL-2 receptors in T-cells. IL-2 is secreted by T lymphocytes upon antigen stimulation and is required for normal immune responsiveness. IL-2 stimulates lymphoid cells to proliferate and differentiate by binding to IL-2 specific cell surface receptors (IL-2R). IL-2 also initiates helper T-cell activation of cytotoxic T-cells and stimulates secretion of interferon-y which in turn activates cytodestructive properties of macrophages. Furthermore, IFN-y and IL-4 are also important activators of MHC class II expression in the transplanted organ, thereby further expanding the rejection cascade by enhancing the immunogenicity of the grafted organ The current model of a T-cell mediated response suggests that T-cells are primed in the T-cell zone of secondary lymphoid organs, primarily by dendritic cells. The initial interaction requires cell to cell contact between antigen-loaded MHC molecules on antigen-presenting cells (hereinafter referred as APC) and the T-cell receptor/CD3 complex on T-cells. Engagement of the TCR/CD3 complex induces CD154 expression predominantly on CD4 T-cells that in turn activate the APC through CD40 engagement, leading to improved antigen presentation. This is caused partly by upregulation of CD80 and CD86 expression on the APC, both of which are ligands for the important CD28 co-stimulatory molecule on T-cells. However, engagement of CD40 also leads to prolonged surface expression of MHC-antigen complexes, expression of ligands for 4-1BB and OX-40 (potent co-stimulatory molecules expressed on activated T-cells). Furthermore, CD40 engagement leads to secretion of various cytokines (e.g., IL-12, IL-15, TNF-$\alpha$, IL-1, IL-6, and IL-8) and chemokines, all of which have important effects on both APC and T-cell activation and maturation. Similar mechanisms are involved in the development of auto-immune disease, such as type I diabetes. In humans and non-obese diabetic mice, insulin-dependent diabetes mellitus results from a spontaneous T-cell dependent auto-immune destruction of insulin-producing pancreatic .beta, cells that intensifies with age. The process is preceded by infiltration of the islets with mononuclear cells (insulitis), primarily composed of T lymphocytes. A delicate balance between auto-aggressive T-cells and suppressor-type immune phenomena determines whether expression of auto-immunity is limited to insulitis or not. Therapeutic strategies that target T-cells have been successful in preventing further progress of the auto-immune disease. These include neonatal thymectomy, administration of cyclosporine, and infusion of anti-pan T-cell, anti-CD4, or anti-CD25 (IL-2R) monoclonal antibodies. The aim of all rejection prevention and auto-immunity reversal strategies is to suppress the patient's immune reactivity to the antigenic tissue or agent, with a minimum of morbidity and mortality. Accordingly, a number of drugs are currently being used or investigated for their immunosuppressive properties. As discussed above, the most commonly used immunosuppressant is cyclosporine, which however has numerous side effects. Accordingly, in view of the relatively few choices

for agents effective at immunosuppression with low toxicity profiles and manageable side effects, there exists a need in the art for identification of alternative immunosuppressive agents and for agents acting as complement to calcineurin inhibition.

The metastasis of cancer cells represents the primary source of clinical morbidity and mortality in the large majority of solid tumors. Metastasis of cancer cells may result from the entry of tumor cells into either lymphatic or blood vessels. Invasion of lymphatic vessels results in metastasis to regional draining lymph nodes. From the lymph nodes, melanoma cells for example tend to metastasize to the lung, liver, and brain. For several solid tumors, including melanoma, the absence or the presence of lymph nodes metastasis is the best predictor of patient survival. Presently, to our knowledge, no treatment is capable of preventing or significantly reducing metastasis. Hence, there is a need in the art for compounds having such anti-metastasis effect for a suitable treatment of cancer patients.

In the field of allergy, IgE is well known for inducing allergy mainly by stimulating mast cells to release histamine. Also, asthma, being characterized by inflammation of airway and bronchospasm, is mainly induced by Th2 cytokines such as IL-5, IL-10 or IL-13. Therefore there is a need in the art for compounds that efficiently inhibit the release of these Th2 cytokines.

There is also a need in the art to improve therapeutic efficiency by providing pharmaceutical compositions or combined preparations exhibiting a synergistic effect as a result of combining two or more immunosuppressant drugs, or antineoplastic drugs or anti-viral drugs or anti-histamine drugs.

Septic shock is a major cause of death in intensive care units (about 150,000 estimated deaths annually in the United States of America, despite treatment with intravenous antibiotics and supportive care) for which very little effective treatment is available at present. Patients with severe sepsis often experience failures of various systems in the body, including the circulatory system, as well as kidney failure, bleeding and clotting. Lipopolysaccharide (hereinafter referred as LPS) is the primary mediator of Gramm-negative sepsis, the most common form of sepsis, by inducing the production of a whole array of macrophage-derived cytokines (such as TNF-$\alpha$; interleukins such as IL-1, IL-6, IL-12; interferon-gamma (hereinafter referred IFN-$\gamma$), etc.). These cytokines may induce other cells (e.g. T cells, NK cells) to make cytokines as well (e.g. IFN-$\gamma$). In addition, other macrophage products (e.g. nitric oxide, hereinafter referred as NO) may also play a role in the pathogenesis of toxic shock. These substances (e.g. NO) may be induced directly due to microbial interactions or indirectly through the action of proinflammatory cytokines. LPS binds to a serum protein known as LPB and the LPS-LPB complex thus formed is recognized by the CD14 toll-like receptor 4 (hereinafter referred as Tlr 4) complex on mononuclear phagocytes. Tlr4 is a signal transducing unit, the activation of which results in the release of mediators such as TNF-$\alpha$, IL-1$\alpha$, IL-1$\beta$ and IL-6. These cytokines are important for the pathogenesis of shock. Their administration produces the clinical symptoms of septic shock and their blockade partially protects against LPS-induced lethal shock.

Current therapeutic strategies for the treatment of septic shock are directed against LPS (e.g. antibodies against LPS or LBP-34-23) or against the cytokines induced by LPS (e.g. TNF antibodies) or against the receptor for LPS (e.a. CD14). Unfortunately the initial clinical data of these approaches are very disappointing and illustrate the redundancy of receptors and mediators involved in the pathogenesis of toxic shock. For instance flagellin seems to be another toxin that plays a role in Gramm-negative Salmonella shock syndrome and that cannot be prevented or treated by therapeutic strategies directed specifically at LPS.

Clinical trials in humans with TNF-$\alpha$ blocking antibodies (such as the IL-1 receptor antagonist or PAF receptor antagonists) have been unsuccessful yet, as have been approaches to down regulate inflammation (e.g. using prednisolone) or to block endotoxins. These products must be administered very early after the onset of the disease, which is in most cases not possible.

The only drug currently approved by health authorities for the treatment of adult patients with the most serious forms of sepsis, including septic shock, is a genetically engineered version of a naturally occurring human protein, Activated Protein C, known as Xigris® or drotecogin-alpha which shows only moderate efficacy. Furthermore, because Activated Protein C interferes with blood clotting, the most serious side effect associated with Xigris® is bleeding, including bleeding that causes stroke. Thus Xigris® is contra-indicated for patients who have active internal bleeding, or who are more likely to bleed because of certain medical conditions including recent strokes, recent head or spinal surgery or severe head trauma. Beacause treatment with Xigris® comes with potentially serious risks, the benefits and risks of treatment with Xigris® must be carefully weighed for each individual patient.

Therefore there is a strong need in the art for new medications, either alone or in combination with the currently suggested treatments, for treating the most serious forms of life-threatening illnesses caused by severe infection, such as septic shock.

TNF-$\alpha$ is generally considered to be the key mediator in the mammalian response to bacterial infection. It is a strong pro-inflammatory agent that will affect the function of almost any organ system, either directly or by inducing the formation of other cytokines like IL-1 or prostaglandines. TNF-$\alpha$ is also a potent anti-tumor agent. If administered in small quantities to humans, it causes fever, headache, anorexia, myalgia, hypotension, capillary leak syndrome, increased rates of lipolysis and skeletal muscle protein degradation (including cachexia). Its use in cancer treatment is

therefore very much limited by its severe side effects.

TNF-α, a pleiotropic cytokine produced mainly by activated macro-phages, exerts an *in vitro* cytotoxic action against transformed cells and *in vivo* anti-tumor activities in animal models. However, despite the fact that TNF-α is used in cancer patients especially to treat melanoma and sarcoma, the major problem hampering its use is toxicity. Indeed, TNF-α induces shock-like symptoms such as bowel swelling and damage, liver cell necrosis, enhanced release of inflammatory cytokines such as IL-1 or IL-6, and hypo-tension probably due to the release of inducers of vessels dilatation such nitric oxide and other proinflammatory cytokines. Cardiovascular toxicity is usually dose-limiting. Hypotension can be severe with systolic blood pressure below 60 mm Hg. Respiratory compromise is common after treatment with TNF-α and may require mechanical ventilation. Upper as well as lower digestive tract symptoms are also common in this type of treatment. Nausea and vomiting can be distressing and in some cases dose-limiting. Watery diarrhea is frequently observed. Neurological sequelae of treatment with TNF-α can also occur.

Hence, compounds that inhibit the toxic effects of TNF-α but that do not inhibit TNF-α anti-tumor effect are highly desirable for the treatment of cancer patients. Presently, several clinical trials involving TNF-α are being developed for the cancer of organs such as liver, lung, kidney and pancreas, which are based on a procedure including the steps of organ isolation, injection of TNF-α into the isolated organ, and reperfusion of the treated organ. However, even for isolated organ perfusion, some TNF-α usually escapes to the general blood circulation and leads to the mortality of about 10% of the patients thus treated. Many patients treated by this procedure also require intensive care unit rescue to cope with the toxic side-effects of such TNF-α treatment.

Combined treatment of TNF-α with alkylating drugs in an isolated organ perfusion model has received considerable attention. TNF-α is currently successfully used in isolated limb perfusion of human cancer patients and, in combination with melphalan and interferon-gamma, against melanoma, sarcomas and carcinomas.

The gastrointestinal mucosa is very sensitive to chemotherapeutic drugs. Mucositis caused by chemotherapy usually begins rapidly after initiation of the treatment with inflammation and ulceration of the gastrointestinal tract and leading to diarrhea. Severe, potentially life-threatening, diarrhea may require interruption of the chemotheraputic treatment and subsequent dose reduction of the therapeutic agent. The oral cavity is often the place of severe side effects from cancer therapy that adversely affects the quality of life of the patient and its ability to tolerate the therapy. These side effects can be caused by radiotherapy as well as chemotherapy. A relationship between both serum and mucosal levels of TNF-α and IL-1 correlates with nonhematologic toxicities, including mucositis.

Radiation injuries occurring e.g. after a single high-dose irradiation include apoptosis as well as radiation necrosis. Even normal tissues protected by shielding during irradiation may be considerably damaged. It was found in experimental animal models that the radiation injuries after a single high-dose irradiation typically used for the treatment of various malignant tumors consist of radiation necrosis and apoptosis, which were correlated with the expression of TNF-α and TGF-β1.

Irradiation may induce graft-versus-host disease (hereinafter referred as GVHD) in cancer patients. This disease may occur especially in patients receiving allogeneic bone marrow transplantation as a treatment for cancers such as leukemia or lymphoma and can lead to the death of about 25% of the relevant patients. Before bone marrow transplantation, leukaemia patients for example receive either total body or total lymphoid irradiation to suppress their immune system. However, such irradiation induces not only necrosis but also the release of proinflammatory cytokines mainly TNF-α, IL-1 and IL-6 which in turn induce direct host tissues inflammation and activation of donor cells against host antigens leading to GVHD.

Cisplatin is an effective chemotherapeutic agent used in the treatment of a wide variety of both pediatric and adult malignancies, including testicular, germ cell, head and neck (cervical), bladder and lung cancer. Dose-dependent and cumulative nephrotoxicity is the major side effect of cisplatin, sometimes requiring a reduction in dose or discontinuation of the treatment. Other side effects of cisplatin include kidney damage, loss of fertility, harmful effect on a developing baby, temporary drop in bone marrow function causing drop in white blood cell count, anaemia, drop in platelets causing bleeding, loss of appetite, numbness or tingling in limbs, loss of taste, allergic reactions, and hearing disorders (difficulty in hearing some high-pitched sounds, experiencing ringing in the ears). Blurred vision may also be a side effect with high doses of cisplatin. It was shown that TNF-α is a key element in a network of proinflammatory chemokines and cytokines activated in the kidney by cisplatin. Blockade of TNF-α action would prevent the activation of this cytokine network and would provide protection against cisplatin nephrotoxicity. Hence, compounds that inhibit the toxic effects of cisplatin but that do not inhibit cisplatin anti-tumor effects are highly desirable for the treatment of cancer patients.

A surplus of TNF-α also causes a dramatic change of endothelial cells. In particular, TNF-α is an important mediator of skeletal muscle degeneration associated with cachexia, a debilitating syndrome characterized by extreme weight loss and whole-body wasting. Cachexia is usually a secondary condition whereby there is excessive tissue catabolism in combination with deficient anabolism. It is frequently seen in patients afflicted with chronic diseases such as cancer, cardiopulmonary diseases, aging, malabsortive disorders, excessive physical stress, easting disorders and acquired immmuno-deficiency syndrome (AIDS). Some authors consider that the elevated TNF-α values found in at least 50% of cancer patients in the active stage of the disease can result in cachexia. TNF-α levels in clinically healthy

adults, as well as in adult cancer patients, are well documented, for instance by Nenova et al. in Archives of Hellenic Medicine (2000) 17:619-621. Serum TNF-α concentrations in healthy children as well as in children with malignancies are documented for instance by Saarinen et al. in Cancer Research (1990) 50:592-595. A very significant proportion of cancer mortalities result from cachexia rather than from tumor burden. Chronic wasting disease (cachexia) may result when excessive cellular damage results in the release of substances (TNF-α, collagenase, hyaluronidase) that further catabolize the so-called healthy tissue resulting in an inability to assimilate nutrients required for anabolic restructuring of associated tissue.

Infants infected with human immunodeficiency virus type 1 (HIV-1) show growth retardation and severe weight loss that can lead to death. The overproduction of certain cytokines has been implicated as a possible cause for this. For instance, according to Rautonen et al. in AIDS (1991) 5:1319-1325, serum IL-6 concentrations are elevated and associated with elevated TNF-α concentrations in children with HIV infection. Swapan et al. in Journal of Virology (2002) 76:11710-11714 have shown that reduction of TNF-α levels by either anti-TNF-α antibodies or human chorionic gonadotropin inhibits the expression of HIV-1 proteins and prevents cachexia and death.

Very few drugs have been suggest at present for the treatment of cachexia. Some high-dose progestins like megestrol acetate, an agent used for the treatment of metastatic breast cancer, and medroxyprogesterone acetate were shown in randomized clinical trials to provide a statistically significant advantage as regards improved appetite and body weight gain. Hence, compounds that stimulate appetite and body weight gain without inhibiting the anti-tumor effect or anti-viral effect of co-administered drugs are highly desirable for the treatment of cachexia. More specifically, there is a need in the art for treating cachexia by the administration of compounds that reduce TNF-α levels in the serum of humans.

TNF-α is also suspected to play a role, through a possible dual action in the hematopoietic environment, in the development of hematologic malignancies such as idiopathic myelodysplastic syndromes occurring most often in elderly people but also occasionally in children, these syndromes being currently regarded as the early phase of acute leukemia.

WO 00/39129 discloses pteridine derivatives wherein the piperazin-1-yl group at position 4 of the pteridine ring is itself substituted in its position 4 with a methyl group. WO 01/21619 discloses hemihydrates and dihydrates of pteridine derivatives wherein the piperazin-1-yl group at position 4 of the pteridine ring is itself substituted in its position 4 with a methyl group.

There is a strong need in the art to improve, or to provide alternatives to, the existing prophylactic or therapeutic solutions to all the aforesaid diseases. Meeting these various needs in the art constitutes the main goal of the present invention.

SUMMARY OF THE INVENTION

In a first embodiment, the present invention relates to a group of novel pteridine derivatives having the general formula (I): wherein:

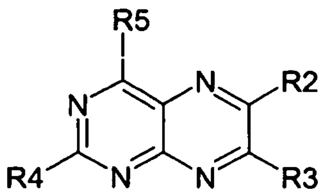

(I)

- $R_5$ is a group represented by the general formula (II):

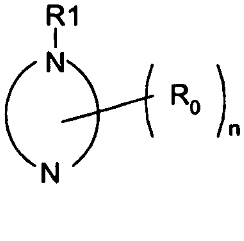

(II)

wherein:

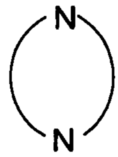

(III)

schematically represents a piperazin-1-yl group or a homopiperazin-1-yl group, and wherein:

- each substituent $R_0$ of the heterocyclic ring (III) is a group independently selected from methyl and phenyl ;
- n is 0, 1 or 2;
- $R_1$ is a substituent group selected from the group consisting of formyl, acyl, thio-acyl, amide, thioamide, sulfonyl, sulfinyl, carboxylate, thiocarboxylate, amino-substituted acyl, alkoxyalkyl, $C_{3-10}$ cycloalkyl-alkyl, $C_{3-10}$ cycloalkyl, dialkylaminoalkyl, heterocyclic-substituted alkyl, acyl-substituted alkyl, thioacyl-substituted alkyl, amido-substituted alkyl, thioamido-substituted alkyl, carboxylato-substituted alkyl, thiocarboxylato-substituted alkyl, (amino-substituted acyl)alkyl, heterocyclic, carboxylic acid ester, w-cyanoalkyl, w-carboxylic ester-alkyl, halo $C_{1-7}$ alkyl, $C_{2-7}$ alkenyl, $C_{2-7}$ alkynyl, arylalkenyl, aryloxyalkyl, arylalkyl and aryl, wherein the aryl moiety of each of said arylalkenyl, aryloxyalkyl, arylalkyl and aryl radicals is optionally substituted with one or more substituents independently selected from the group consisting of halogen, $C_{1-7}$ alkyl, $C_{2-7}$ alkenyl, $C_{2-7}$ alkynyl, halo $C_{1-7}$ alkyl, nitro, hydroxyl, sulfhydryl, amino, $C_{1-7}$ alkoxy, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thio-heterocyclic, arylalkylthio, heterocyclic-substituted alkylthio, formyl, carbamoyl, sulfon-amido, hydroxylamino, alkoxyamino, mercaptoamino, thioalkyl-amino, acylamino, thioacylamino, cyano, carboxylic acid or esters, alkylamino, cycloalkylamino, alkenyl-amino, cycloalkenylamino, alkynylamino, arylamino, aryl-alkylamino, hydroxyalkylamino, mercaptoalkylamino and heterocyclic amino;
- $R_3$ is hydrogen;
- $R_4$ is amino;
- $R_2$ is a phenyl group optionally substituted with one or more substituents selected from the group consisting of halogen, $C_{1-7}$ alkyl and $C_{1-7}$ alkoxy ;

and/or a pharmaceutically acceptable addition salt thereof and/or a stereoisomer thereof and/or a mono- or a di-N-oxide thereof and/or a solvate thereof and/or a dihydro- or tetrahydropteridine derivative thereof.

The above novel compounds of this first embodiment have in common the structural features present in the general formula (I), in particular the pteridine ring is substituted by at least one N,N-containing heterocyclic group being itself N-substituted by a carbonyl or thiocarbonyl or sulfonyl radical or by certain hydrocarbonyl radicals other than $C_{1-7}$ alkyl. They also have a potential specific biological activity profile and consequent usefulness in medicinal chemistry.

It has been found unexpectedly that at least one desirable biological property such as the ability to decrease the proliferation of lymphocytes, or to decrease T-cell activation, or to decrease B-cell or monocytes or macrophages activation, or to inhibit the release of certain cytokines, or in inhibiting human TNF-$\alpha$ production is a feature which is present in the said group of novel compounds. As a consequence, the invention relates to pharmaceutical compositions comprising as an active principle at least one pteridine derivative having the general formula (I), and/or a pharmaceutically acceptable addition salt thereof and/or a stereoisomer thereof and/or a mono- or a di-N-oxide thereof and/or a solvate and/or a dihydro- or tetrahydropteridine derivative thereof.

Compounds having the general formulae (I) are highly active immunosuppressive agents, antineoplastic agents, anti-allergic agents or anti-viral agents which, together with one or more pharmaceutically acceptable carriers, may be formulated into pharmaceutical compositions for the prevention or treatment of pathologic conditions such as immune and autoimmune disorders, organ and cells transplant rejections, allergic conditions, cell proliferative disorders, cardiovascular disorders, disorders of the central nervous system and viral diseases. Compounds having the general formulae (I) are also useful for the prevention or treatment of a TNF-$\alpha$-related disorder in a mammal, such as for instance:

- septic or endotoxic shock,
- TNF-$\alpha$- mediated diseases,
- pathologies and conditions associated with and/or induced by abnormal levels of TNF-$\alpha$ occurring in a systemic, localized or particular tissue type or location in the body of the mammal,
- toxic effects of TNF-$\alpha$ and/or anti-cancer chemotherapeutic agents,
- injuries after irradiation of a tissue of the mammal by radio-elements, and

- cachexia.

In a further embodiment, the present invention relates to combined preparations containing at least one compound of the general formula (I) and one or more drugs selected from immunosuppressant and/or immunomodulator drugs, antineoplastic drugs, and antiviral agents.

Further disclosed are various processes and methods for making the novel pteridine derivatives defined in general formulae (I), as well as their pharmaceutically acceptable salts, N-oxides, solvates, enantiomers and dihydro- and tetrahydroderivatives.

## BRIEF DESCRIPTION OF THE DRAWINGS

Figure 1 schematically shows a first method for making trisubstituted and tetrasubstituted pteridine derivatives having the formula (I) wherein $R_5$ is a group having the formula (II).

Figure 2 schematically shows a second method for making trisubstituted and tetrasubstituted pteridine derivatives having the formula (I) wherein $R_5$ is a group having the formula (II).

Figure 3 schematically shows a third method for making trisubstituted and tetrasubstituted pteridine derivatives having the formula (I) wherein $R_5$ is a group having the formula (II).

Figure 4 schematically shows a fourth method for making trisubstituted and tetrasubstituted pteridine derivatives having the formula (I) wherein $R_5$ is a group having the formula (II).

Figure 5 schematically shows a fifth method for making trisubstituted and tetrasubstituted pteridine derivatives having the formula (I) wherein $R_5$ is a group having the formula (II).

Figure 6 schematically shows a sixth method for making trisubstituted and tetrasubstituted pteridine derivatives having the formula (I) wherein $R_5$ is a group having the formula (II).

Figure 7 schematically shows a seventh method for making trisubstituted and tetrasubstituted pteridine derivatives having the formula (I) wherein $R_5$ is a group having the formula (II).

Figure 8 schematically shows a method for making trisubstituted and tetrasubstituted pteridine derivatives having the formula (I) wherein $R_4$ and $R_5$ are identical groups having the formula (II). (reference)

Figure 9 schematically shows another method for making trisubstituted and tetrasubstituted pteridine derivatives having the formula (I) wherein $R_5$ is a specific group having the formula (II).

## DEFINITIONS

Unless otherwise stated herein, the term " trisubstituted " means that three of the carbon atoms being in positions 2, 4 and 6 or, alternatively, in positions 2, 4 and 7 of the pteridine ring (according to standard atom numbering for the pteridine ring) are substituted with an atom or group other than hydrogen. The term " tetrasubstituted " means that all four carbon atoms being in positions 2, 4, 6 and 7 of the pteridine ring are substituted with an atom or group other than hydrogen.

As used herein with respect to a substituting radical, and unless otherwise stated, the term "$C_{1-7}$ alkyl " means straight and branched chain saturated acyclic hydrocarbon monovalent radicals having from 1 to 7 carbon atoms such as, for example, methyl, ethyl, propyl, n-butyl, 1-methylethyl (isopropyl), 2-methylpropyl (isobutyl), 1,1-dimethylethyl (ter-butyl), 2-methylbutyl, n-pentyl, dimethylpropyl, n-hexyl, 2-methylpentyl, 3-methylpentyl, n-heptyl.

As used herein with respect to a substituting radical, and unless otherwise stated, the term " acyl " broadly refers to a carbonyl (oxo) group adjacent to a $C_{1-7}$ alkyl radical, a $C_{3-10}$ cycloalkyl radical, an aryl radical, an arylalkyl radical or a heterocyclic radical, all of them being such as herein defined; representative examples include acetyl, benzoyl, naphthoyl; similarly, the term " thioacyl " refers to a C=S (thioxo) group adjacent to one of the said radicals.

As used herein with respect to a substituting radical, and unless otherwise stated, the term "$C_{1-7}$ alkylene " means the divalent hydrocarbon radical corresponding to the above defined $C_{1-7}$ alkyl, such as methylene, bis(methylene), tris(methylene), tetramethylene, hexamethylene.

As used herein with respect to a substituting radical, and unless otherwise stated, the term " $C_{3-10}$ cycloalkyl " means a mono- or polycyclic saturated hydrocarbon monovalent radical having from 3 to 10 carbon atoms, such as for instance cyclopropyl, cyclobutyl, cyclopentyl, cyclohexyl, cycloheptyl, cyclooctyl or a $C_{7-10}$ polycyclic saturated hydrocarbon monovalent radical having from 7 to 10 carbon atoms such as, for instance, norbornyl, fenchyl, trimethyltricycloheptyl or adamantyl.

As used herein with respect to a substituting radical, and unless otherwise stated, the term " $C_{3-10}$ cycloalkyl-alkyl " refers to an aliphatic saturated hydrocarbon monovalent radical (preferably a $C_{1-7}$ alkyl such as defined above) to which a $C_{3-10}$ cycloalkyl (such as defined above) is already linked such as cyclohexylmethyl, cyclopentylmethyl.

As used herein with respect to a substituting radical, and unless otherwise stated, the term " $C_{3-10}$ cycloalkylene" means the divalent hydrocarbon radical corresponding to the above defined $C_{3-10}$ cycloalkyl.

As used herein with respect to a substituting radical, and unless otherwise stated, the term " aryl " designate any mono- or polycyclic aromatic monovalent hydrocarbon radical having from 6 up to 30 carbon atoms such as but not limited to phenyl, naphthyl, anthracenyl, phenantracyl, fluoranthenyl, chrysenyl, pyrenyl, biphenylyl, terphenyl, picenyl, indenyl, biphenyl, indacenyl, benzocyclobutenyl, benzocyclooctenyl, including fused benzo-$C_{4-8}$ cycloalkyl radicals (the latter being as defined above) such as, for instance, indanyl, tetrahydronaphtyl, fluorenyl, all of the said radicals being optionally substituted with one or more substituents independently selected from the group consisting of halogen, amino, trifluoromethyl, hydroxyl, sulfhydryl and nitro, such as for instance 4-fluorophenyl, 4-chlorophenyl, 3,4-dichlorophenyl, 4-cyanophenyl, 2,6-dichlorophenyl, 2-fluorophenyl, 3-chlorophenyl, 3,5-dichlorophenyl.

As used herein, e.g. with respect to a substituting radical such as the combination of substituents in positions 6 and 7 of the pteridine ring together with the carbon atoms in positions 6 and 7 of the pteridine ring, and unless otherwise stated, the term " homocyclic" means a mono- or polycyclic, saturated or mono-unsaturated or polyunsaturated hydrocarbon radical having from 4 up to 15 carbon atoms but including no heteroatom in the said ring; for instance said combination of substituents in positions 6 and 7 of the pteridine ring may form a $C_{2-6}$ alkylene radical, such as tetramethylene, which cyclizes with the carbon atoms in positions 6 and 7 of the pteridine ring.

As used herein with respect to a substituting radical (including the combination of substituents in positions 6 and 7 of the pteridine ring together with the carbon atoms in positions 6 and 7 of the pteridine ring), and unless otherwise stated, the term " heterocyclic " means a mono- or polycyclic, saturated or mono-unsaturated or polyunsaturated monovalent hydrocarbon radical having from 2 up to 15 carbon atoms and including one or more heteroatoms in one or more heterocyclic rings, each of said rings having from 3 to 10 atoms (and optionally further including one or more heteroatoms attached to one or more carbon atoms of said ring, for instance in the form of a carbonyl or thiocarbonyl or selenocarbonyl group, and/or to one or more heteroatoms of said ring, for instance in the form of a sulfone, sulfoxide, N-oxide, phosphate, phosphonate or selenium oxide group), each of said heteroatoms being independently selected from the group consisting of nitrogen, oxygen, sulfur, selenium and phosphorus, also including radicals wherein a heterocyclic ring is fused to one or more aromatic hydrocarbon rings for instance in the form of benzo-fused, dibenzo-fused and naphto-fused heterocyclic radicals; within this definition are included heterocyclic radicals such as diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxa-thiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzo-dioxocinyl, benzotrioxepinyl, benzoxathiazepinyl, benzoxadiazepinyl, benzothia-diazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoiso-quinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphto-pyranyl, oxabicycloheptyl, azabenzimidazolyl, azacycloheptyl, azacyclooctyl, azacyclononyl, azabicyclononyl, tetrahydrofuryl, tetrahydropyranyl, tetrahydro-pyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thiourazolyl, thiotriazolyl, thiopyranyl, thiopyronyl, coumarinyl, quinoleinyl, oxyquinoleinyl, quinuclidinyl, xanthinyl, dihydropyranyl, benzo-dihydrofuryl, benzothiopyronyl, benzothiopyranyl, benzoxazinyl, benzoxazolyl, benzodioxolyl, benzodioxanyl, benzothiadiazolyl, benzotriazinyl, benzo-thiazolyl, benzoxazolyl, phenothioxinyl, phenothiazolyl, phenothienyl (benzothiofuranyl), phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl, cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, oxetanonyl, homopiperazinyl, homopiperidinyl, thietyl, thietanyl, diazabicyclooctyl, diazetyl, diaziridinonyl, diaziridinethionyl, chromanyl, chromanonyl, thiochromanyl, thiochromanonyl, thiochromenyl, benzofuranyl, benzisothiazolyl, benzo-carbazolyl, benzochromonyl, benziso-alloxazinyl, benzocoumarinyl, thiocoumarinyl, phenometoxazinyl, phenoparoxazinyl, phentriazinyl, thiodiazinyl, thiodiazolyl, indoxyl, thioindoxyl, benzodiazinyl (e.g. phtalazinyl), phtalidyl, phtalimidinyl, phtalazonyl, alloxazinyl, dibenzopyronyl (i.e. xanthonyl), xanthionyl, isatyl, isopyrazolyl, isopyrazolonyl, urazolyl, urazinyl, uretinyl, uretidinyl, succinyl, succinimido,

benzylsultimyl, benzylsultamyl and the like, including all possible isomeric forms thereof, wherein each carbon atom of said heterocyclic ring may be independently substituted with a substituent selected from the group consisting of halogen, nitro, $C_{1-7}$ alkyl (optionally containing one or more functions or radicals selected from the group consisting of carbonyl (oxo), alcohol (hydroxyl), ether (alkoxy), acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxylalkylamino, mercaptoalkylamino, heterocyclic-substituted alkylamino, heterocyclic amino, heterocyclic-substituted arylamino, hydrazino, alkylhydrazino, phenylhydrazino, sulfonyl, sulfonamido and halogen), $C_{3-7}$ alkenyl, $C_{2-7}$ alkynyl, halo $C_{1-7}$ alkyl, $C_{3-10}$ cycloalkyl, aryl, arylalkyl, alkylaryl, alkylacyl, arylacyl, hydroxyl, amino, $C_{1-7}$ alkylamino, cycloalkylamino, alkenylamino, cyclo-alkenylamino, alkynylamino, arylamino, arylalkylamino, hydroxyalkylamino, mercaptoalkylamino, heterocyclic-substituted alkylamino, heterocyclic amino, heterocyclic-substituted arylamino, hydrazino, alkylhydrazino, phenylhydrazino, sulfhydryl, $C_{1-7}$ alkoxy, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof; depending upon the number of unsaturations in the 3 to 10 membered ring, heterocyclic radicals may be sub-divided into heteroaromatic (or " heteroaryl") radicals and non-aromatic heterocyclic radicals; when a heteroatom of the said non-aromatic heterocyclic radical is nitrogen, the latter may be substituted with a substituent selected from the group consisting of $C_{1-7}$ alkyl, $C_{3-10}$ cycloalkyl, aryl, arylalkyl and alkylaryl.

As used herein with respect to a substituting radical, and unless otherwise stated, the terms " $C_{1-7}$ alkoxy ", "$C_{3-10}$ cycloalkoxy ", " aryloxy", "arylalkyloxy ", " oxyheterocyclic ", "thio $C_{1-7}$ alkyl", " thio $C_{3-10}$ cycloalkyl ", " arylthio ", " arylalkylthio " and " thioheterocyclic" refer to substituents wherein a $C_{1-7}$ alkyl radical, respectively a $C_{3-10}$ cycloalkyl, aryl, arylalkyl or heterocyclic radical (each of them such as defined herein), are attached to an oxygen atom or a divalent sulfur atom through a single bond, such as methoxy, ethoxy, propoxy, butoxy, pentoxy, isopropoxy, sec-butoxy, tert-butoxy, isopentoxy, cyclopropyloxy, cyclobutyloxy, cyclopentyloxy, thiomethyl, thioethyl, thiopropyl, thiobutyl, thiopentyl, thiocyclopropyl, thiocyclobutyl, thiocyclopentyl, thiophenyl, phenyloxy, benzyloxy, mercaptobenzyl, cresoxy.

As used herein with respect to a substituting atom, and unless otherwise stated, the term halogen means any atom selected from the group consisting of fluorine, chlorine, bromine and iodine.

As used herein with respect to a substituting radical, and unless otherwise stated, the term " halo $C_{1-7}$ alkyl " means a $C_{1-7}$ alkyl radical (such as above defined) in which one or more hydrogen atoms are independently replaced by one or more halogens (preferably fluorine, chlorine or bromine), such as but not limited to difluoromethyl, trifluoromethyl, trifluoroethyl, octafluoropentyl, dodecafluoroheptyl, dichloromethyl.

As used herein with respect to a substituting radical, and unless otherwise stated, the terms " $C_{2-7}$ alkenyl " designate a straight and branched acyclic hydrocarbon monovalent radical having one or more ethylenic unsaturations and having from 2 to 7 carbon atoms such as, for example, vinyl, 1-propenyl, 2-propenyl (allyl), 1-butenyl, 2-butenyl, 2-pentenyl, 3-pentenyl, 3-methyl-2-butenyl, 3-hexenyl, 2-hexenyl, 2-heptenyl, 1,3-butadienyl, pentadienyl, hexadienyl, heptadienyl, heptatrienyl, including all possible isomers thereof.

As used herein with respect to a substituting radical, and unless otherwise stated, the term " $C_{3-10}$ cycloalkenyl" mean a monocyclic mono- or polyunsaturated hydrocarbon monovalent radical having from 3 to 8 carbon atoms, such as for instance cyclopropenyl, cyclobutenyl, cyclopentenyl, cyclopentadienyl, cyclohexenyl, cyclohexadienyl, cycloheptenyl, cyclohepta-dienyl, cycloheptatrienyl, cyclooctenyl, cyclooctadieny,l or a $C_{7-10}$ polycyclic mono- or polyunsaturated hydrocarbon mono-valent radical having from 7 to 10 carbon atoms such as dicyclopentadienyl, fenchenyl (including all isomers thereof, such as $\alpha$-pinolenyl), bicyclo[2.2.1]hept-2-enyl, bicyclo[2.2.1]hepta-2,5-dienyl, cyclo-fenchenyl.

As used herein with respect to a substituting radical, and unless otherwise stated, the term " $C_{2-7}$ alkynyl " defines straight and branched chain hydrocarbon radicals containing one or more triple bonds and optionally at least one double bond and having from 2 to 7 carbon atoms such as, for example, acetylenyl, 1-propynyl, 2-propynyl, 1-butynyl, 2-butynyl, 2-pentynyl, 1-pentynyl, 3-methyl-2-butynyl, 3-hexynyl, 2-hexynyl, 1-penten-4-ynyl, 3-penten-1-ynyl, 1,3-hexadien-1-ynyl.

As used herein with respect to a substituting radical, and unless otherwise stated, the terms " arylalkyl ", " arylalkenyl " and " heterocyclic-substituted alkyl " refer to an aliphatic saturated or ethylenically unsaturated hydrocarbon monovalent radical (preferably a $C_{1-7}$ alkyl or $C_{2-7}$ alkenyl radical such as defined above) onto which an aryl or heterocyclic radical (such as defined above) is already bonded, and wherein the said aliphatic radical and/or the said aryl or heterocyclic radical may be optionally substituted with one or more substituents independently selected from the group consisting of halogen, amino, hydroxyl, sulfhydryl, $C_{1-7}$ alkyl, trifluoromethyl and nitro, such as benzyl, 4-chlorobenzyl, 4-fluorobenzyl, 2-fluorobenzyl, 3,4-dichlorobenzyl, 2,6-dichlorobenzyl, 3-methylbenzyl, 4-methylbenzyl, 4-*ter*-butylbenzyl, phenylpropyl, 1-naphthylmethyl, phenylethyl, 1-amino-2-phenylethyl, 1-amino-2-[4-hydroxy-phenyl] ethyl, 1-amino-2-[indol-2-yl]ethyl, styryl, pyridylmethyl (including all isomers thereof), pyridylethyl, 2-(2-pyridyl)isopropyl, oxazolylbutyl, 2-thienylmethyl, pyrrolylethyl, morpholinylethyl, imidazol-1-yl-ethyl, benzodioxolylmethyl and 2-furylmethyl.

As used herein with respect to a substituting radical, and unless otherwise stated, the term " alkylaryl " and "alkyl-substituted heterocyclic" refer to an aryl or heterocyclic radical (such as defined above) onto which are bonded one or more aliphatic saturated or unsaturated hydrocarbon mono-valent radicals, preferably one or more $C_{1-7}$ alkyl, $C_{2-7}$ alkenyl or $C_{3-10}$ cycloalkyl radicals as defined above such as o-toluyl, m-toluyl, p-toluyl, 2,3-xylyl, 2,4-xylyl, 3,4-xylyl, o-cumenyl, m-cumenyl, p-cumenyl, o-cymenyl, m-cymenyl, p-cymenyl, mesityl, ter-butylphenyl, lutidinyl (i.e. dimethylpyridyl), 2-methylaziridinyl, methylbenzimidazolyl, methylbenzo-furanyl, methylbenzothiazolyl, methylbenzotriazolyl, methylbenzoxazolyl and methylbenzselenazolyl.

As used herein with respect to a substituting radical, and unless otherwise stated, the term " alkoxyaryl " refers to an aryl radical (such as defined above) onto which is (are) bonded one or more $C_{1-7}$ alkoxy radicals as defined above, preferably one or more methoxy radicals, such as, 2-methoxyphenyl, 3-methoxyphenyl, 4-methoxyphenyl, 3,4-dimethoxyphenyl, 2,4,6-trimethoxyphenyl, methoxynaphtyl.

As used herein with respect to a substituting radical, and unless otherwise stated, the terms " alkylamino ", " cycloalkylamino ", " alkenyl-amino ", " cycloalkenylamino " , " arylamino ", " arylalkylamino ", " heterocyclic-substituted alkylamino ", " heterocyclic-substituted arylamino ", " heterocyclic amino ", " hydroxyalkylamino ", " mercaptoalkylamino " and " alkynylamino " mean that respectively one (thus monosubstituted amino) or even two (thus disubstituted amino) $C_{1-7}$ alkyl, $C_{3-10}$ cycloalkyl, $C_{2-7}$ alkenyl, $C_{3-10}$ cycloalkenyl, aryl, arylalkyl, heterocyclic-substituted alkyl, heterocyclic-substituted aryl, heterocyclic (provided in this case the nitrogen atom is attached to a carbon atom of the heterocyclic ring), mono- or polyhydroxy $C_{1-7}$ alkyl, mono- or polymercapto $C_{1-7}$ alkyl or $C_{2-7}$ alkynyl radical(s) (each of them as defined herein, respectively) is/are attached to a nitrogen atom through a single bond such as anilino, benzylamino, methylamino, dimethylamino, ethylamino, diethylamino, isopropylamino, propenylamino, n-butylamino, ter-butylamino, dibutylamino, morpholinoalkylamino, 4-morpholinoanilino, hydroxymethylamino, β-hydroxyethylamino and ethynylamino; this definition also includes mixed disubstituted amino radicals wherein the nitrogen atom is attached to two such radicals belonging to two different sub-set of radicals, e.g. an alkyl radical and an alkenyl radical, or to two different radicals within the same sub-set of radicals, e.g. methylethylamino; among disubstituted amino radicals, symetrically substituted are more easily accessible and thus usually preferred.

As used herein with respect to a substituting radical, and unless otherwise stated, the terms "(thio)carboxylic acid ester", "(thio)carboxylic acid thioester " and " (thio)carboxylic acid amide" refer to radicals wherein the carboxyl or thiocarboxyl group is directly attached to the pteridine ring (e.g. in the 6- and/or 7-position) and wherein said carboxyl or thiocarboxyl group is bonded to the hydrocarbonyl residue of an alcohol, a thiol, a polyol, a phenol, a thiophenol, a primary or secondary amine, a polyamine, an amino-alcohol or ammonia, the said hydrocarbonyl residue being selected from the group consisting of alkyl, alkenyl, alkynyl, cycloalkyl, cycloalkenyl, aryl, arylalkyl, alkylaryl, alkylamino, cycloalkylamino, alkenylamino, cycloalkenylamino, arylamino, arylalkylamino, heterocyclic-substituted alkylamino, heterocyclic amino, heterocyclic-substituted arylamino, hydroxyalkylamino, mercaptoalkylamino or alkynylamino (such as above defined, respectively).

As used herein with respect to a substituting radical, and unless otherwise stated, the term " amino-acid " refers to a radical derived from a molecule having the chemical formula $H_2N$-CHR-COOH, wherein R is the side group of atoms characterizing the amino-acid type; said molecule may be one of the 20 naturally-occurring amino-acids or any similar non naturally-occurring amino-acid.

As used herein and unless otherwise stated, the term " stereoisomer " refers to all possible different isomeric as well as conformational forms which the compounds of formula (I), (IV) and (V) may possess, in particular all possible stereochemically and conformationally isomeric forms, all diastereo-mers, enantiomers and/or conformers of the basic molecular structure. Some compounds of the present invention may exist in different tautomeric forms, all of the latter being included within the scope of the present invention.

As used herein and unless otherwise stated, the term " enantiomer " means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

As used herein and unless otherwise stated, the term " solvate " includes any combination which may be formed by a pteridine derivative of this invention with a suitable inorganic solvent (e.g. hydrates) or organic solvent, such as alcohols, ketones, esters.

As used herein and unless otherwise stated, the terms " dihydro-pteridine derivative " and " tetrahydropteridine derivative " refer to the hydrogenation products of the pteridine derivatives having the general formula (I), i.e. derivatives wherein two hydrogen atoms are present in positions 5 and 6, or 7 and 8, of the pteridine ring, or wherein four hydrogen atoms are present in positions 5, 6, 7 and 8 of the said ring; such hydrogenated derivatives are easily accessible from the pteridine derivatives using hydrogenation methods well known in the art.

DETAILED DESCRIPTION OF THE INVENTION

An object of the invention is to provide a pharmaceutical composition having high immunosuppressive activity. Thus, the present invention relates in particular to the medical applications of a group of pteridine derivatives, their pharmaceutically acceptable salts, N-oxides, solvates, polymorphs, dihydro- and tetrahydroderivatives and enantiomers, possessing unexpectedly desirable pharmaceutical properties, in particular which are highly active immunosuppressive agents, and as such are useful in the treatment in transplant rejection and/or in the treatment of certain inflammatory diseases.

Surprisingly, the compounds of the present invention show a broader therapeutic spectrum profile than merely immunosuppressive activity, as is evidenced by the results obtained in the diversity of test procedures disclosed hereinbelow. A further advantageous feature of the compounds of the present invention resides in their excellent oral activity.

In the first embodiment of the invention, the novel pteridine derivatives are as defined in the general formula (I), wherein each of the substituents $R_0$, $R_1$, $R_2$, $R_3$, $R_4$ and $R_5$ may independently correspond to any of the definitions given above, in particular with any of the individual meanings (such as illustrated above) of generic terms used for substituting radicals such as " $C_{1-7}$ alkyl ", " $C_{3-10}$ cycloalkyl ", " $C_{2-7}$ alkenyl ", " $C_{2-7}$ alkynyl ", " aryl ", " homocyclic ", " heterocyclic ", " halogen ", " $C_{3-10}$ cycloalkenyl ", "alkylaryl ", "arylalkyl ", "alkylamino", " cycloalkylamino ", "alkenylamino ", "alkynylamino", "arylamino", " arylalkylamino ", " heterocyclic-substituted alkylamino, heterocyclic amino, heterocyclic-substituted arylamino, ", "hydroxyalkylamino ", " mercaptoalkylamino ", " alkynylamino ", " $C_{1-7}$ alkoxy", " $C_{3-10}$ cycloalkoxy ", "thio $C_{1-7}$ alkyl ", " thio $C_{3-10}$ cycloalkyl ", " halo $C_{1-7}$ alkyl ", "amino-acid ".

Stereoisomers of the compounds of this invention may be formed by using reactants in their single enantiomeric form wherever possible in the manufacturing process or by resolving the mixture of stereoisomers by conventional methods. One such method is liquid chromatography using one or more suitable chiral stationary phases including, for example, poly-saccharides, in particular cellulose or amylose derivatives. Commercially available polysaccharide-based chiral stationary phases are ChiralCel™ CA, OA, OB, OC, OD, OF, OG, OJ and OK, and Chiralpak™ AD, AS, OP(+) and OT(+). Appropriate eluents or mobile phases for use in combination with said polysaccharide-based chiral stationary phases are hydrocarbons such as hexane, optionally admixed with an alcohol such as ethanol, isopropanol. The above mixture of enantiomers may alternatively be separated by making use of microbial resolution or by resolving the diastereoisomeric salts formed with chiral acids such as mandelic acid, camphorsulfonic acid, tartaric acid, lactic acid or with chiral bases such as brucine. The resolving agent may be cleaved from the separated diastereoisomers, e.g. by treatment with acids or bases, in order to generate the pure enantiomers of the compounds of the invention. Conventional resolution methods were compiled e.g. by Jaques et al. in " Enantiomers, Racemates and Resolution " (Wiley Interscience, 1981).

In the general formula (II), the said heterocyclic group may be substituted, at one or more carbon atoms, by a number n of substituents $R_0$ wherein n is an integer from 0 to 6 and wherein, when n is at least 2, each $R_0$ may be defined independently from the others. The presence of one or more such substituents $R_0$ is a suitable way for introducing chirality into the pteridine derivatives having the general formula (I). In practice, the choice of substituents $R_0$ may be restricted by the commercial availability of the substituted heterocyclic amine, depending upon the specific nature of the heterocyclic group.

More preferably the schematic notation (III)

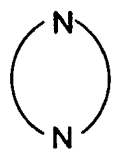

represents a piperazin-1-yl group or a homopiperazin-1-yl group, in which case n is 0, 1 or 2, and $R_0$ is methyl or phenyl (such as for instance in 2-methylpiperazin-1-yl, 2-phenylpiperazin-1-yl and 2,5-dimethyl-piperazin-1-yl).

As shown in the general formula (II) taken together with the definition of $R_1$, a requirement of an embodiment of the invention is that one of the two nitrogen atoms of the heterocyclic ring bears a substituent $R_1$ which has a carbonyl (oxo) or thiocarbonyl (thioxo) or sulfonyl function preferably immediately adjacent to the said nitrogen atom. In other words, this embodiment means that when $R_1$ is selected from, respectively, acyl, thioacyl, amide, thioamide, sulfonyl, sulfinyl, carboxylate and thiocarboxylate, then $R_1$ together with the nitrogen atom to which it is attached forms, respectively, an amide, thioamide, urea, thiourea, sulfonamido, sulfinamido, carbamato or thiocarbamato group.

Especially useful species of pteridine derivatives having the general formula (I) are those wherein $R_5$ is a piperazin-1-yl group or a homopiperazin-1-yl group, said group being substituted in the 4 position with a substituent $R_1$, wherein $R_1$ is selected from the group consisting of:

- $COR_8$ wherein $R_8$ is selected from the group consisting of hydrogen; $C_{1-7}$ alkyl; $C_{3-10}$ cycloalkyl; aryl optionally substituted with one or more substituents selected from the group consisting of halogen, $C_{1-7}$ alkyl, cyano and $C_{1-7}$ alkoxy; heterocyclic optionally substituted with one or more halogen atoms; arylalkyl; aryloxyalkyl; arylalkoxyalkyl; alkoxyalkyl; arylalkoxy; aryloxy; arylalkenyl; heterocyclic-substituted alkyl; alkylamino and arylamino; representative examples of $R_8$ are methyl, ethyl, pentyl, cyclohexyl, phenyl, 4-fluorophenyl, 4-chlorophenyl, 3,4-dichlorophenyl, 4-butylphenyl, 4-cyanophenyl, 2-methoxyphenyl, 3-methoxyphenyl, 4-pentoxyphenyl, naphtyl, 2-thienyl, 4-pyridinyl, 1-tetrahydropyrrolyl, 2-tetrahydropyrrolyl, 2-furanyl, 3-furanyl, 2,4-dichloro-5-fluoro-3-pyridinyl, diethylamino, diisopropylamino, diphenylamino, phenyl-ethyl, 4-chlorobenzyl, phenoxymethyl, benzyloxymethyl, methoxymethyl, 2-thienylmethyl, styryl, benzyloxy, phenoxy, 1-amino-2-phenylethyl, 1-amino-2-[4-hydroxyphenyl]ethyl and 1-amino-2-[indol-2-yl]ethyl;
- $CSR_9$, wherein $R_9$ is selected from the group consisting of alkylamino and aryloxy, such as dimethylamino and phenoxy;
- $SO_2$ $R_{10}$, wherein $R_{10}$ is selected from the group consisting of aryl and arylalkyl, such as phenyl and benzyl; and
- $R_{11}$, wherein $R_{11}$ is selected from the group consisting of aryl, arylalkyl, arylalkenyl, alkoxyalkyl, heterocyclic-substituted alkyl, $C_{3-10}$cycloalkylalkyl, heterocyclic, $C_{3-10}$ cycloalkyl, alkylaminoalkyl, aryloxyalkyl, ω-cyanoalkyl, ω-carboxylatoalkyl and carboxamidoalkyl.

Further disclosed are various processes and methods for making the novel pteridine derivatives having the general formula (I). As a general rule, the preparation of these compounds is based on the principle that, starting from a suitable pteridine precursor (a diaminopyrimidine), each of the substituents $R_2$, $R_3$, $R_4$ and $R_5$ may be introduced separately (except, of course, when $R_2$ together with $R_3$ forms a homocyclic or heterocyclic radical) without adversely influencing the presence of one or more substituents already introduced at other positions on the pteridine ring or the capacity to introduce further substituents later on. Methods of manufacture have been developed by the present inventors which may be used alternatively to, or may be combined with, the methods of synthesis already known in the art of pteridine derivatives (depending upon the targeted final compound). For instance, methods for simultaneously introducing $R_2$ and $R_3$ in the form of a homocyclic or heterocyclic radical at positions 6 and 7 of the pteridine ring are already known from U.S. Patent No. 2,581,889. The synthesis of mono- and di-*N*-oxides of the pteridine derivatives of this invention can easily be achieved by treating the said derivatives with an oxidizing agent such as, but not limited to, hydrogen peroxide (e.g. in the presence of acetic acid) or a peracid such as chloroperbenzoic acid. Dihydro- and tetrahydropteridine derivatives of this invention can easily be obtained by catalytic hydrogenation of the corresponding pteridine derivatives, e.g. by placing the latter in a hydrogen atmosphere in the presence of platinum oxide or platinum. The methods for making the pteridine derivatives of the present invention will now be explained in more details by reference to the appended figures 1 to 9 wherein, unless otherwise stated hereinafter, each of the substituting groups or atoms $R_2$, $R_3$, $R_4$ and $R_5$ is as defined in formula (I) of the summary of the invention and, more specifically, may correspond to any of the individual meanings disclosed above. For a reason of convenience, each of figures 1 to 4 shows piperazin-1-yl as a representative example of the heterocyclic ring schematically represented as

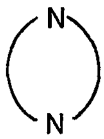

in the general formula (11), however it should be understood that the methods of the invention are not particularly limited to piperazin-1-yl but can be applied successfully to any other heterocyclic ring meeting the requirements specified hereinabove, in particular homopiperazin-1-yl.

In the description of the reaction steps involved in each figure, reference is made to the use of certain catalysts and/or certain types of solvents. It should be understood that each catalyst mentioned should be used in a catalytic amount well known to the skilled person with respect to the type of reaction involved. Solvents that may be used in the following reaction steps include various kinds of organic solvents such as protic solvents, polar aprotic solvents and non-polar solvents as well as aqueous solvents which are inert under the relevant reaction conditions. More specific examples include aromatic hydrocarbons, chlorinated hydrocarbons, ethers, aliphatic hydrocarbons, alcohols, esters, ketones, amides, water or mixtures thereof, as well as supercritical solvents such as carbon dioxide (while performing the reaction under supercritical conditions). The suitable reaction temperature and pressure conditions applicable to each kind of reaction step will not be detailed herein but do not depart from the relevant conditions already known to the skilled person with respect to the type of reaction involved and the type of solvent used (in particular its boiling point).

Figure 1 schematically shows a first method for making trisubstituted pteridine derivatives having the formula (I) wherein $R_5$ is a group having the formula (II) and wherein $R_2$ or $R_3$ is hydrogen, as well as tetrasubstituted pteridine

derivatives having the formula (I) wherein $R_5$ is a group having the formula (II). In step (a), a nitroso group is introduced on position 5 of the pyrimidine ring of a 2-$R_4$-substituted 4-oxo-6-aminopyrimidine by using sodium nitrite under aqueous acidic conditions. Reduction of the nitroso group in step (b) is achieved either catalytically ($Pt/H_2$) in the presence of a protic solvent, or chemically using sodium dithionite or ammonium sulfide in water. Then in a next step (c), condensing the resulting 2-$R_4$-substituted 4-oxo-5,6-diamino-pyrimidine with an $\alpha$-ketoaldoxime bearing a radical $R_2$, wherein $R_2$ may be *inter alia* $C_{1-7}$ alkyl, $C_{3-10}$ cycloalkyl, aryl or heteroaryl, in a protic solvent such as methanol under acidic conditions regioselectively yields a 4-oxopteridine bearing a $R_4$ substituent in position 2 and a $R_2$ substituent in position 6 of the pteridine ring. Alternatively, a 2-$R_4$-substituted 4-oxo-7-$R_3$-substituted pteridine derivative can be obtained in step (d) by reacting the 2-$R_4$-substituted 4-oxo-5,6-diamino-pyrimidine with a monosubstituted glyoxal bearing the group $R_3$, wherein $R_3$ may be *inter alia* $C_{1-7}$ alkyl, $C_{3-10}$ cycloalkyl, aryl or heteroaryl, under neutral or basic conditions. Alternatively, a 2-$R_4$-substituted-4-oxo-6,7-disubstituted pteridine derivative can be obtained in step (e) by reacting the 2-$R_4$-substituted 4-oxo-5,6-diamino-pyrimidine with a disubstituted glyoxal bearing groups $R_2$ and $R_3$, wherein each of $R_2$ and $R_3$ is independently selected (i.e. $R_2$ and $R_3$ may be identical or different) from the group consisting of $C_{1-7}$ alkyl, $C_{3-10}$ cycloalkyl, aryl and heteroaryl, under neutral or basic conditions. Activation of the (tautomeric) hydroxyl substituent in position 4 of the pteridine ring for a nucleophilic displacement reaction occurs in step (f) by preparing the corresponding 4-[(1,2,4)-triazolyl] pteridine derivative, e.g. using $POCl_3$ or 4-chlorophenyl phosphoro-dichloridate and 1,2,4-triazole in pyridine as solvent. When $R_4$ is an amino group, protection of $R_4$ may further be necessary before carrying out this reaction. The amino group can be protected for instance by an acetyl group, which can be hydrolysed back to the amino group in a next step. Nucleophilic substitution is performed in step (g) by mixing the triazolyl pteridine derivative with a nucleophile having the general formula (IX) :

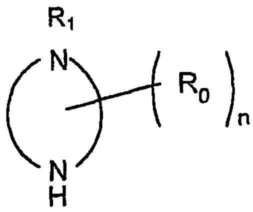
(IX)

wherein $R_0$ and n are as already defined above with respect to formula (II) and wherein $R_1$ is hydrogen or is as defined above with respect to formula (II), such as piperazine or an appropriate N-alkylpiperazine, N-arylpiperazine or N-alkylarylpiperazine, at room temperature in a polar aprotic solvent such as 1,4-dioxane. When piperazine is introduced in step (g), then in step (h), the second nitrogen atom of the piperazin-1-yl substituent in position 4 of the pteridine ring can be coupled with the desired carboxylic acid or thiocarboxylic acid chloride or sulfonyl chloride $R_1Cl$ at room temperature in a solvent such as pyridine.

Representative examples of commercially available N-alkylpiperazines, N-arylpiperazines and N-alkylarylpiperazines that can suitably be used in this method, as well as in some of the further methods described herein, include 1-cyclohexylpiperazine, 1-cyclopentylpiperazine, 1-(2,6-dichlorobenzyl)-piperazine, 1-(3,4-dichlorophenyl)-piperazine, 1-[2-(dimethylamino)-ethyl]-piperazine, 1-[3-(dimethylamino)-propyl]piperazine, 1-(3,4-dimethylphenyl)piperazine, 1-(2-ethoxyethyl)-piperazine, 1-isobutylpiperazine, 1-(1-methyl-piperidin-4-yl-methyl)-piperazine, 1-(2-nitro-4-trifluoromethylphenyl)-piperazine, 1-(2-phenoxyethyl)-piperazine, 1-(1-phenylethyl)-piperazine, 2-(piperazin-1-yl)-acetic acid ethyl ester, 2-(piperazin-1-yl)-acetic acid *N*-methyl-*N*-phenyl amide, 2-(piperazin-1-yl)-acetic acid *N*-(2-thiazolyl)-amide, 2-[2-(piperazin-1-yl)-ethyl]-1,3-dioxolan-3-(1-piperazinyl)propionitrile, 1-[(2-pyridyl)-methyl]piperazine and 1-thiazol-2-yl-piperazine.

Figure 2 schematically shows a second method for making trisubstituted pteridine derivatives having the formula (I) wherein $R_5$ is a group having the formula (II) and wherein $R_2$ or $R_3$ is hydrogen, as well as tetrasubstituted pteridine derivatives having the formula (I) wherein $R_5$ is a group having the formula (II). In step (a), the diazonium salt of p-chloroaniline is first formed by using sodium nitrite under aqueous acidic conditions and then reacted with a 2-$R_4$-substituted 4-chloro-6-amino-pyrimidine to yield an azo intermediate. In step (b), the chlorine atom in position 4 of the pyrimidinyl ring is replaced by nucleophile having the above general formula (IX) such as a piperazinyl group or an appropriate N-alkylpiperazinyl, N-arylpiperazinyl or N-alkylarylpiperazinyl group. Reductive cleavage of the azo compound then yields the corresponding 2-$R_4$-substituted 4-(piperazin-1-yl)-5,6-diaminopyrimidine in step (c). Condensation of the latter with an $\alpha$-ketoaldoxime bearing a radical $R_2$, wherein $R_2$ may be *inter alia* $C_{1-7}$ alkyl, $C_{3-10}$ cycloalkyl, aryl or heteroaryl, in a protic solvent such as methanol under acidic conditions leads in step (d) to the formation of a 2-$R_4$-substituted 4-(piperazin-1-yl)-6-$R_2$-substituted pteridine. Alternatively, a 2-$R_4$-substituted 4-(piperazin-1-yl)-7-$R_3$-substituted pteridine derivative can be obtained in step (e) by reacting the 2-$R_4$-substituted 4-(piperazin-1-yl)-5,6-diaminopyrimidine with a monosubstituted glyoxal bearing the group $R_3$, wherein $R_3$ may be *inter alia* $C_{1-7}$ alkyl, $C_{3-10}$ cycloalkyl, aryl or heteroaryl, under neutral or basic conditions. Alternatively, a 2-$R_4$-substituted-4-(piperazin-1-yl)-6,7-disubstituted pteridine derivative can be obtained in step (f) by reacting the 2-$R_4$-substituted 4-(piperazin-1-yl)-5,6-diamino-pyrimidine with a disubstituted glyoxal bearing groups $R_2$ and $R_3$, wherein each of $R_2$ and $R_3$ is independently selected (i.e. $R_2$ and $R_3$ may be identical or different) from the group consisting of $C_{1-7}$ alkyl, $C_{3-10}$ cycloalkyl, aryl and heteroaryl, under neutral or basic conditions. When a piperazinyl group is introduced at position 4 of the pyrimidine scaffold in step (b), then coupling of the second nitrogen atom of the piperazin-1-yl group with an acid or sulfonyl chloride $R_1Cl$ can occur in the last step (g) in the same way as in the first method above.

Figure 3 schematically shows a third method for making trisubstituted pteridine derivatives having the formula (I) wherein $R_5$ is a group having the formula (II) and wherein $R_2$ or $R_3$ is hydrogen, as well as tetrasubstituted pteridine derivatives having the formula (I) wherein $R_5$ is a group having the formula (II). This method starts from the pyrimidine derivative obtained after step (b) of the first method above. Formation of the pteridine ring occurs in step (b) through reaction of the said pyrimidine derivative with a suitable $\alpha$-ketoaldoxime bearing a radical $R_2$, wherein $R_2$ may be *inter alia* $C_{1-7}$ alkyl, $C_{3-10}$ cycloalkyl, aryl or heteroaryl, in a protic solvent such as methanol under acidic conditions. Alternatively, a 2-$R_4$-substituted 4-oxo-7-$R_3$-substituted pteridine derivative can be obtained in step (c) by reacting the 2-$R_4$-substituted 4-oxo-5,6-diaminopyrimidine with a monosubstituted glyoxal bearing the group $R_3$, wherein $R_3$ may be *inter alia* $C_{1-7}$ alkyl, $C_{3-10}$ cycloalkyl, aryl or heteroaryl, under neutral or basic conditions. Alternatively, a 2-$R_4$-substituted 4-oxo-6,7-disubstituted pteridine derivative can be obtained in step (d) by reacting the 2-$R_4$-substituted 4-oxo-5,6-diamino-pyrimidine with a disubstituted glyoxal bearing groups $R_2$ and $R_3$, wherein each of $R_2$ and $R_3$ is independently selected (i.e. $R_2$ and $R_3$ may be identical or different) from the group consisting of $C_{1-7}$ alkyl, $C_{3-10}$ cycloalkyl, aryl and heteroaryl, under neutral or basic conditions. A nucleophilic group such as piperazin-1-yl or N-alkylpiperazinyl, N-arylpiperazinyl or N-alkylarylpiperazinyl is then directly introduced, in step (e), at position 4 of the pteridine ring by reaction of the 2-$R_4$-substituted 4-oxopteridine with a nucleophile having the above general formula (IX) such as, but not limited to, piperazine or an appropriate N-alkylpiperazine, N-arylpiperazine or N-alkylarylpiperazine, and 1,1,1,3,3,3-hexamethyldisilazane as a reagent. When piperazine was used in step (e), then in step (f) coupling of the second nitrogen atom of the piperazin-1-yl group with an acid or sulfonyl chloride $R_1Cl$ can proceed in the same way as in the first method above.

Figure 4 schematically shows a fourth method for making trisubstituted pteridine derivatives having the formula (I) wherein $R_5$ is a group having the formula (II) and wherein $R_2$ or $R_3$ is hydrogen, as well as tetrasubstituted pteridine derivatives having the formula (I) wherein $R_5$ is a group having the formula (II). In this method, $R_4$ preferably is amino. In a first step (a), the chlorine atom at position 4 of the pyrimidine ring of a 2-$R_4$-substituted 4-chloro-6-aminopyrimidine is displaced by an appropriate $R_1$-*N*-monosubstituted piperazine wherein $R_1$ may be acyl, alkyl, aryl, alkylaryl or sulfonyl, thus e.g. yielding a novel polysubstituted 2,6-diaminopyrimidine having the general formula (VII) wherein n = 0, $R_7$ is hydrogen and the heterocyclic ring having the formula (III)

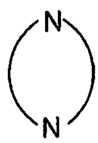

**(III)**

is for instance a piperazinyl group.

Many *N*-monosubstituted piperazines required for step (a) are commercially available, such as for instance:

- 1-(2-furoyl) piperazine,
- 1-(4-chlorobenzenesulfonyl) piperazine ,
- 1-(4-fluorobenzoyl) piperazine,
- 1-(4-methoxyphenylsulfonyl) piperazine,
- 1-(ethoxycarbonyl) piperazine,
- 1-(tetrahydro-2-furoyl) piperazine,
- 1-(thien-2-ylcarbonyl) piperazine,
- 1-[(2,3-dihydro-1,4-benzodioxin-2-yl)carbonyl] piperazine,
- 1-acetylpiperazine,
- 1-formylpiperazine, and
- 1-methanesulfonylpiperazine.

N-acyl-, N-thioacyl- or N-sulfonyl- monosubstituted piperazines which are not commercially available may easily be prepared by reacting piperazine with any commercially available carboxylic acid chloride, thiocarboxylic acid chloride

or sulfonyl chloride under standard acylation, thioacylation or sulfonylation conditions .

The method shown in figure 4 is also applicable while starting, in the first step (a), from an appropriate $R_1$-monosubstituted homopiperazine wherein $R_1$ may be acyl or sulfonyl, thus e.g. yielding a novel polysubstituted 2,6-diaminopyrimidine having the general formula (VII) wherein n = 0, $R_7$ is hydrogen and the heterocyclic ring having the formula (III) is a homopiperazinyl group. Commercially available monosubstituted homopiperazines required for such step (a) are, for instance, N-acetylhomopiperazine, 1-[3-chloro-5-(trifluoromethyl)-2-pyridyl]-homopiperazine, 1-[4-(trifluoromethyl)pyrimi-din-2-yl]-homopiperazine, 1-(4-fluorobenzyl)-homopiperazine, 1-(2-chloro-6-fluoro-benzyl)-homopiperazine, 1-(5-nitro-2-pyridyl)-homopiperazine and (5-isoquinoline-sulfonyl)-homopiperazine. *N*-acyl-, *N*-thioacyl- or *N*-sulfonyl-monosubstituted homopiperazines which are not commercially available may easily be prepared by reacting homopiperazine with any commercially available carboxylic acid chloride, thiocarboxylic acid chloride or sulfonyl chloride under standard acylation, thioacylation or sulfonylation conditions.

Introduction of a nitroso group at position 5 of the pyrimidine ring occurs in step (b) under aqueous acidic conditions in the presence of sodium nitrite, thus e.g. yielding a novel polysubstituted 2,6-diaminopyrimidine having the general formula (VII) wherein n = 0, $R_7$ is nitroso and the heterocyclic ring having the formula (III):

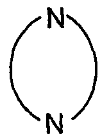

(III)

is for instance a piperazinyl group (as shown in figure 4) or a homopiperazinyl group (not shown in figure 4).

Reduction of the nitroso functionality of this intermediate into a free amino group is then effected in step (c) by means of reducing agents such as $Na_2S_2O_4$ or $(NH_4)_2S$ in water, or catalytically ($Pt/H_2$) in the presence of a protic solvent, thus e.g. yielding a novel polysubstituted 2,5,6-triaminopyrimidine having the general formula (VII) wherein n = 0, $R_7$ is amino and the heterocyclic ring having the general formula (III)

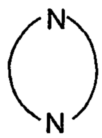

(III)

is for instance a piperazinyl group (as shown in figure 4) or a homopiperazinyl group (not shown in figure 4).

In order to regioselectively obtain a 2,4,6-trisubstituted pteridine derivative, the substituted 5,6-diaminopyrimidine is then reacted in step (d) with an $\alpha$-ketoaldoxime bearing the group $R_2$, wherein $R_2$ may be *inter alia* $C_{1-7}$ alkyl, $C_{3-10}$ cycloalkyl, aryl or heteroaryl, under acidic conditions in the presence of a solvent such a methanol. Alternatively, a 2,4,7-trisubstituted pteridine derivative can be obtained in step (f) by reacting the substituted 5,6-diaminopyrimidine with a monosubstituted glyoxal bearing the group $R_3$, wherein $R_3$ may be *inter alia*, $C_{1-7}$ alkyl, $C_{3-10}$ cycloalkyl, aryl or heteroaryl, under neutral or basic conditions. Alternatively, a 2,4,6,7-tetrasubstituted pteridine derivative can be obtained in step (e) by reacting the substituted 5,6-diaminopyrimidine with a disubstituted glyoxal bearing the groups $R_2$ and $R_3$, wherein each of $R_2$ and $R_3$ is independently selected (i.e. $R_2$ and $R_3$ may be identical or different) from the group consisting of $C_{1-7}$ alkyl, $C_{3-10}$ cycloalkyl, aryl and heteroaryl, under neutral or basic conditions.

Figure 5 schematically shows a fifth method for making trisubstituted pteridine derivatives having the formula (I) wherein $R_5$ is a group having the formula (II) and wherein $R_3$ is hydrogen. In particular, figure 5 shows a scheme making use of a coupling compound such as a carboxylic or sulfonic acid having the general formula $R_{11}ZOOH$, wherein Z is selected from the group consisting of the carbon atom and the SO group, and wherein $R_{11}$ is a group selected from the group consisting of $C_{1-7}$ alkyl, $C_{3-10}$ cycloalkyl, aryl, heteroaryl and alkylaryl, wherein the said group $R_{11}$ is optionally substituted with one or more substituents selected from the group consisting of halogen, amino and protected amino groups, and wherein the said substituent may be at any position (such as the $\alpha$ position or the $\omega$ position) with respect to the carboxylic or sulfonic acid group. In particular, the said coupling compound may be a carboxylic acid $R_{11}CO_2H$ or a sulfonic acid $R_{11}SO_3H$ or a preferably amino-protected naturally-occurring amino-acid (e.g. L-alanine, L-phenylalanine, L-tyrosine, L-proline, L-tryptophane, L-leucine, L-isoleucine, L-lysine, L-valine, glycine, L-histidine, L-serine, L-arginine, L-aspartic acid, L-cysteine or L-glutamine) or synthetic non naturally-occurring amino-acid. This method provides coupling, in one single step (a), of the said compound to the second nitrogen atom of a heterocyclic ring having the general formula (III) and being substituted at position 4 of a pteridine ring. That is, this method starts for instance

from a pteridine intermediate such as obtained after step (g) of the scheme shown in figure 1, or after any of steps (d), (e) and (f) of the scheme shown in figure 2, or after step (e) of the scheme shown in figure 3. When the coupling compound is a carboxylic acid $R_{11}CO_2H$, the said pteridine intermediate is reacted with the coupling compound preferably in an aprotic solvent, such as dimethyl-formamide or dichloromethane or mixtures thereof, and in the presence of a suitable coupling reagent, such as 1,3-dicyclohexylcarbodiimide or 1,3-diisopropylcarbodiimide or diisopropylethyl-amine or o-benzotriazol-1-yl-N,N,N',N'-tetramethyluronium tetrafluoroborate. As is usual for this kind of coupling reaction, all free amino groups of the amino-acids are most preferably protected before carrying out the coupling reaction. When the amino-acid has two or more amino groups, the protecting groups for the said amino groups may be the same or different. A few examples of amino-protecting groups are benzyloxycarbonyl (which may be introduced by reaction of the desired amino-acid with benzylchloroformate under alcaline conditions, e.g. making use of sodium hydroxide or hydrogenocarbonate) and 9-fluorenylmethoxycarbonyl (which may be introduced by reaction of the desired amino-acid with 9-fluorenylmethyl chloroformate). Another example of an amino-protecting group is a tert-butoxycarbonyl group which may be introduced by reaction of the desired amino-acid with di-tert-butyl dicarbonate under alcaline conditions. Other suitable amino-protecting groups include triphenylmethyl (trityl) and trifluoroacetyl groups. First, the amino-protected amino-acid is coupled to the second nitrogen atom of the heterocyclic ring e.g. by using any method conventional in peptide synthesis. Finally, in order to afford for instance the desired (4-substituted-piperazin-1-yl) or (4-substituted-homopiperazin-1-yl) pteridine derivative, the amino-protecting group is removed by deprotection methods conventional in the art, such as:

- when the amino-protecting group is a phenylmethoxycarbonyl group, cleavage of the benzylic ether function by hydrogenolysis, e.g. using $H_2$, Pd-C at about 25°C, or under strongly acidic conditions (e.g. making use of bromhydric acid), or
- when the amino-protecting group is a tert-butoxycarbonyl group, by treatment with an acid, e.g. using aqueous hydrochloric acid or trifluoroacetic acid, under conditions mild enough to avoid further cleavage of the molecule, or
- when the amino-protecting group is a 9-fluorenylmethoxycarbonyl group, by treatment with a base such as piperidine.

Figure 6 schematically shows a sixth method for making trisubstituted pteridine derivatives having the formula (I) wherein $R_5$ is a group having the formula (II) and wherein $R_3$ or $R_2$ is hydrogen, as well as tetrasubstituted pteridine derivatives having the formula (I) wherein $R_5$ is a group having the formula (II). In step (a), the thiol function of 2-mercapto-4,6-diaminopyrimidine is alkylated, preferably methylated by reaction with methyl iodide in the presence of a solvent such as ethanol, in order to yield 2-thiomethyl-4,6-diaminopyrimidine. Introduction of a nitroso group in the 5-position of the pyrimidine ring is then achieved in step (b) by using sodium nitrite under aqueous acidic conditions. In step (c), the methylthio group in the 2-position is exchanged for a group $R_4$ by reaction with an appropriate nucleophile, wherein $R_4$ is as defined above and preferably is primary or secondary amino, $C_{1-7}$ alkoxy, aryloxy. $C_{3-10}$ cycloalkoxy, heteroaryloxy, mercapto $C_{1-7}$alkyl, mercaptoaryl, mercapto $C_{3-10}$cycloalkyl or mercapto-heteroaryl. Reduction of the nitroso group is then achieved in step (d) either catalytically ($Pt/H_2$) in the presence of a protic solvent or chemically using sodium dithionite or ammonium sulfide in the presence of water. Then in step (e), the resulting 2-$R_4$-substituted-4,5,6-triaminopyrimidine is condensed, under acidic condi-tions in the presence of a solvent such as methanol, with an $\alpha$-ketoaldoxime bearing the group $R_2$, wherein $R_2$ may be $C_{1-7}$ alkyl, $C_{3-10}$ cycloalkyl, aryl or heteroaryl, into a 2,6-substituted-4-aminopteridine derivative. Alternatively, the corresponding 2,7-substituted-4-aminopteridine derivative can be obtained in step (f) by reacting the 2-$R_4$-substituted-4,5,6-triaminopyrimidine with a monosubstituted glyoxal bearing a group $R_3$, wherein $R_3$ may be inter alia $C_{1-7}$ alkyl, $C_{3-10}$ cycloalkyl, aryl or heteroaryl. Alternatively, a 2-$R_4$-substituted 4-amino-6,7-disubstituted pteridine derivative can be obtained in step (g) by reacting the 2-$R_4$-substituted 4,5,6-triaminopyrimidine with a disubstituted glyoxal bearing groups $R_2$ and $R_3$, wherein each of $R_2$ and $R_3$ is independently selected (i.e. $R_2$ and $R_3$ may be identical or different) from the group consisting of $C_{1-7}$ alkyl, $C_{3-10}$ cycloalkyl, aryl and heteroaryl, under neutral or basic conditions. In step (h), acidic or basic hydrolysis of the amino group at position 4 of the pteridine ring is performed and results in the corresponding 4-oxopteridine derivative. In step (i), the hydroxyl group of the tautomeric form of the latter is activated by nucleophilic displacement, e.g. by preparing the 4-[(1,2,4)-triazolyl] pteridine derivative. Finally in a first part of step (j), a nucleophilic displacement is performed by mixing the said 4-triazolylpteridine derivative with a nucleophile having the above general formula (IX).

When this nucleophile, and optionally also the nucleophile used in step (c), has a heterocyclic ring containing at least two nitrogen atoms, the second nitrogen atom of each heterocyclic ring can be acylated, thioacylated or sulfonylated in a last part of step (j) by treating the intermediate with an appropriate carboxylic acid chloride, thiocarboxylic acid chloride or sulfonyl chloride $R_1Cl$ in a aprotic solvent such as dimethyl-formamide, pyridine or dichloromethane and, if necessary, in the presence of a base such as a tertiary amine (e.g. triethylamine).

Figure 7 schematically shows a seventh method for making trisubstituted pteridine derivatives having the formula (I) wherein $R_5$ is a group having the formula (II) and wherein $R_3$ or $R_2$ is hydrogen, as well as tetrasubstituted pteridine derivatives having the formula (I) wherein $R_5$ is a group having the formula (II), starting from the 2-thiomethyl-5-nitroso-4,6-diaminopyrimidine obtained after step (b) of the scheme shown in figure 6. Reduction of the nitroso group

is achieved in step (a) either catalytically ($Pt/H_2$) in the presence of a protic solvent or chemically using sodium dithionite or ammonium sulfide in the presence of water. Then in step (b), 2-thiomethyl-4,5,6-triaminopyrimidine is condensed, under acidic conditions in the presence of a solvent such as methanol, with an α-ketoaldoxime bearing the group $R_2$, wherein $R_2$ may be *inter alia* $C_{1-7}$ alkyl, $C_{3-10}$ cycloalkyl, aryl or heteroaryl, thus regioselectively yielding a 2-thiomethyl-4-amino-6-$R_2$-substituted-pteridine derivative. Alternatively, the corresponding 2-thiomethyl-4-amino-7-$R_3$-substituted pteridine is obtained in step (c) by reacting 2-thiomethyl-4,5,6-triamino-pyrimidine with a monosubstituted glyoxal bearing a group $R_3$, wherein $R_3$ may be *inter alia* $C_{1-7}$ alkyl, $C_{3-10}$ cycloalkyl, aryl or heteroaryl. Alternatively, the corresponding 2-thiomethyl-4-amino-6-$R_2$-7-$R_3$-substituted pteridine is obtained in step (d) by reacting 2-thiomethyl-4,5,6-triamino-pyrimidine with a disubstituted glyoxal bearing groups $R_2$ and $R_3$, wherein each of $R_2$ and $R_3$ is independently selected (i.e. $R_2$ and $R_3$ may be identical or different) from the group consisting of $C_{1-7}$ alkyl, $C_{3-10}$ cycloalkyl, aryl and heteroaryl, under neutral or basic conditions. In step (e), the methylthio group in the 2-position is oxidized to the corresponding sulfone by using oxidizing agents such as chloroperoxybenzoic acid in chloroform or hydrogen peroxide in acetic acid. The methylsulfonyl group is easily exchanged in step (f) by reaction with a nucleophile having the general formula (IX) above. When this nucleophile has an heterocyclic ring containing at least two nitrogen atoms, the second nitrogen atom of said heterocyclic ring can be acylated, thioacylated or sulfonylated in step (f) by treatment with an appropriate carboxylic acid chloride, thiocarboxylic acid chloride or sulfonyl chloride $R_1Cl$ in a aprotic solvent such as dimethylformamide, pyridine or dichloromethane and, if necessary, in the presence of a base such as a tertiary amine (e.g. triethylamine). In step (g), acidic or basic hydrolysis of the amino group at position 4 of the pteridine ring is performed and results in the corresponding 4-oxopteridine derivative. In step (h), the hydroxyl group of the tautomeric form of the latter is activated by nucleophilic displacement, e.g. by preparing the 4-[(1,2,4)-triazolyl] pteridine derivative. Finally in a first part of step (i), a nucleophilic displacement is performed by mixing the said 4-triazolylpteridine derivative with a nucleophile having the general formula (IX) above. When this nucleophile has a heterocyclic ring containing at least two nitrogen atoms, the second nitrogen atom of each heterocyclic ring can be acylated or sulfonylated in a last part of step (i) by treating the intermediate with an appropriate acid chloride or sulfonyl chloride $R_1Cl$ in a aprotic solvent such as dimethylformamide, pyridine or dichloromethane and, if necessary, in the presence of a base such as a tertiary amine (e.g. triethylamine).

Figure 8 (reference) schematically shows a method for making trisubstituted (wherein $R_2$ or $R_3$ is hydrogen) and tetrasubstituted pteridine derivatives having the formula (I) wherein $R_4$ and $R_5$ are identical groups having the formula (II). In step (a), a nitro group is introduced in position 5 of a 6-amino-2,4-dioxopyrimidine under strongly acidic conditions (e.g. $HNO_3$, $H_2SO_4$). Then, in step (b), both hydroxyl groups from the tautomeric form are converted to chloro groups by treatment with a chlorinating agent such as $POCl_3$ or $SOCl_2$. Both chloro groups are then displaced in step (c) with a nucleophile having the above general formula (IX), thus yielding novel 6-aminopyrimidines having the general formula (IV) wherein $R_1$ is hydrogen and $R_6$ is nitro. The nitro group of the latter is then reduced in step (d) to an amino group by treatment with a reducing agent (e.g. $Pt/H_2$), thus yielding novel 6-aminopyrimidines having the general formula (IV) wherein $R_1$ is hydrogen and $R_6$ is amino. Finally, reaction of the latter with an α-ketoaldoxime bearing the group $R_2$, wherein $R_2$ may be *inter alia* aryl, $C_{1-7}$ alkyl, $C_{1-10}$ cycloalkyl or heteroaryl, regioselectively yields the desired 2,4,6-trisubstituted pteridine derivative in step (e). Alternatively, reaction of the 2,4-substituted-5,6-diaminopyrimidine from step (d) with a monosubstituted glyoxal bearing a group $R_3$ wherein $R_3$ may be *inter alia* $C_{1-7}$ alkyl, $C_{3-10}$ cycloalkyl, aryl or heteroaryl yields the desired 2,4,7-trisubstituted pteridine derivative in step (f). Alternatively, reaction of the 2,4-substituted-5,6-diaminopyrimidine from step (d) with a disubstituted glyoxal bearing groups $R_2$ and $R_3$, wherein each of $R_2$ and $R_3$ is independently selected (i.e. $R_2$ and $R_3$ may be identical or different) from the group consisting of $C_{1-7}$ alkyl, $C_{3-10}$ cycloalkyl, aryl and heteroaryl, under neutral or basic conditions, yields the desired 2,4,6,7-tetrasubstituted pteridine derivative in step (g). Finally, when the nucleophile used in step (c) has a heterocyclic ring containing at least two nitrogen atoms, the second nitrogen atom can be acylated, thioacylated or sulfonylated in a last step (not shown in the figure) by treatment with an appropriate carboxylic acid chloride, thiocarboxylic acid chloride or sulfonyl chloride $R_1Cl$ in a aprotic solvent such as dimethylformamide, pyridine or dichloromethane and, if necessary, in the presence of a base such as a tertiary amine (e.g. triethylamine).

Figure 9 schematically shows a method for making trisubstituted and tetrasubstituted pteridine derivatives having the formula (I) wherein $R_5$ is a piperazine substituted in position 4 with a ureido group. In the first step (a), a *N*-alkyl substituted aniline derivative is reacted with the commercially available *N,N*-carbonyldiimidazole using a polar aprotic solvent such as tetrahydrofuran or 1,4-dioxane. The reactivity of the carbamoyl imidazole was then increased by *N*-alkylation of the imidazole moiety in step (b) by reaction of *N*-alkyl aniline carbamoyl imidazole with an appropriate alkyl halide, such as, for example methyl iodide, leading to the formation of the corresponding imidazolium salt. Subsequent addition, in step (c), of a 4-*N*-piperazinopteridine (optionally substituted in positions 2 and/or 6 and/or 7) in an aprotic solvent such as dimethylformamide, pyridine or dichloromethane and, if necessary, in the presence of a base such as a tertiary amine (e.g. triethylamine) yields the desired substituted pteridine derivative.

In another particular embodiment, the invention relates to a group of pteridine derivatives, as well as pharmaceutical compositions comprising such pteridine derivatives as active principle, having the above general formula (I) and being in the form of a pharmaceutically acceptable salt. The latter include any therapeutically active non-toxic addition

salt which compounds having the general formula (I) are able to form with a salt-forming agent. Such addition salts may conveniently be obtained by treating the pteridine derivatives of the invention with an appropriate salt-forming acid or base. For instance, pteridine derivatives having basic properties may be converted into the corresponding therapeutically active, non-toxic acid addition salt form by treating the free base form with a suitable amount of an appropiate acid following conventional procedures. Examples of such appropriate salt-forming acids include, for instance, inorganic acids resulting in forming salts such as hydrohalides (e.g. hydrochloride and hydrobromide), sulfate, nitrate, phosphate, diphosphate, carbonate, bicarbonate, and organic monocarboxylic or dicarboxylic acids resulting in forming salts such as, for example, acetate, propanoate, hydroxyacetate, 2-hydroxypropanoate, 2-oxopropanoate, lactate, pyruvate, oxalate, malonate, succinate, maleate, fumarate, malate, tartrate, citrate, methanesulfonate, ethanesulfonate, benzoate, 2-hydroxybenzoate, 4-amino-2-hydroxybenzoate, benzene-sulfonate, p-toluenesulfonate, salicylate, p-aminosalicylate, pamoate, bitartrate, camphorsulfonate, edetate, 1,2-ethanedisulfonate, fumarate, glucoheptonate, gluconate, glutamate, hexylresorcinate, hydroxynaphtoate, hydroxyethanesulfonate, mandelate, methylsulfate, pantothenate, stearate, as well as salts derived from ethanedioic, propanedioic, butanedioic, (Z)-2-butenedioic, (E)2-butenedioic, 2-hydroxybutanedioic, 2,3-dihydroxybutane-dioic, 2-hydroxy-1,2,3-propanetricarboxylic and cyclohexanesulfamic acids.

Pteridine derivatives of the general formula (I) having acidic properties may be converted in a similar manner into the corresponding therapeutically active, non-toxic base addition salt form. Examples of appropriate salt-forming bases include, for instance, inorganic bases like metallic hydroxides such as those of alkali and alkaline-earth metals like calcium, lithium, magnesium, potassium and sodium, or zinc, resulting in the corresponding metal salt; organic bases such as ammonia, alkylamines, benzathine, hydrabamine, arginine, lysine, N,N'-dibenzylethylenediamine, chloroprocaine, choline, diethanolamine, ethylene-diamine, N-methylglucamine, procaine.

Reaction conditions for treating the pteridine derivatives having the general formula (I) of this invention with an appropriate salt-forming acid or base are similar to standard conditions involving the same acid or base but different organic compounds with basic or acidic properties, respectively. Preferably, in view of its use in a pharmaceutical composition or in the manufacture of medicament for treating specific diseases, the pharmaceutically acceptable salt will be designed, i.e. the salt-forming acid or base will be selected so as to impart greater water-solubility, lower toxicity, greater stability and/or slower dissolution rate to the pteridine derivative of this invention.

The pteridine derivatives represented by the general formula (I), or a pharmaceutically acceptable salt or a solvate thereof can be used as a biologically-active ingredient, i.e. a medicine or for the manufacture of a medicament. In particular the said medicament may be for the prevention or treatment of a pathologic condition selected from the group consisting of:

- immune disorders, in particular organ and cells transplant rejections, and autoimmune disorders,
- cardiovascular disorders,
- allergic conditions,
- disorders of the central nervous system,
- TNF-$\alpha$-related disorders,
- viral diseases, and
- cell proliferative disorders.

The pathologic conditions and disorders concerned by the said use are detailed hereinbelow. Any of the uses mentioned may be restricted to a non-medical use (e.g. in a cosmetic composition), a non-therapeutic use, a non-diagnostic use, a non-human use (e.g. in a veterinary composition), or exclusively an *in-vitro* use, or a use with cells remote from an animal.

The invention further relates to a pharmaceutical composition comprising:

(a) one or more pteridine derivatives represented by the general formula (I) and
(b) one or more pharmaceutically acceptable carriers.

In a further embodiment, this invention provides combinations, preferably synergistic combinations, of one or more pteridine derivative represented by the general formula (I) with one or more biologically-active drugs being selected from the group consisting of immunosuppressant and/or immunomodulator drugs, antineoplastic drugs and antiviral agents. As is conventional in the art, the evaluation of a synergistic effect in a drug combination may be made by analyzing the quantification of the interactions between individual drugs, using the median effect principle described by Chou et al. in *Adv. Enzyme* Reg. (1984) 22:27. Briefly, this principle states that interactions (synergism, additivity, antagonism) between two drugs can be quantified using the combination index (hereinafter referred as CI) defined by the following equation:

$$CI_x = \frac{ED_x^{1c}}{ED_x^{1a}} + \frac{ED_x^{2c}}{ED_x^{2a}}$$

wherein $ED_x$ is the dose of the first or respectively second drug used alone (1a, 2a), or in combination with the second or respectively first drug (1 c, 2c), which is needed to produce a given effect. The said first and second drug have synergistic or additive or antagonistic effects depending upon $CI < 1$, $CI = 1$, or $CI > 1$, respectively. As will be explained in more detail herein-below, this principle may be applied to a number of desirable effects such as an activity against transplant rejection, an activity against immunosuppression or immunomodulation, an activity against allergy or an activity against cell proliferation.

For instance the present invention relates to a pharmaceutical composition or combined preparation having synergistic effects against immuno-suppression or immunomodulation and containing:

(a) one or more immunosuppressant and/or immunomodulator drugs, and
(b) at least one pteridine derivative represented by the general formula (I) and
(c) optionally one or more pharmaceutical excipients or pharmaceutically acceptable carriers, for simultaneous, separate or sequential use in the treatment or prevention of autoimmune disorders and/or in transplant-rejections.

Suitable immunosuppressant drugs for inclusion in the synergistic compositions or combined preparations of this invention belong to a well known therapeutic class. They are preferably selected from the group consisting of cyclosporin A, substituted xanthines (e.g. methylxanthines such as pentoxyfylline), daltroban, sirolimus, tacrolimus, rapamycin (and derivatives thereof such as defined below), leflunomide (or its main active metabolite A771726, or analogs thereof called malononitrilamides), mycophenolic acid and salts thereof (including the sodium salt marketed under the trade name Mofetil®), adrenocortical steroids, azathioprine, brequinar, gusperimus, 6-mercaptopurine, mizoribine, chloroquine, hydroxychloroquine and monoclonal antibodies with immunosuppressive properties (e.g. etanercept, infliximab or kineret). Adrenocortical steroids within the meaning of this invention mainly include glucocorticoids such as ciprocinonide, desoxycorticisterone, fludrocortisone, flumoxonide, hydrocortisone, naflocort, procinonide, timobesone, tipredane, dexamethasone, methylprednisolone, methotrexate, prednisone, prednisolone, triamcinolone and pharmaceutically acceptable salts thereof. Rapamycin derivatives as referred herein include O-alkylated derivatives, particularly 9-deoxorapamycins, 26-dihydrorapamycins, 40-O-substituted rapamycins and 28,40-0,0-disubstituted rapamycins (as disclosed in U.S. Patent No. 5,665,772) such as 40-0-(2-hydroxy) ethyl rapamycin - also known as SDZ-RAD -, pegylated rapamycin (as disclosed in U.S. Patent No. 5,780,462), ethers of 7-desmethylrapamycin (as disclosed in U.S. Patent No. 6,440,991) and polyethylene glycol esters of SDZ-RAD (as disclosed in U.S. Patent No. 6,331,547).

Suitable immunomodulator drugs for inclusion into the synergistic immunomodulating pharmaceutical compositions or combined preparations of this invention are preferably selected from the group consisting of acemannan, amiprilose, bucillamine, ditiocarb sodium, imiquimod, Inosine Pranobex, interferon-β, interferon-γ, lentinan, levamisole, pidotimod, romurtide, platonin, procodazole, propagermanium, thymomodulin, thymopentin and ubenimex.

Synergistic activity of the pharmaceutical compositions or combined preparations of this invention against immunosuppression or immuno-modulation may be readily determined by means of one or more lymphocyte activation tests. Usually activation is measured via lymphocyte proliferation. Inhibition of proliferation thus always means immunosuppression under the experimental conditions applied. There exist different stimuli for lymphocyte activation, in particular:

a) co-culture of lymphocytes of different species (mixed lymphocyte reaction, hereinafter referred as MLR) in a so-called mixed lymphocyte culture test: lymphocytes expressing different minor and major antigens of the HLA-DR type (= alloantigens) activate each other non-specifically;
b) a CD3 assay wherein there is an activation of the T-lymphocytes via an exogenously added antibody (OKT3). This antibody reacts against a CD3 molecule located on the lymphocyte membrane which has a co-stimulatory function. Interaction between OKT3 and CD3 results in T-cell activation which proceeds via the $Ca^{2+}$/calmodulin/calcineurin system and can be inhibited e.g. by cyclosporin A (hereinafter referred as CyA);
c) a CD28 assay wherein specific activation of the T-lymphocyte proceeds via an exogenously added antibody against a CD28 molecule which is also located on the lymphocyte membrane and delivers strong co-stimulatory signals. This activation is $Ca^{2+}$-independent and thus cannot be inhibited by CyA.

Determination of the immunosuppressing or immunomodulating activity of the pteridine derivatives of this invention, as well as synergistic combinations comprising them, is preferably based on the determination of one or more, preferably at least three lymphocyte activation *in vitro* tests, more preferably including at least one of the MLR test, CD3

assay and CD28 assay referred above. Preferably the lymphocyte activation *in vitro* tests used include at least two assays for two different clusters of differentiation preferably belonging to the same general type of such clusters and more preferably belonging to type I transmembrane proteins. Optionally the determination of the immuno-suppressing or immunomodulating activity may be performed on the basis of other lymphocyte activation *in vitro* tests, for instance by performing a TNF-α assay or an IL-1 assay or an IL-6 assay or an IL-10 assay or an IL-12 assay or an assay for a cluster of differentiation belonging to a further general type of such clusters and more preferably belonging to type II transmembrane proteins such as CD69, CD 71 or CD134.

The synergistic effect may be evaluated by the median effect analysis method described herein-before. Such tests may for instance, according to standard practice in the art, involve the use of equiment, such as flow cytometer, being able to separate and sort a number of cell subcategories at the end of the analysis, before these purified batches can be analysed further.

Synergistic activity of the pharmaceutical compositions of this invention in the prevention or treatment of transplant rejection may be readily determined by means of one or more leukocyte activation tests performed in a Whole Blood Assay (hereinafter referred as **WBA)** described for instance by Un et al. in Transplantation (1997) 63:1734-1738. WBA used herein is a lymphoproliferation assay performed *in vitro* using lymphocytes present in the whole blood, taken from animals that were previously given the pteridine derivative, and optionally the other immunosuppressant drug, *in vivo.* Hence this assay reflects the *in vivo* effect of substances as assessed by an *in vitro* read-out assay. The synergistic effect may be evaluated by the median effect analysis method described herein-before. Various organ transplantation models in animals are also available *in vivo,* which are strongly influenced by different immunogenicities, depending on the donor and recipient species used and depending on the nature of the transplanted organ. The survival time of transplanted organs can thus be used to measure the suppression of the immune response.

The pharmaceutical composition or combined preparation with synergistic activity against immunosuppression or immunomodulation according to this invention may contain the pteridine derivative of formula (I) over a broad content range depending on the contemplated use and the expected effect of the preparation. Generally, the pteridine derivative content of the combined preparation is within the range of 0.1 to 99.9% by weight, preferably from 1 to 99% by weight, more preferably from 5 to 95% by weight.

The invention further relates to a composition or combined preparation having synergistic effects against cell proliferation and containing:

(a) one or more antineoplastic drugs, and
(b) at least one pteridine derivative represented by the general formula (I) and
(c) optionally one or more pharmaceutical excipients or pharmaceutically acceptable carriers, for simultaneous, separate or sequential use in the treatment or prevention of cell proliferative disorders.

Suitable antineoplastic drugs for inclusion into the synergistic antiproliferative pharmaceutical compositions or combined preparations of this invention are preferably selected from the group consisting of alkaloids, alkylating agents (including alkyl sulfonates, aziridines, ethylenimines, methylmelamines, nitrogen mustards and nitrosoureas), antibiotics, antimetabolites (including folic acid analogs, purine analogs and pyrimidine analogs), enzymes, interferon and platinum complexes. More specific examples include acivicin; aclarubicin; acodazole; acronine; adozelesin; aldesleukin; altretamine; ambomycin; ametantrone; aminoglutethimide; amsacrine; anastrozole; anthramycin; asparaginase; asperlin; azacitidine; azetepa; azotomycin; batimastat; benzodepa; bicalutamide; bisantrene; bisnafide; bizelesin; bleomycin; brequinar; bropirimine; busulfan; cactinomycin; calusterone; caracemide; carbetimer; carboplatin; carmustine; carubicin; carzelesin; cedefingol; chlorambucil; cirolemycin; cisplatin; cladribine; crisnatol; cyclophosphamide; cytarabine; dacarbazine; dactinomycin; daunorubicin; decitabine; dexormaplatin; dezaguanine; diaziquone; docetaxel; doxorubicin; droloxifene; dromostanolone; duazomycin; edatrexate; eflomithine; elsamitrucin; enloplatin; enpromate; epipropidine; epirubicin; erbulozole; esorubicin; estramustine; etanidazole; ethiodized oil 131; etoposide; etoprine; fadrozole; fazarabine; fenretinide; floxuridine; fludarabine; fluorouracil; flurocitabine; fosquidone; fostriecin; gemcitabine; Gold 198; hydroxyurea; idarubicin; ifosfamide; ilmofosine; interferon α-2a; interferon α-2b; interferon α-n1; interferon α-n3; interferon β-1a; interferon γ-1b; iproplatin; irinotecan; lanreotide; letrozole; leuprolide; liarozole; lometrexol; lomustine; losoxantrone; masoprocol; maytansine; mechlorethamine; megestrol; melengestrol; melphalan; menogaril; mercaptopurine; methotrexate; metoprine; meturedepa; mitindomide; mitocarcin; mitocromin; mitogillin; mitomalcin; mitomycin; mitosper; mitotane; mitoxantrone; mycophenolic acid; nocodazole; nogala-mycin; ormaplatin; oxisuran; paclitaxel; pegaspargase; peliomycin; pentamustine; peplomycin; perfosfamide; pipobroman; piposulfan; piroxantrone; plicamycin; plomestane; porfimer; pofiromycin; prednimustine; procarbazine; puromycin; pyrazofurin; riboprine; rogletimide; safingol; semustine; simtrazene; sparfosate; sparsomycin; spirogermanium; spiromustine; spiroplatin; streptonigrin; streptozocin; strontium 89 chloride; sulofenur; talisomycin; taxane; taxoid; tecogalan; tegafur; teloxantrone; temoporfin; teniposide; teroxirone; testolactone; thiamiprine; thioguanine; thiotepa; tiazofurin; tirapazamine; topotecan; toremifene; trestolone; triciribine; trimetrexate; triptorelin; tubulozole; uracil mustard; uredepa; vapreotide; verteporfin; vinblastine; vincristine; vindesine; vinepidine;

vinglycinate; vinleurosine; vinorelbine; vinrosidine; vinzolidine; vorozole; zeniplatin; zinostatin; zorubicin; and their pharmaceutically acceptable salts.

Other suitable anti-neoplastic compounds include 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogens such as benorterone, cioteronel, cyproterone, delmadinone, oxendolone, topterone, zanoterone and their pharmaceutically acceptable salts; antiestrogens such as clometherone; delmadinone; nafoxidine; nitromifene; raloxifene; tamoxifen; toremifene; trioxifene and their pharmaceutically acceptable salts; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin; azasetron; azatoxin; azatyrosine; baccatin III derivatives; balanol; batimastat; BCR/ABL antagonists; benzochlorins; benzoylstaurosporine; β-lactam derivatives; β-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors; castanospermine; cecropin B; cetrorelix; chlorins; chloroquinoxaline sulfonamide; cicaprost; cis-porphyrin; clomifene and analogues thereof; clotrimazole; collismycin A and B; combretastatin and analogues thereof; conagenin; crambescidin 816; cryptophycin and derivatives thereof; curacin A; cyclopentanthraquinones; cycloplatam; cypemycin; cytarabine; cytolytic factor; cytostatin; dacliximab; dehydrodidemnin B; deslorelin; dexifosfamide; dexrazoxane; dexverapamil; didemnin B; didox; diethylnorspermine; dihydro-5-azacytidine; dihydrotaxol; dioxamycin; diphenyl spiromustine; docosanol; dolasetron; doxifluridine; droloxifene; dronabinol; duocarmycin SA; ebselen; ecomustine; edelfosine; edrecolomab; elemene; emitefur; epristeride; estrogen agonists and antagonists; exemestane; filgrastim; finasteride; flavopiridol; flezelastine; fluasterone; fluorodaunorunicin; forfenimex; formestane; fotemustine; gadolinium texaphyrin; gallium nitrate; galocitabine; ganirelix; gelatinase inhibitors; glutathione inhibitors; hepsulfam; heregulin; hexamethylene bisacetamide; hypericin; ibandronic acid; idoxifene; idramantone; ilomastat; imidazoacridones; imiquimod; immunostimulant peptides; insulin-like growth factor-1 receptor inhibitor, interferon agonists; iobenguane; iododoxorubicin; ipomeanol; irinotecan; iroplact; irsogladine; isobengazole; isohomohalicondrin B; itasetron; jasplakinolide; kahalalide F; lamellarin-N; leinamycin; lenograstim; lentinan; leptolstatin; leukemia inhibiting factor; leuprorelin; levamisole; liarozole; lissoclinamide; lobaplatin; lombricine; lonidamine; lovastatin; loxoribine; lurtotecan; lutetium texaphyrin; lysofylline; mannostatin A; marimastat; masoprocol; maspin; matrilysin inhibitors; matrix metalloproteinase inhibitors; merbarone; meterelin; methioninase; metoclopramide; MIF inhibitors; mifepristone; miltefosine; mirimostim; mitoguazone; mitolactol; mitonafide; mitotoxin fibroblast growth factor-saporin; mofarotene; molgramostim; human chorionic gonadotrophin monoclonal antibody; mopidamol; mycaperoxide B; myriaporone; N-acetyldinaline; N-substituted benzamides; nafarelin; nagrestip; naloxone; pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; octreotide; okicenone; onapristone; ondansetron; ondansetron; oracin; osaterone; oxaliplatin; oxaunomycin; palauamine; palmitoylrhizoxin; pamidronic acid; panaxytriol; panomifene; parabactin; pazelliptine; peldesine; pentosan; pentostatin; pentrozole; perflubron; perillyl alcohol; phenazinomycin; phenylacetate; phosphatase inhibitors; picibanil; pilocarpine; pirarubicin; piritrexim; placetin A and B; plasminogen activator inhibitor, propyl bis-acridone; prostaglandin J2; proteasome inhibitors; protein kinase C inhibitors; protein tyrosine phosphatase inhibitors; purine nucleoside phosphorylase inhibitors; purpurins; pyrazoloacridine; raltitrexed; ramosetron; ras farnesyl protein transferase inhibitors; ras inhibitors; ras-GAP inhibitors; retelliptine; rhenium 186 etidronate; rhizoxin; retinamide; rohitukine; romurtide; roquinimex; rubiginone B1; ruboxyl; saintopin; sarcophytol A; sargramostim; sizofiran; sobuzoxane; sodium borocaptate; sodium phenylacetate; solverol; somatomedin binding protein; sonermin; sparfosic acid; spicamycin D; splenopentin; spongistatin 1; squalamine; stem-cell division inhibitors; stipiamide; stromelysin inhibitors; sulfinosine; suradista; suramin; swainsonine; tallimustine; tamoxifen; tauromustine; tazarotene; tecogalan; tellurapyrylium; telomerase inhibitors; temozolomide; tetrachlorodecaoxide; tetrazomine; thaliblastine; thiocoraline; thrombopoietin; thymalfasin; thymopoietin receptor agonist; thymotrinan; thyroid stimulating hormone; tin ethyl etiopurpurin; titanocene; topsentin; tretinoin; triacetyluridine; tropisetron; turosteride; tyrosine kinase inhibitors; tyrphostins; ubenimex; urogenital sinus-derived growth inhibitory factor, urokinase receptor antagonists; variolin B; velaresol; veramine; verdins; verteporfin; vinxaltine; vitaxin; zanoterone; zilascorb; and their pharmaceutically acceptable salts.

The compounds of this invention may also be administered in combination with anti-cancer agents which act by arresting cells in the G2-M phases due to stabilized microtubules. In addition to Taxol (paclitaxel), and analogs and derivatives thereof, other examples of anti-cancer agents which act by this mechanism include the following marketed drugs and drugs in development: erbulozole, dolastatin, mivobulin isethionate, discodermolide, altorhyrtins, spongistatins, cemadotin hydrochloride, epothilones desoxyepothilone, 16-aza-epothilone, 21-aminoepothilone, 21-hydroxyepothilone, 26-fluoroepothilone, auristatin, soblidotin, cryptophycin, vitilevuamide, tubulysin, canadensol, centaureidin, oncocidin, fijianolide, laulimalide, narcosine, nascapine, hemiasterlin, vanadocene acetylacetonate, monsatrol, inanocine,

eleutherobins, caribaeoside, caribaeolin, halichondrin, diazonamide, taccalonolide, diozostatin, phenylahistin, myoseverin, resverastatin phosphate sodium, and their pharmaceutically acceptable salts.

Synergistic activity of the pharmaceutical compositions or combined preparations of this invention against cell proliferation may be readily determined by means of one or more tests such as the measurement of the radioactivity resulting from the incorporation of $^3$H-thymidine in culture of tumor cell lines. For instance, different tumor cell lines are selected in order to evaluate the anti-tumor effects of the test compounds, such as:

- RPMI1788: human Peripheral Blood Leucocytes (PBL) Caucasian tumor line,
- Jurkat: human acute T cell leukemia,
- EL4: C57Bl/6 mouse lymphoma, or
- THP-1: human monocyte tumor line.

Depending on the selected tumor cell line, different culture media may be used, such as for example:

- for RPMI1788 and THP-1: RPMI-1640 + 10% FCS + 1% NEAA + 1% sodium pyruvate + $5x10^{-5}$ mercapto-ethanol + antibiotics (G-418 0.45 $\mu$g/ml).
- for Jurkat and EL4: RPMI-1640 + 10% FCS + antibiotics (G-418 0.45 $\mu$g/ml).

In a specific embodiment of the synergy determination test, the tumor cell lines are harvested and a suspension of $0.27x10^6$ cells/ml in whole medium is prepared. The suspensions (150 $\mu$l) are added to a microtiter plate in triplicate. Either complete medium (controls) or the test compounds at the test concentrations (50 $\mu$l) are added to the cell suspension in the microtiter plate. The cells are incubated at 37°C under 5% $CO_2$ for about 16 hours. $^3$H-thymidine is added, and the cells incubated for another 8 hours. The cells are harvested and radioactivity is measured in counts per minute (CPM) in a ß-counter. The $^3$H-thymidine cell content, and thus the measured radioactivity, is proportional to the proliferation of the cell lines. The synergistic effect is evaluated by the median effect analysis method as disclosed hereinbefore.

The pharmaceutical composition or combined preparation with synergistic activity against cell proliferation according to this invention may contain the pteridine derivative of the general formula (I) over a broad content range depending on the contemplated use and the expected effect of the preparation. Generally, the pteridine derivative content of the combined preparation is within the range of 0.1 to 99.9% by weight, preferably from 1 to 99% by weight, more preferably from 5 to 95% by weight.

The invention further relates to a pharmaceutical composition or combined preparation having synergistic effects against a viral infection and containing:

(a) one or more anti-viral agents, and

(b) at least one pteridine derivative represented by the general formula (I); and

(c) optionally one or more pharmaceutical excipients or pharmaceutically acceptable carriers, for simultaneous, separate or sequential use in the treatment or prevention of a viral infection.

Suitable anti-viral agents for inclusion into the synergistic antiviral compositions or combined preparations of this invention include, for instance, retroviral enzyme inhibitors belonging to categories well known in the art, such as HIV-1 IN inhibitors, nucleoside reverse transcriptase inhibitors (e.g. zidovudine, lamivudine, didanosine, stavudine, zalcitabine), non-nucleoside reverse transcriptase inhibitors (e.g. nevirapine, delavirdine), other reverse transcriptase inhibitors (e.g. foscamet sodium), and HIV-1 protease inhibitors (e.g. saquinavir, ritonavir, indinavir, nelfinavir). Other suitable antiviral agents include for instance acemannan, acyclovir, adefovir, alovudine, alvircept, amantadine, aranotin, arildone, atevirdine, avridine, cidofovir, cipamfylline, cytarabine, desciclovir, disoxaril, edoxudine, enviradene, enviroxime, famciclovir, famotine, fiacitabine, fialuridine, floxuridine, fosarilate, fosfonet, ganciclovir, idoxuridine, kethoxal, lobucavir, memotine, methisazone, penciclovir, pirodavir, somantadine, sorivudine, tilorone, trifluridine, valaciclovir, vidarabine, viroxime, zinviroxime, moroxydine, podophyllotoxin, ribavirine, rimantadine, stallimycine, statolon, tromantadine and xenazoic acid, and their pharmaceutically acceptable salts.

Especially relevant to this aspect of the invention is the inhibition of the replication of viruses selected from the group consisting of picoma-, toga-, bunya, orthomyxo-, paramyxo-, rhabdo-, retro-, arena-, hepatitis B-, hepatitis C-, hepatitis D-, adeno-, vaccinia-, papilloma-, herpes-, corona-, varicella- and zoster-virus, in particular human immunodeficiency virus (HIV). Synergistic activity of the pharmaceutical compositions or combined preparations of this invention against viral infection may be readily determined by means of one or more tests such as the isobologram method, as previously described by Elion et al. in J. Biol. Chem. (1954) 208:477-488 and by Baba et al. in Antimicrob. Agents Chemother. (1984) 25:515-517, using $EC_{50}$ for calculating the fractional inhibitory concentration (hereinafter referred as FIC). When the minimum FIC index corresponding to the FIC of combined compounds (e.g., $FIC_x + FIC_y$) is equal

to 1.0, the combination is said to be additive; when it is beween 1.0 and 0.5, the combination is defined as subsynergistic, and when it is lower than 0.5, the combination is by defined as synergistic. When the minimum FIC index is between 1.0 and 2.0, the combination is defined as subantagonistic and, when it is higher than 2.0, the combination is defined as antagonistic.

The pharmaceutical composition or combined preparation with synergistic activity against viral infection according to this invention may contain the pteridine derivative of the general formula (I) over a broad content range depending on the contemplated use and the expected effect of the preparation. Generally, the pteridine derivative content of the combined preparation is within the range of 0.1 to 99.9% by weight, preferably from 1 to 99% by weight, more preferably from 5 to 95% by weight.

The pharmaceutical compositions and combined preparations according to this invention may be administered orally or in any other suitable fashion. Oral administration is preferred and the preparation may have the form of a tablet, aqueous dispersion, dispersable powder or granule, emulsion, hard or soft capsule, syrup, elixir or gel. The dosing forms may be prepared using any method known in the art for manufacturing these pharmaceutical compositions and may comprise as additives sweeteners, flavoring agents, coloring agents, preservatives and the like. Carrier materials and excipients are detailed hereinbelow and may include, *inter alia,* calcium carbonate, sodium carbonate, lactose, calcium phosphate or sodium phosphate; granulating and disintegrating agents, binding agents and the like. The pharmaceutical composition or combined preparation of this invention may be included in a gelatin capsule mixed with any inert solid diluent or carrier material, or has the form of a soft gelatin capsule, in which the ingredient is mixed with a water or oil medium. Aqueous dispersions may comprise the biologically active composition or combined preparation in combination with a suspending agent, dispersing agent or wetting agent. Oil dispersions may comprise suspending agents such as a vegetable oil. Rectal administration is also applicable, for instance in the form of suppositories or gels. Injection (e.g. intramuscularly or intraperiteneously) is also applicable as a mode of administration, for instance in the form of injectable solutions or dispersions, depending upon the disorder to be treated and the condition of the patient.

Auto-immune disorders to be prevented or treated by the pharmaceutical compositions or combined preparations of this invention include both systemic auto-immune diseases such as lupus erythematosus, psoriasis, vasculitis, polymyositis, scleroderma, multiple sclerosis, ankylosing spondilytis, rheumatoid arthritis and Sjögren syndrome; auto-immune endocrine disorders such as thyroiditis; and organ-specific auto-immune diseases such as Addison disease, hemolytic or pernicious anemia, Goodpasture syndrome, Graves disease, idiopathic thrombocytopenic purpura, insulin-dependent diabetes mellitus, juvenile diabetes, uveitis, Crohn's disease, ulcerative colitis, pemphigus, atopic dermatitis, autoimmune hepatitis, primary biliary cirrhosis, autoimmune pneumonitis, autoimmune carditis, myasthenia gravis, glomerulonephritis and spontaneous infertility.

Transplant rejections to be prevented or treated by the pharmaceutical compositions or combined preparations of this invention include the rejection of transplanted or grafted organs or cells (both allografts and xenografts), such as host versus graft reaction disease. The term " organ" as used herein means all organs or parts of organs in mammals, in particular humans, such as kidney, lung, bone marrow, hair, cornea, eye (vitreous), heart, heart valve, liver, pancreas, blood vessel, skin, muscle, bone, intestine or stomach. " Rejection " as used herein mean all reactions of the recipient body or of the transplanted organ which in the end lead to cell or tissue death in the transplanted organ or adversely affect the functional ability and viability of the transplanted organ or the recipient. In particular, this means acute and chronic rejection reactions. Also included in this invention is preventing or treating the rejection of cell transplants and xenotransplantation. The major hurdle for xenotransplantation is that even before the T lymphocytes, responsible for the rejection of allografts, are activated, the innate immune system, especially T-independent B lymphocytes and macrophages are activated. This provokes two types of severe and early acute rejection called hyper-acute rejection and vascular rejection, respectively. The present invention addresses the problem that conventional immunosuppressant drugs like cyclosporin A are ineffective in xeno-transplantation. The ability of the compounds of this invention to suppress T-independent xeno-antibody production as well as macrophage activation may be evaluated in the ability to prevent xenograft rejection in athymic, T-deficient mice receiving xenogenic hamster-heart grafts.

Cell proliferative disorders to be prevented or treated by the pharmaceutical compositions or combined preparations of this invention include any kind of tumor progression or invasion or metastasis inhibition of a cancer, preferably one selected from the group consisting of lung cancer, leukaemia, ovarian cancer, sarcoma, Kaposi's sarcoma, meningioma, colon cancer, lymp node tumor, glioblastoma multiforme, prostate cancer or skin carcinose.

CNS disorders to be prevented or treated by the pharmaceutical compositions of this invention include cognitive pathologies such as dementia, cerebral ischemia, trauma, epilepsy, schizophrenia, chronic pain and neurologic disorders such as depression, social phobia and obsessive compulsive disorders.

Cardiovascular disorders to be prevented or treated by the pharmaceutical compositions of this invention include ischemic disorders, infarct or reperfusion damage, atherosclerosis and stroke.

Allergic conditions to be prevented or treated by the pharmaceutical compositions of this invention include those caused by the pollen of graminae, the presence of pets, as well as more severe forms, such as asthma, characterized by inflammation of airways and bronchospasm. Without wishing to be bound by theory, the antiallergic effect of the

compounds of the invention may be related to their suppression of certain B-cell activation pathways, which can lead to the suppression of IgE release. It may also be related to their properties of inhibiting certain Th2 cytokines, such as IL-5, IL-13 or IL-10, involved in asthma.

TNF-α-related disorders to be prevented or treated by the pharmaceutical compositions of this invention include the following:

- septic or endotoxic shock or sepsis, especially in patients with a serum level of interleukin-6 above 1,000 pg/ml at start of treatment;
- vascular TNF-α- mediated diseases such as disseminated intravascular coagulation and Kawasaki's pathology;
- pathologies and conditions associated with and/or induced by abnormal levels of TNF-α (herein defined as exceeding by at least 10 % and at most 500% the TNF-α level present in a normal healthy subject) occurring in a systemic, localized or particular tissue type or location in the body of the mammal; such tissue types include blood, lymph, liver, kidney, spleen, heart muscle or blood vessels, brain or spinal cord white matter or grey matter, cartilage, ligaments, tendons, lung, pancreas, ovary, testes and prostate. Abnormal TNF levels can also be localized to specific regions or cells in the body, such as joints, nerve blood vessel junctions and bones. Such pathologies include alcohol-induced hepatitis; neurodegenerative diseases such as extrapyramidal and cerebellar disorders including lesions of the corticospinal system; disorders of the basal ganglia; hyperkinetic movement disorders such as chorea; drug-induced movement disorders; hypokinetic movement disorders, such as Parkinson's disease; spinocerebellar degenerations such as spinal ataxia, multiple systems degenerations (including Dejerine-Klumpke syndrome) and systemic disorders (including Refsum's disease, abetalipoprotemia, ataxia and telangiectasia); disorders of.the motor unit, such as neurogenic muscular atrophies (anterior hom cell degeneration, such as amyotrophic lateral sclerosis, infantile spinal muscular atrophy and juvenile spinal muscular atrophy); Alzheimer's disease; Wernicke-Korsakoff syndrome; Creutzfeldt-Jakob disease; Hallerrorden-Spatz disease; and primary or secondary myelodysplastic syndromes;
- toxic effects of TNF-α and/or anti-cancer chemotherapeutic agents, especially side effects associated with TNF generation during neoplastic therapy, for instance following use of cisplatin;
- injuries after irradiation of a tissue of a mammal by radio-elements, such as radiation-induced graft-versus-host disease; and
- cachexia and similar chronic wasting diseases, whether associated with cancer or with other chronic diseases such as malabsortive disorders, excessive physical stress, eating disorders and AIDS.

The medicament of this invention may be for prophylactic use, i.e. where circumstances are such that an elevation in the TNF level might be expected or alternatively, may be for use in reducing the TNF level after it has reached an undesirably high level or as the TNF level is rising.

The term " pharmaceutically acceptable carrier or excipient " as used herein in relation to pharmaceutical compositions and combined preparations means any material or substance with which the active principle, i.e. the pteridine derivative of the general formula (I), and optionally the immunosuppressant or immunomodulator or antineoplastic drug or antiviral agent, may be formulated in order to facilitate its application or dissemination to the locus to be treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, pellets or powders.

Suitable pharmaceutical carriers for use in the said pharmaceutical compositions and their formulation are well known to those skilled in the art. There is no particular restriction to their selection within the present invention although, due to the usually low or very low water-solubility of the pteridine derivatives of this invention, special attention will be paid to the selection of suitable carrier combinations that can assist in properly formulating them in view of the expected time release profile. Suitable pharmaceutical carriers include additives such as wetting agents, dispersing agents, stickers, adhesives, emulsifying or surface-active agents, thickening agents, complexing agents, gelling agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, dissolving, spray-drying, coating and/or grinding the active ingredients, in a one-step or a multi-steps procedure, with the selected carrier material and, where appropriate, the other additives such as surface-active agents, may also be prepared by micronisation, for instance in view to obtain them in the form of microspheres usually having a diameter of about 1 to 10 μm, namely for the manufacture of microcapsules for controlled or sustained release of the biologically active ingredient(s).

Suitable surface-active agents to be used in the pharmaceutical compositions of the present invention are non-ionic, cationic and/or anionic materials having good emulsifying, dispersing and/or wetting properties. Suitable anionic

surfactants include both water-soluble soaps and water-soluble synthetic surface-active agents. Suitable soaps are alkaline or alkaline-earth metal salts, unsubstituted or substituted ammonium salts of higher fatty acids ($C_{10}$-$C_{22}$), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanyl-phosphatidylcholine, dipalmitoylphoshatidylcholine and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups- Such compounds usually contain from 1 to 5 ethyleneglycol units per propyleneglycol unit. Representative examples of non-ionic surfactants are nonylphenol-polyethoxyethanol, castor oil polyglycolic ethers, polypropylene/ polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, preferably halides, having 4 hydrocarbon radicals optionally substituted with halo, phenyl, substituted phenyl or hydroxy; for instance quaternary ammonium salts containing as N-substituent at least one $C_8$-$C_{22}$ alkyl radical (e.g. cetyl, lauryl, palmityl, myristyl, oleyl and the like) and, as further substituents, unsubstituted or halogenated lower alkyl, benzyl and/or hydroxy-lower alkyl radicals.

A more detailed description of surface-active agents suitable for this purpose may be found for instance in " McCutcheon's Detergents and Emulsifiers Annual " (MC Publishing Crop., Ridgewood, New Jersey, 1981), " Tensid-Taschenbuch ", 2nd ed. (Hanser Verlag, Vienna, 1981) and "Encyclopaedia of Surfactants (Chemical Publishing Co., New York, 1981).

Structure-forming, thickening or gel-forming agents may be included into the pharmaceutical compositions and combined preparations of the invention. Suitable such agents are in particular highly dispersed silicic acid, such as the product commercially available under the trade name Aerosil; bentonites; tetraalkyl ammonium salts of montmorillonites (e.g., products commercially available under the trade name Bentone), wherein each of the alkyl groups may contain from 1 to 20 carbon atoms; cetostearyl alcohol and modified castor oil products (e.g. the product commercially available under the trade name Antisettle).

Gelling agents which may be included into the pharmaceutical compositions and combined preparations of the present invention include cellulose derivatives such as carboxymethylcellulose, cellulose acetate and the like; natural gums such as arabic gum, xanthum gum, tragacanth gum, guar gum and the like; gelatin; silicon dioxide; synthetic polymers such as carbomers, and mixtures thereof. Gelatin and modified celluloses represent a preferred class of gelling agents.

Other optional excipients which may be included in the pharmaceutical compositions and combined preparations of the present invention include additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium metabisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylene-diamine tetraacetic acid; flavoring agents such as natural vanillin; buffers such as citric acid and acetic acid; extenders or bulking agents such as silicates, diatomaceous earth, magnesium oxide or aluminum oxide; densification agents such as magnesium salts; and mixtures thereof.

Additional ingredients may be included in order to control the duration of action of the biologically-active ingredient in the compositions and combined preparations of the invention. Control release compositions may thus be achieved by selecting appropriate polymer carriers such as for example polyesters, polyamino-acids, polyvinyl-pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethyl-cellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition or combined preparation of the invention may also require protective coatings.

Pharmaceutical forms suitable for injectable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol, complexing agents such as cyclodextrins and the like, and mixtures thereof.

Since, in the case of combined preparations including the pteridine derivative of this invention and an immunosuppressant or immunomodulator or antihistamine or antineoplastic drug or antiviral agent, both ingredients do not necessarily bring out their synergistic therapeutic effect directly at the same time in the patient to be treated, the said combined preparation may be in the form of a medical kit or package containing the two ingredients in separate but adjacent form. In the latter context, each ingredient may therefore be formulated in a way suitable for an administration route different from that of the other ingredient, e.g. one of them may be in the form of an oral or parenteral formulation whereas the other is in the form of an ampoule for intravenous injection or an aerosol.

The effective amount of a pteridine derivative having the general formula (I), optionally together with an effective amount of another immunosuppressant or immunomodulator or antineoplastic drug or antiviral agent, or a pharmaceutical composition comprising the same, is usually in the range of 0.01 mg to 20 mg, preferably 0.1 mg to 5 mg, per day per kg bodyweight for humans. Depending upon the pathologic condition to be treated and the patient's condition, the said effective amount may be divided into several sub-units per day or may be administered at more than one day intervals. The patient to be treated may be any warm-blooded animal, preferably a human being, suffering from said pathologic condition.

The following examples are intended to illustrate several embodiments of the present invention, as well as the preparation of the pteridine derivatives and their pyrimidine intermediates.

Example 1- preparation of 2,6-diamino-5-nitroso-4-hydroxypyrimidine(reference)

The following illustrates the method step (a) shown in figure 1. To a solution of 2,6-diamino-4-hydroxypyrimidine (12.9 g, 102 mmoles) in 200 ml of a 10% acetic acid solution in water at 80 °C was added dropwise a solution of $NaNO_2$ (7.05 g, 102 mmoles) in 20 ml water. A pink precipitate was formed, which was further stirred for 1 hour at 80 °C. The reaction mixture was cooled down in the refrigerator overnight. The precipitate was filtered off and dried over $P_2O_5$, yielding the title compound as a pink powder (15.43 g, 97%). Its spectral data are in accordance with literature data (Traube in *Ber.* (1900) 33:1371 and Landauer et al. in J. Chem. Soc. (1953) 3721-3722.

Example 2- synthesis of 2,5,6-triamino-4-hydroxypyrimidine (reference)

The following illustrates the method step (b) shown in figure 1. A suspension of the compound of example 1 (15 g, 96.7 mmoles) in an ammonium sulfide solution (20 % in water, 200 ml) was stirred overnight at 50 °C. The reaction mixture was cooled down in the refrigerator and the formed precipitate was filtered off, yielding the title compound as a yellow powder (11.33 g, 83 %). The spectral data are identical with literature data (same as for example 1).

Example 3 - synthesis of 3.4-dimethoxyphenylglyoxalmonoxime (reference)

In a mixture of dioxane (250 ml) and water (10 ml), $SeO_2$ (0.33 mole) was heated to 50 °C. After solution of $SeO_2$, 3,4-dimethoxyacetophenone was added and the mixture heated under reflux for 16 hours. The hot solution was filtered to remove selenium. The filtrate was evaporated, the oily residue dissolved in $CHCl_3$ (300 ml), then washed with saturated $NaHCO_3$ solution (100 ml) and water. The organic phase was dried over $Na_2S_2O_4$, filtered and evaporated. The yellow oil was distilled in vacuum, the resulting 3,4-dimethoxyphenylglyoxal was dissolved in MeOH (50 ml) and water (200 ml), then acetonoxime (0.25 mol) was added and the pH adjusted to 4 by 2 N HCl. The solution was heated to 50 °C for 2 hours, then cooled to 0 °C and the resulting crystals collected. After washing with cold water and drying in a vacuum desiccator, 3,4-dimethoxyphenylglyoxalmonooxime was obtained with a yield of 71 %. Recrystallization can be achieved from $CHCl_3$ or acetone. The compound was further characterized by nuclear magnetic resonance spectra as follows: $^1$H-NMR (200 MHz, DMSO-$d_6$): δ 3.80 (3 H, s), 3.84 (3 H, s), 7.06 (1 H, d), 7.51 (1 H, s), 7.75 (1 H, d), 8.10 (1 H, s) and 12.51 (1 H, s) ppm.

Example 4 - synthesis of 2-amino-6-(3,4-dimethoxyphenyl) pterine (reference)

The following illustrates the method step (c) shown in figure 1. To a boiling solution of the compound of example 2 (2.4 g, 17 mmole) in methanol (100 ml, with 0.9 N HCl) was added dropwise a solution of the compound of example 3 (3.8 g, 18.2 mmole) in methanol (100 ml). The reaction mixture was heated under reflux for 4 hours. A precipitate was formed, which was filtered off, washed with water, ethanol and diethyl ether. The precipitate was dried over $P_2O_5$ under vacuum, yielding the title compound as a yellow powder (4.33 g, 85 %). This compound was further characterized by nuclear magnetic resonance spectra as follows:

- $^1$H-NMR (500 MHz, TFA): δ 4.11 (3 H, s), 4.07 (3 H, s), 7.21 (1 H, d), 7.78 (1 H, dd), 7.81 (1 H, d) and 9.32 (1 H, s) ppm.
- $^{13}$C-NMR (125 MHz, TFA): δ at 56.39, 56.76, 111.94, 113.21, 123.22, 127.41, 127.91, 145.92, 149.39, 150.46, 152.47, 153.15, 155.13 and 161.59 ppm.

Example 5 - synthesis of 2-acetylamino-6-(3,4-dimethoxyphenyl) pterine (reference)

A suspension of the compound of example 4 (10.46 g, 35 mmole) in acetic anhydride (600 ml) and acetic acid (200 ml) was refluxed for 1 hour until a clear solution was formed. By cooling down the reaction mixture in a refrigerator, a precipitate was formed which was filtered off, washed with ethyl acetate and diethyl ether. The precipitate was dried over $P_2O_5$ under vacuum, yielding the title compound as a yellow powder (9.19 g, 77 %). This compound was further characterized as follows:

- mass spectrum (MS): m/z (%): 300 ($[M+H]^+$, 100)
- $^1$H-NMR (200 MHz, DMSO-$d_6$): δ 2.22 (3 H, s), 3.84 (3 H, s), 3.87 (3 H, s), 7.14 (1 H, d), 7.75 (2 H, m) and 9.51 (1 H, s) ppm.

Example 6 - synthesis of 2-acetylamino-4-(1,2,4-triazolyl)-6-[3,4-(dimethoxy-phenyl)] pteridine (reference)

The following illustrates the method step (f) shown in figure 1. To a solution of phosphorus oxychloride (1.68 ml, 18 mmole) and 1,2,4-triazole (4.96 g, 72 mmole) in dry pyridine (110 ml) was added the compound of example 5. (2.45 g, 7.2 mmole). The suspension was stirred at room temperature for 4 hours. The precipitate was filtered off, washed with pyridine, toluene and diethyl ether. The resulting solid was dried over $P_2O_5$ under vacuum, affording the title compound as a yellow powder (2 g, -yield: 80 %) characterized by mass spectrum data as follows: m/z (%): 392 ($[M+H]^+$, 100).

Example 7 - synthesis of 2-acetylamino-4-(piperazin-1-yl)-6-(3,4-dimethoxy-phenyl) pteridine (reference)

The following illustrates the method step (g) shown in figure 1. To a suspension of the compound of example 6 (3.92 g, 10 mmole) in dioxane (200 ml) was added piperazine (1.29 g, 15 mmole). The suspension was stirred for 16 hours at room temperature. The precipitate was filtered off and washed with dioxane, ethanol and diethyl ether. The solid was dried over $P_2O_5$ under vacuum, yielding the title compound as a yellow powder (3.48 g, 85 %).

Examples 8 to 21 - synthesis of 2-amino-4-(*N*-acyl-piperazin-1-yl)-6-(3,4-dimethoxyphenyl) pteridines

The following illustrates the method step (h) shown in figure 1. To a suspension of the compound of example 7 (409 mg, 1 mmole) in dry pyridine (40 ml) was added under nitrogen a suitable carboxylic acid chloride (1.2 mmole). The suspension was stirred at room temperature for 24 hours. The solvent was concentrated *in vacuo* and the crude residue was dissolved in a mixture of $CH_3OH$/20 % $K_2CO_3$ in water (1:1). The solution was stirred at room temperature for 16 hours. Evaporation of the solvents *in vacuo,* followed by purification of the residue by preparative TLC (silica, using a $CH_3OH/CH_2Cl_2$ mixture (5:95) as the eluent) afforded the desired compound as a yellow powder. This procedure provided, with a yield ranging from 33 % to 75 % depending upon the carboxylic acid chloride used, the following pure final pteridine derivatives which were characterized by their mass spectrum MS and optionally by their $^1$H-NMR (500 MHz, DMSO-$d_6$) spectrum:

- 2-amino-4-(*N*-acetylpiperazin-1-yl)-6-(3,4-dimethoxyphenyl)pteridine (example 8): MS 410 ($[M+H]^+$;
- 2-amino-4-[(*N*-propionyl)-piperazin-1-yl]-6-(3,4-dimethoxyphenyl) pteridine (example 9): MS 424 ($[M+H]^+$;
- 2-amino-4-[(*N*-hexanoyl)-piperazin-1-yl]-6-(3,4-dimethoxyphenyl) pteridine (example 10): MS 466 ($[M+H]^+$;
- 2-amino-4-(*N*-benzoylpiperazin-1-yl)-6-(3,4-dimethoxyphenyl) pteridine (example 11): MS 472 ($[M+H]^+$; $^1$H-NMR: δ at 3.65 (br s, 2 H), 3.80-3.90 (m, 8 H), 4.37 (br s, 4 H), 6.76 (br s, 2 H, $NH_2$), 7.07 (d, 1 H), 7.48 (m, 5 H), 7.59 (br d, 1 H), 7.66 (dd, 1 H) and 9.31 (s, 1 H) ppm;

- 2-amino-4-[*N*-(4-chlorobenzoyl)]piperazin-1-yl)-6-(3,4-dimethoxyphenyl) pteridine (example 12): MS 506 ($[M+H]^+$;
- 2-amino-4-[(*N*-2-thiophenecarbonyl)-piperazin-1-yl]-6-(3,4-dimethoxy-phenyl) pteridine (example 13): MS 478 ($[M+H]^+$;
- 2-amino-4-[(*N*-diethylcarbamoyl)-piperazin-1-yl]-6-(3,4-dimethoxy-phenyl) pteridine (example 14): MS 467 ($[M+H]^+$;
- 2-amino-4-[(*N*-hydrocinnamoyl)-piperazin-1-yl]-6-(3,4-dimethoxyphenyl) pteridine (example 15): MS 500 ($[M+H]^+$;
- 2-amino-4-[*N*-(4-cyanobenzoyl)-piperazin-1-yl]-6-(3,4-dimethoxyphenyl) pteridine (example 16): MS 497 ($[M+H]^+$;
- 2-amino-4-[(*N*-phenoxyacetyl)-piperazin-1-yl]-6-(3,4-dimethoxy-phenyl) pteridine (example 17): MS 502 ($[M+H]^+$; $^1$H-NMR δ at 3.75 (br d, 4 H), 3.83 (s, 3 H), 3.86 (s, 3 H), 4.32 (br s, 2 H), 4.41 (br s, 2 H), 4.90 (s, 2H), 6.77 (br s, 2 H), 6.94-6.97 (m, 3 H), 7.09 (d, 1 H), 7.28-7.31 (m, 2 H), 7.62 (d, 1 H), 7.67 (dd, 1 H) and 9.32 (s, 1 H) ppm ;
- 2-amino-4-[(*N*-4-butylbenzoyl)-piperazin-1-yl]-6-(3,4-dimethoxyphenyl) pteridine (example 18): MS 528 ($[M+H]^+$;
- 2-amino-4-[(*N*-isonicotinoyl)-piperazin-1-yl]-6-(3,4-dimethoxyphenyl) pteridine (example 19): MS 473 ($[M+H]^+$; $^1$H-NMR δ at 3.58 (br s, 2 H), 3.82 (s, 6 H), 3.87 (br s, 2 H), 4.33 (br s, 2 H), 4.40 (br s, 2 H), 6.77 (br s, 2 H), 7.07 (d, 1 H), 7.47 (dd, 2 H), 7.59 (br d), 7.66 (dd, 1 H), 8.71 (dd, 2 H) and 9.32 (s, 1 H) ppm;
- 2-amino-4-[(*N*-diisopropylcarbamoyl)-piperazin-1-yl]-6-(3,4-dimethoxy-phenyl) pteridine (example 20): MS 495 ($[M+H]^+$; and
- 2-amino-4--[*N*-(4-pentoxybenzoyl)-piperazin-1-yl]-6-(3,4-dimethoxy-phenyl) pteridine (example 21): MS 558 ($[M+H]^+$.

Example 22 - synthesis of 2.6-diamino-4-chloro-5-*p*-chloroahenylazopyri-,midine (reference)

The following illustrates the method step (a) shown in figure 2. A solution of p-chloroaniline (25.5 g, 0.2 mole) in 6 N HCl (100 ml) was cooled to 0 °C and then $NaNO_2$ (13.8 g, 0.2 mole) in water (40 ml) was added dropwise with stirring. After the addition was completed, the solution was stirred for another 30 minutes. Urea (5 g) was added to destroy the excess of $HNO_2$. The diazonium salt solution was then poured into a solution of 2,6-diamino-4-chloropyrimidine (26 g, 0.18 mole) in water (500 ml) and stirred for 30 minutes. Then potassium acetate (70 g) was added and the mixture was stirred for 16 hours at room temperature. The resulting precipitate was collected by suction, washed with water and dried in a vacuum desscicator over $P_2O_5$ to give 44 g (81 %) of a yellow solid. Recrystallization can be achieved from DMF/$H_2O$. The spectral data are in analogy with those described in literature (Fröhlich et al. in J. Med. Chem. (1999) 42:4108-4121).

Example 23 - synthesis of 2,6-diamino-4-(piperazin-1-yl)-5-*p*-chlorophenylazo-pyrimidine (reference)

The following illustrates the method step (b) shown in figure 2. A solution of the compound of example 22 (5 g, 16.6 mole) in DMF (50 ml) and piperazine (10 g) was heated in an oilbath to 70 °C for 5 hours. Water (50 ml) was added and the reaction mixture was cooled down. The yellow precipitate was filtered off, washed with water and dried. Recrystallization can be achieved from ethanol.

Example 24 - synthesis of 2,5,6-triamino-4-(piperazin-1-yl) pyrimidine (reference)

The following illustrates the method step (c) shown in figure 2. To a suspension of the compound of example 23 (2.03 g, 6.1 mmole) in ethanol (98 ml) and water (98 ml) was added zinc (4.04 g) and acetic acid (2.02 ml). The suspension was refluxed until a clear solution was obtained, i.e. for 1 hour. Zinc was filtered off and the filtrate was evaporated, followed by coevaporation with toluene. A brown precipitate was obtained, which was resuspended in diethyl ether and stirred overnight at room temperature in order to remove *p*-chloroaniline. The precipitate was filtered off and dried in a vacuum dessicator over $P_2O_5$.

Example 25 - synthesis of 2-amino-4-(piperazin-1-yl)-6-(3,4-dimethoxyphenyl) pteridine (reference)

The following illustrates the method step (d) shown in figure 2. To a solution of the compound of example 24 (2.65 mmole, 554 mg) in methanol (30 ml) was added 3,4-dimethoxyphenyglyoxaloxime (2.65 mmole, 554 mg). The pH of the reaction mixture was adjusted to 3 by adding a few drops of concentrated hydrochloric acid. The mixture was refluxed for 3 hours. A yellow precipitate was formed. The reaction mixture was cooled down and neutralized by the addition of concentrated $NH_3$ till pH 9. The precipate was filtered off and used for further reaction without any purification.

Examples 26 to 32 - synthesis of 2-amino-4-(*N*-acylpiperazin-1-yl)-6-(3,4-dimethoxyphenyl) pteridines and 2-amino-4-(*N*-sulfonylpiperazin-1-yl)-6-(3,4-dimethoxyphenyl) pteridines

The following illustrates the method step (g) shown in figure 2. To a suspension of the crude compound of

example 25 (506 mg, 1.38 mmole) in pyridine (30 ml) was added a suitable carboxylic acid chloride or sulfonyl chloride (2.07 mmole). The suspension was stirred at room temperature overnight. After pyridine evaporation, the residue was purified by flash chromatography on silica gel, the mobile phase being $CH_3OH/CH_2Cl_2$ mixtures (in a ratio gradually ranging from 2:98 to 5:95), optionally followed by preparative TLC on silica gel (the mobile phase being a 7:93 $CH_3OH/CH_2Cl_2$ mixture), thus affording the desired compound as a yellow powder. This procedure provided, with a yield ranging from 30 % to 50 % depending upon the carboxylic acid or sulfonyl chloride used, the following pure final pteridine derivatives which were characterized by their mass spectrum MS and optionally by their $^1$H-NMR (200 MHz, DMSO-$d_6$) spectrum:

- 2-amino-4-[*N*-(3-methoxybenzoyl)piperazin-1-yl]-6-(3,4-dimethoxy-phenyl) pteridine (example 26): MS 502 ([M+H]$^+$; $^1$H-NMR: 3.33 (br s, 4 H), 3.80 (s, 9 H), 4.37 (br s, 4 H), 6.78 (br s, 2 H), 6.99-7.70 (m, 7 H) and 9.33 (s, 1 H) ppm.
- 2-amino-4-[*N*-(2-furoyl)-piperazin-1-yl]-6-(3,4-dimethoxyphenyl) pteridine (example 27): MS 462 ([M+H]$^+$;
- 2-amino-4-[(*N*-benzyloxyacetyl)-piperazin-1-yl]-6-(3,4-dimethoxyphenyl) pteridine (example 28): MS 516 ([M+H]$^+$;
- 2-amino-4-[(*N*-(p-chlorophenoxyacetyl)-piperazin-1-yl]-6-(3,4-dimethoxyphenyl) pteridine (example 29): MS 536 ([M+H]$^+$;
- 2-amino-4-[(*N*-cyclohexylcarbonyl)-piperazin-1-yl]-6-(3,4-dimethoxy-phenyl) pteridine (example 30): MS 478 ([M+H]$^+$;
- 2-amino-4-[(*N*-phenylsulfonyl)-piperazin-1-yl]-6-(3,4-dimethoxy-phenyl) pteridine (example 31): MS 508 ([M+H]$^+$; $^1$H-NMR: 3.14 (br s, 4 H), 3.84 (s, 3 H), 3.87 (s, 3 H), 4.40 (br s, 4 H), 6.88 (br s, 2 H), 7.09 (d, 1 H) and 7.56-7.78 (m, 7 H) ppm ; and
- 2-amino-4-[(N p-fluorobenzoyl)-piperazin-1-yl]-6-(3,4-dimethoxyphenyl) pteridine (example 32): MS 490 ([M+H]$^+$.

Example 33 - synthesis of 2,5,6-triamino-4-hydroxypyrimidine dihydrochloride (reference)

The following illustrates the method step (a) shown in figure 3. To a stirred suspension of 2,4,5-triamino-6-hydroxy-pyrimidine (40.93 g, 290 mmole) in methanol (500 ml) was added dropwise concentrated hydrochloric acid 37% (60.5 ml, 725 mmole). The suspension was stirred 30 minutes at room temperature, filtered and the precipitate was washed with methanol, diethyl ether and dried over KOH under vacuum, affording the title compound as a white powder (53.82 g, yield 88 %). Spectral data are identical with literature data (W. Pfleiderer, Chem. Ber. (1957) 90:2272).

Example 34 - synthesis of 2-amino-6-(3,4-dimethoxyphenyl)pterine (reference)

The following illustrates the method step (b) shown in figure 3. The compound of example 33 (21.5 g, 102 mmole) and 3,4-dimethoxyphenyl-glyoxalmonooxime (25.61 g, 122.4 mmole) were suspended in methanol (400 ml) and the orange suspension was heated under reflux for 2.5 hours. The yellow suspension was cooled with an ice bath and the precipitate was filtered and successively washed with methanol, ethyl acetate, diethyl ether, and dried at 110°C for 3 hours to afford a shinny yellow powder (21.56 g, yield 71 %) which was used without further purification.

Example 35 - preparation of 2-amino-4-(piperazin-1-yl)-6-(3,4-dimethoxy-phenyl) pteridine (reference)

The following illustrates the method step (e) shown in figure 3. Piperazine (12.06 g, 140 mmole) was added to a stirred suspension of the compound of example 34 (5.99 g, 20 mmole) in pyridine (64 ml) and 1,1,1,3,3,3-hexamethyldisilazane (64 ml, 300 mmole) in the presence of a catalytic amount of ammonium sulfate and *p*-toluenesulfonic acid. The mixture was heated under reflux for 15 hours and the brown solution was cooled with an ice bath. Methanol was added and the mixture was evaporated to dryness. The brown residue was co-evaporated 2 times with xylene, and adsorbed on silica gel. The compound was purified on silica gel column chromatography, using a 9/1 $CH_2Cl_2$/MeOH mixture containing 1 % concentrated aqueous ammonia as eluent, thus affording the desired compound (3.12 g, yield 42 %) which was characterized by its mass spectrum MS and $^1$H-NMR (200 MHz, DMSO-$d_6$) spectrum as follows:

- MS: m/z (%): 368 ([M+H]$^+$, 100); and
- $^1$H-NMR (200 MHz, DMSO-$d_6$): δ 2.92 (4 H, br s), 3.83 (3 H, s), 3.86 (3 H, s), 4.27 (4 H, br s), 6.70 (2 H, br s), 7.08 (1 H, d), 7.62-7.69 (2 H, m) and 9.30 (1 H, s) ppm.

Examples 36 to 51 - synthesis of 2-amino-4-(*N*-acylpiperazin-1-yl)-6-(3,4-dimethoxyphenyl) pteridines, 2-amino-4-(*N*-sulfonylpiperazin-1-yl)-6-(3,4-dimethoxyphenyl) pteridines and 2-amino-4-(*N*-thioacylpiperazin-1-yl)-6-(3,4-dimethoxyphenyl) pteridines

The following illustrates the method step (f) shown in figure 3. To a suspension of the compound of example 35 (184 mg, 0.5 mmole) in $CH_2Cl_2$ (10 ml) was added triethylamine (84 µl, 0.6 mmole) and a suitable carboxylic acid, thiocarboxylic or sulfonyl chloride (0.55 mmole). The suspension was stirred at room temperature for 2 to 24 hours. The solution was diluted $CH_2Cl_2$ (20 ml) and extracted with a 5% aqueous solution of sodium hydrogen carbonate (30 ml). The organic layer was concentrated *in vacuo* and the crude residue was subjected to silica gel column chromatography, the mobile phase being $CH_3OH/CH_2Cl_2$ mixtures (in a ratio gradually ranging from 1:99 to 5:95), thus affording the desired compound as a yellow powder. This procedure provided, with a yield ranging from 35 % to 85 % depending upon the carboxylic acid, thiocarboxylic or sulfonyl chloride used, the following pure final pteridine derivatives which were characterized by their mass spectrum MS:

- 2-amino-4-[(*N*-2-thiophenacetyl)-piperazin-1-yl)-6-(3,4-dimethoxy-phenyl) pteridine (example 36): MS 492 ($[M+H]^+$;
- 2-amino-4-[(*N*-cinnamoyl)-piperazin-1-yl]-6-(3,4-dimethoxyphenyl) pteridine (example 37): MS 498 ($[M+H]^+$;
- 2-amino-4-[(*N*-1-pyrrolidinylcarbonyl)-piperazin-1-yl]-6-(3,4-dimethoxy-phenyl) pteridine (example 38): MS 951 ($[2M+Na]^+$,15); 929 ($[2M+H]^+$,15); 465 ($[M+H]^+$,100)
- 2-amino-4-[(*N*-diphenylcarbamoyl)-piperazin-1-yl]-6-(3,4-dimethoxy-phenyl) pteridine (example 39): MS 563 ($[M+H]^+$;
- 2-amino-4-[*N*-(2,6-dichloro-5-fluoro-nicotinoyl)]-piperazin-1-yl]-6-(3,4-dimethoxyphenyl) pteridine (example 40): MS 559 ($[M+H]^+$;
- 2-amino-4-[(*N*-methoxyacetyl)-piperazin-1-yl]-6-(3,4-dimethoxyphenyl) pteridine (example 41): MS 901 ($[2M+Na]^+$, 20); 879 ($[2M+H]^+$, 10) and 440 ($[M+H]^+$, 100);
- 2-amino-4-[*N*-(2-methoxybenzoyl)-piperazin-1-yl]-6-(3,4-dimethoxy-phenyl) pteridine (example 42): MS 502 ($[M+H]^+$;
- 2-amino-4-[(*N*-benzylsulfonyl)-piperazin-1-yl]-6-(3,4-dimethoxyphenyl) pteridine (example 43): MS 522 ($[M+H]^+$;
- 2-amino-4-[*N*-(3,4-dichlorobenzoyl)-piperazin-1-yl]-6-(3,4-dimethoxy-phenyl) pteridine (example 44): MS 540 ($[M+H]^+$;
- 2-amino-4-[*N*-(4-chlorophenylacetyl)-piperazin-1-yl]-6-(3,4-dimethoxy-phenyl) pteridine (example 45): MS 520 ($[M+H]^+$;
- 2-amino-4-[(*N*-(1-naphtoyl)-piperazin-1-yl]-6-(3,4-dimethoxyphenyl) pteridine (example 46): MS 522 ($[M+H]^+$;
- 2-amino-4-[M-(3-furoylcarbonyl)-piperazin-1 -yl]-6-(3,4-dimethoxyphenyl) pteridine (example 47): MS 490 ($[M+H]^+$;
- 2-amino-4-[(*N*-benzyloxycarbonyl)-piperazin-1-yl]-6-(3,4-dimethoxy-phenyl) pteridine (example 48): MS 502 ($[M+H]^+$;
- 2-amino-4-[(*N*-dimethylthiocarbamoyl)-piperazin-1 -yl)-6-(3,4-dimethoxy-phenyl) pteridine (example 49): MS 455 ($[M+H]^+$;
- 2-amino-4-[(*N*-phenoxycarbonyl)-piperazin-1-yl]-6-(3,4-dimethoxy-phenyl) pteridine (example 50): MS 487 ($[M+H]^+$; and
- 2-amino-4-[(*N*-phenoxythiocarbonyl)-piperazin-1-yl]-6-(3,4-dimethoxy-phenyl) pteridine (example 51): MS 504 ($[M+H]^+$.

Example 52 - synthesis of 2,6-diamino-4-(*N*-acetylpiperazin-1-yl)-pyrimidine (reference)

The following illustrates the method step (a) shown in figure 4. N-acetylpiperazine (12.82 g, 100 mmole) was added to a stirred suspension of 4-chloro-2,6-diaminopyrimidine (7.23 g, 50 mmole, m.p. 199°C, commercially available for instance from Merck or from Qiaoji Group Co. Ltd., Hong-Kong) in water (100 ml), and the mixture was refluxed for 21 hours. The orange solu-tion was cooled and made alkaline with NaOH 10M (5 ml) to lead to a white precipitation. The solution was filtered; the solid was washed with cold water and dried over $P_2O_5$ in a vacuum dessicator to afford the desired compound as a white powder (9.77 g, yield 82%) which was characterized by the following mass spectrum MS m/z (%): 237 ($[M+H]^+$, - 100); 195 ($[M-Ac+H]^+$, 25).

Example 53 - synthesis of 2,6-diamino-5-nitroso-4-(*N*-acetylpiperazin-1-yl)-pyrimidine (reference)

The following illustrates the method step (b) shown in figure 4. Acetic acid (4 ml) was added dropwise to a stirred suspension of the compound of example 52 (9.45 g, 40 mmole) and sodium nitrite (3.04 g, 44 mmole) in water (200 ml) at room temperature. The purple mixture was stirred for 1 hour and cooled down to 5°C for 14 hours. The precipitate was filtered, washed with water, diethyl ether and dried over $P_2O_5$ in a vacuum dessicator to afford the titled

compound as a purple powder (10.53 g, yield 99%) which was characterized by the following mass spectrum MS *m/z* (%):288 ($[M+Na]^+$, 60); 266 ($[M+H]^+$, 100).

Example 54 - synthesis of 2,5,6-triamino-4-(*N*-acetylpiperazin-1-yl)-pyrimidine (reference)

The following illustrates the method step (c) shown in figure 4. To a suspension of the compound obtained in example 53 (1 g, 3.77 mmole) in water (25 ml) was added sodium dithionite (1.97 g, 11.3 mmole). The suspension was heated to 50 °C till a clear solution was obtained. Water was evaporated *in vacuo* and the residue was co-evaporated with toluene twice. The crude material was used for further reaction without any purification.

Example 55 - synthesis of 2-amino-4-(*N*-acetylpiperazin-1-yl)-6-phenyl-pteridine

The following illustrates the method step (d) shown in figure 4. The crude product obtained in example 54 and isonitrosoacetophenone (653 mg, 4.0 mmole) were suspended in a 1.25 M HCl solution in MeOH (20 ml) and the mixture was refluxed for 3 hours. The reaction mixture was cooled down to room temperature and neutralized with a 25 % aqueous $NH_3$ solution to pH 9. The mixture was evaporated to dryness and the residue was partitioned between $CHCl_3$ and $H_2O$. The organic layer was separated, evaporated to dryness and purified by silica gel chromatography, the mobile phase consisting of $CH_3OH/CH_2Cl_2$ mixtures (in a ratio gradually ranging from 2:98 to 4:96), thus affording the desired compound as a yellow powder (978 mg, yield 70 %) was characterized by its mass spectrum MS as follows: MS m/z (%): 721 ($[2M+Na]^+$, 60); 372 ($[M+Na]^+$, 10) and 350 ($[M+H]^+$, 100).

Example 56 - synthesis of 2-amino-4-(*N*-acetylpiperazin-1-yl)-6-(4-tolyl) pteridine

The method of example 55 was repeated, except for using 4-methylphenylglyoxalmonoxime (4.0 mmole) instead of isonitrosoaceto-phenone. This afforded the title compound as a yellow powder (900 mg, yield 62 %), which was characterized by its mass spectrum MS as follows: MS m/z (%): 362 ($[M+H]^+$, 100).

Example 57 - synthesis of 2-amino-4-(*N*-acetylpiperazin-1-yl)-6-(4-fluoro-phenyl) pteridine

The following illustrates the method step (d) shown in figure 4. The crude product obtained in example 54 was dissolved in a 1.25 M HCl solution in MeOH (20 ml) and 4-fluorophenylglyoxalmonoxime (504 mg, 3.0 mmole) was added portionwise. The mixture was refluxed for 3 hours. The reaction mixture was cooled down to room temperature and neutralized with $NH_3$ 25 % aqueous solution to pH 9. The yellow precipitate was filtered off and washed with water. The precipitate was adsorbed on silica gel and purified by flash chromatography, the mobile phase consisting of $CH_3OH/CH_2Cl_2$ mixtures (in a ratio gradually ranging from 1:99 to 4:96), thus affording the title compound as a yellow powder (734 mg, 53 % yield), which was characterized by its mass spectrum MS as follows: MS m/z (%): 368 ($[M+H]^+$, 100).

Example 58 - synthesis of 2-amino-4-(N-acetylpiperazin-1-yl)-6-(4-chloro-phenyl) pteridine

The method of example 57 was repeated, except for using 4-chlorophenylglyoxalmonoxime (554 mg, 3.0 mmole) instead of 4-fluorophenyl-glyoxalmonoxime. This afforded the title compound as a yellow powder (924 mg, 64 % yield), which was characterized by its mass spectrum MS as follows: MS m/z (%): 384 ($[M+H]^+$, 100).

Example 59 - synthesis of 2-amino-4-(*N*-acetylpiperazin-1-yl)-6-(4-acetamido-phenyl) pteridine (reference)

The method of example 57 was repeated, except for using 4-acetyl-benzamidophenylglyoxalmonoxime (206 mg, 3.0 mmole) instead of 4-fluoro-phenylglyoxalmonoxime, and performing silica gel flash chromato-graphy with a $CH_3OH/CH_2Cl_2$ gradient from 2:98 to 10:90. This afforded the title compound as a yellow powder (871 mg, 57 % yield), which was characterized by its mass spectrum MS as follows: MS *m/z* (%): 407 ($[M+H]^+$, 100).

Examples 60 to 63 - synthesis of 2-amino-4-[*N*-(α-aminoacyl)-piperazin-1-yl]-6-(3,4-dimethoxyphenyl) pteridines

The following illustrates the method step (a) shown in figure 5, wherein $R_{11}$ is a radical having an amino group in α position with respect to the carboxylic acid group . The compound of example 35 (0.367 g, 1 mmole) and a tert-butoxycarbonyl-protected amino-acid, such as L-phenylalanine (example 60), L-tyrosine (example 61), L-proline (example 62) or L-tryptophane (example 63), were suspended in dry dimethylformamide at room temperature under nitrogen and diisopropylethylamine (0.418 ml, 2.4 mmole), followed by *o*-benzotriazol-1-yl-N,N,N',N'-tetramethyluronium tetrafluoroborate (0.482 g, 1.5 mmole) were added. The mixture was stirred for 2 hours and diluted with dichloromethane

(50 ml). The organic layer was washed with a saturated solution of sodium hydrogen carbonate (50 ml), dried over anhydrous sodium sulfate and evaporated to dryness. The crude residue was purified by silica gel chromatography, the mobile phase consisting of $CH_3OH/CH_2Cl_2$ mixtures (in a ratio gradually ranging from 3:97 to 6:94), with 0.5% concentrated aqueous ammonia if needed. This procedure provided tert-butoxycarbonyl-protected 2-amino-4-[*N*-(α-aminoacyl)-piperazin-1-yl]-6-(3,4-dimethoxyphenyl) pteridine intermediates with yields ranging from 50 % to 80 % depending upon the tert-butoxycarbonyl-protected amino-acid used.

Then the said tert-butoxycarbonyl-protected intermediate (0.5 mmole) was deprotected either by being suspended in a mixture of dioxane (10 ml) and HCl 6M (20 ml) and stirred at room temperature until complete mixture or by using a solution of 20 % trifluoroacetic acid in dichloromethane (10 ml). The medium treated with HCl was then neutralized with NaOH 10M and volatiles were removed, whereas the mixture treated with trifluoroacetic acid was directly evaporated to dryness. The residue was adsorbed on silica and purified by silica gel column chromatography, the mobile phase consisting of $CH_3OH/CH_2Cl_2$ mixtures (in a ratio gradually ranging from 4:96 to 6:94) containing 0.5% of concentrated aqueous ammonia.

This procedure provided, with a yield ranging from 50 % to 70 % depending upon the tert-butoxycarbonyl-protected amino-acid used, the following pure final pteridine derivatives as yellow powders which were characterized by their mass spectrum MS as follows:

- 2-amino-4-[*N*-(2-(*S*)-amino-3-phenylpropionyl)-piperazin-1-yl]-6-(3,4-dimethoxyphenyl) pteridine (example 60) : MS 515 ($[M+H]^+$;
- 2-amino-4-[*N*-[2-(*S*)-amino-3-(4-hydroxyphenyl)propionyl]-piperazin-1-yl]-6-(3,4-dimethoxyphenyl) pteridine (example 61) : MS 531 ($[M+H]^+$;
- 2-amino-4-[*N*-(pyrrolidin-2-(*S*)-yl)carbonyl-piperazin-1-yl]-6-(3,4-dimethoxy-phenyl) pteridine (example 62) : MS 465 ($[M+H]^+$; and
- 2-amino-4-[[*N*-2-(*S*)-amino-3-(indol-2-yl)propionyl]-piperazin-1-yl]-6-(3,4-dimethoxyphenyl) pteridine (example 63) : MS 554 ($[M+H]^+$.

Example 64 - synthesis of 2-amino-4-(*N*-phenylpiperazin-1-yl)-6-(3,4-dimethoxyphenyl) pteridine

To a suspension of the compound of example 6 (196 mg, 0.5 mmole) in dioxane (10 ml) was added *N*-phenylpiperazine (0.23 ml, 1.5 mmole). The suspension was stirred at room temperature overnight. The precipitate was filtered off and washed with dioxane and diethylether, yielding the crude 2-acetylamino-4-(*N*-phenylpiperazine)-6-(3,4-dimethoxyphenylpteridine). Deprotection of the acetylamino group was achieved by dissolving this crude compound in methanol (5 ml) and a 20 % $K_2CO_3$ solution in water (5 ml). The solution was stirred overnight. Solvents were evaporated *in vacuo* and the residue was purified by preparative TLC (silica, using a $CH_3OH/CH_2Cl_2$ (5:95) mixture as an eluent), affording the title compound as a yellow powder (84 mg, yield 38 %) which was characterized by its mass spectrum MS as follows: MS *m*/*z* (%): 444 ($[M+H]^+$, 100).

Example 65 synthesis of 2-amino-4-(*N*-benzylpiperazin-1-yl)-6-(3,4-dimethoxvphenyl) pteridine

Repeating the method of example 64, except for using *N*-benzyl-piperazine (0.26 ml, 1.5 mmole) instead of *N*-phenylpiperazine, afforded the title compound as a yellow powder (75 mg, yield 33%) which was characterized by its mass spectrum MS as follows: MS m/z (%): 458 ($[M+H]^+$ , 100).

Example 66 - synthesis of 2-amino-4-(*N*-trans-cinnamylpiperazin-1-yl)-6-(3,4-dimethoxyphenyl) pteridine

Repeating the method of example 64, except for using *N*-cinnamyl-piperazine (0.306 ml, 1.5 mmole) instead of *N*-phenylpiperazine, afforded the title compound as a yellow powder (99 mg, yield 41 %) which was characterized by its mass spectrum MS as follows: MS m/z (%): 484 ($[M+H]^+$ , 100).

Examples 67 to 70 - synthesis of 2-substituted 4,6-diamino-5-nitroso-pyrimidines (reference)

To a suspension of 4,6-diamino-2-methylmercapto-5-nitroso-pyrimidine (1 g, 5.41 mmole), which may be prepared and characterised for instance as disclosed by Baddiley et al. in J. Chem. Soc. (1943) 383, in water (25 ml) was added a large excess (162 mmole) of an appropriate amine. After heating the reaction mixture at 65 °C during 3 hours, a pink suspension was formed. The reaction mixture was then cooled down to + 4°C for 4 days. The pink precipitate was filtered off and washed with water, yielding the pure following compounds, each being characterised by its mass spectrum (MS), in yields ranging from 30 to 50 % :

- 2-phenylethylamino-4,6-diamino-5-nitroso-pyrimidine (example 67) was obtained from phenylethylamine ; MS: m/z (%): 259 ($[M+H]^+$, 100).
- 2-(2-thienylmethylamino)-4,6-diamino-5-nitroso-pyrimidine (example 68) was obtained from 2-thiophenemethylamine ; MS: m/z (%): 251 ($[M+H]^+$, 100).
- 2-pyrrolidino-4,6-diamino-5-nitroso-pyrimidine (example 69) was obtained from pyrrolidine ; MS: m/z (%): 209 ($[M+H]^+$, 100).
- 2-benzylamino-4,6-diamino-5-nitroso-pyrimidine (example 70) was obtained from benzylamine ; MS: m/z (%): 245 ($[M+H]^+$, 100).

Example 71 to 74 - synthesis of 2-substituted-4,5,6-triamino-pyrimidine sulfates (reference)

To a suspension of a 2-substituted-4,6-diamino-5-nitroso-pyrimidine obtained in one of examples 67 to 70 (1 mmole) in water (25 ml) was added portionwise sodium dithionite (3 mmole). The resulting suspension was refluxed until a yellow solution was formed. A sulfuric acid solution (2.5 ml of a 50 % solution in water) was then added. The reaction mixture was cooled down to + 4°C for 5 hours. The white precipitate formed was filtered off, yielding the pure following compounds in yields ranging from 60% to 75%.

- 2-phenylethylamino- 4,5,6-triamino-pyrimidine sulfate (example 71),
- 2-(2-thienylmethylamino)- 4,5,6-triamino-pyrimidine sulfate (example 72),
- 2-pyrrolidino- 4,5,6-triamino-pyrimidine sulfate (example 73), and
- 2-benzylamino- 4,5,6-triamino-pyrimidine sulfate (example 74).

Examples 75 to 78 - synthesis of 2-substituted-4,5,6-triamino-pyrimidine dihydrochlorides (reference)

To a suspension of a 2-substituted-4,5,6-triamino-pyrimidine sulfate obtained in one of examples 71 to 74 (1 mmole) in water (6 ml) at 80 °C was added dropwise a solution of barium chloride dihydrate (0.9 mmole) in water (2 ml). The resulting suspension was stirred for 30 minutes at 80 °C, then the reaction mixture was cooled down and barium sulfate was filtered off over Celite. The filtrate was evaporated in vacuo and co-evaporated with toluene yielding each of the following compounds as a yellow powder in yields ranging from 90 % to 98 %:

- 2-phenylethylamino- 4,5,6-triamino-pyrimidine dihydrochloride (example 75),
- 2-(2-thienylmethylamino)- 4,5,6-triamino-pyrimidine dihydrochloride (example 76),
- 2-pyrrolidino- 4,5,6-triamino-pyrimidine dihydrochloride (example 77), and
- 2-benzylamino- 4,5,6 triamino-pyrimidine dihydrochloride (example 78).

Examples 79 to 82 - synthesis of 2-substituted-4-amino-6-(3,4-dimethoxy-phenyl) pteridines (reference)

The following procedure is in accordance with step (e) of figure 6. To a solution of a 2- substituted-4,5,6-triamino-pyrimidine dihydrochloride obtained in one of examples 75 to 78 (1 mmole) in methanol (15 ml) was added the 3,4-dimethoxyphenylglyoxaloxime obtained according to example 3 (1 mmole). The resulting solution was refluxed for 2 hours, thus forming a yellow suspension. The reaction mixture was cooled down and neutralised by addition of a 33% aqueous ammonia solution until pH 9 was reached. The yellow precipitate was filtered off and further purified by silica gel flash chromatography (eluting with solvent mixture $CH_3OH/CH_2Cl_2$, gradient from 1:99 to 3:97), yielding as a yellow powder each of the pure following compounds, which was characterised by its mass spectrum (MS) and its ultraviolet spectrum (UV):

- 2-phenylethylarnino-4-amino-6-(3,4-dimethoxyphenyl) pteridine (example 79) was obtained from the salt of example 75; MS: m/z (%): 403 ($[M+H]^+$, 100), 827 ($[2M+Na]^+$, 20); UV (MeOH, nm): 287, 315, 412.
- 2-(2-thienylmethylamino)- 4-amino-6-(3,4-dimethoxyphenyl) pteridine (example 80) was obtained from the salt of example 76; MS: m/z (%): 394 ($[M+H]^+$, 100); UV (MeOH, nm): 287, 314, 410.
- 2-pyrrolidino-4-amino-6-(3,4-dimethoxyphenyl) pteridine (example 81) was obtained from the salt of example 77; MS: m/z (%): 353 ($[M+H]^+$, 100), 727 ([2M+Na)]+, 10); UV (MeOH, nm): 319, 423.
- 2-benzylamino-4-amino-6-(3,4-dimethoxyphenyl) pteridine (example 82) was obtained from the salt of example 78; MS: m/z (%): 389 ($[M+H]^+$, 100); UV (MeOH, nm): 287, 315, 411.

Example 83 - synthesis of 4,5,6-triamino-2-methylmercaptopyrimidine dihydrochloride (reference)

To a suspension of a 2-methylmercapto-4,5,6-triamino-pyrimidine sulfate (44.3 mmole), which may be prepared

and characterised for instance as disclosed by Taylor et al. in J. Am. Chem. Soc. (1952) 74:1644-1647, in water (135 ml) at 80 °C was added dropwise a solution of barium chloride dihydrate (39.8 mmole) in water (25 ml). The suspension was stirred for 30 minutes at 80 °C. The reaction mixture was cooled down and barium sulfate was filtered off over Celite. The filtrate was evaporated in vacuo and co-evaporated with toluene yielding the title compound as a yellow powder (10.2 g, 94 % yield).

Example 84 - synthesis of 4-amino-2-methylmercapto-6-(3,4-dimethoxy-phenyl) pteridine (reference)

To a suspension of 4,5,6-triamino-2-methylmercaptopyrimidine dihydrochloride (7.42 mmole, 1.81 g) in methanol (20 ml) was added a solution of 3,4-dimethoxyphenylglyoxaloxime (5.94 mmole, 1.24 g) in methanol. The resulting reaction mixture was refluxed for 3 hours. The reaction mixture was neutralised with concentrated aqueous ammonia until pH 9 was reached. The resulting precipitate was filtered off and further purified by flash chromatography (silica, using an ethyl acetate / hexane mixture in a 4:6 ratio) yielding the pure title compound as a yellow powder which was characterised as follows: MS: m/z (%) 330 ($[M+H]^+$, 100), 681 ($[2M+Na]^+$, 30); UV (MeOH, nm): 292, 397.

Example 85 - synthesis of 4-amino-2-methylmercapto-6-phenyl-pteridine (reference)

A method similar to that of example 84 was used, starting from phenylglyoxal monoxime instead of 3,4-dimethoxyphenylglyoxalmonoxime. The title compound was characterised as follows: MS: m/z (%): 270 ($[M+H]^+$, 100); UV (MeOH, nm): 286, 379.

Example 86 - synthesis of 4-amino-2-morpholino-6-(3,4-dimethoxyphenyl) pteridine (reference)

A solution of the compound of example 84 (100 mg, 0.304 mmole) in morpholine (12 ml) was refluxed overnight. The solvents were removed in vacuo and the residue was purified first by flash chromatography (silica, gradient from 2: 98 to 3:97 $CH_3OH$ /$CH_2Cl_2$) and then by preparative TLC (silica, EtOAc/hexane 1 :1) yielding the title compound as a yellow powder (70 mg, 63 % yield) characterised as follows: MS: m/z (%): 369 ($[M+H]^+$, 100), 759 ($[2M+Na]^+$, 20); UV (MeOH. nm): 297, 315, 418.

Example 87 - synthesis of 4-amino-2-piperidino-6-(3,4-dimethoxyphenyl) pteridine (reference)

A method similar to that of example 86 was used, starting from piperidine instead of morpholine. The title compound obtained as a yellow powder (58 mg, 52 %) was characterised as follows: MS: m/z (%): 367 ($[M+H]^+$, 100), 755 ($[2M+Na]^+$, 10); UV (MeOH, nm): 319, 425.

Example 88 - synthesis of 2-amino-4-(homopiperazin-1-yl)-6-(3,4-dimethoxyphenyl) pteridine

Homopiperazine (1.39 g) was added to a stirred suspension of 2-amino-6-(3,4-dimethoxyphenyl)pteridine (520 mg) in pyridine (9 ml) and 1,1,1,3,3,3-hexamethyldisilazane (9.2 ml) in the presence of a catalytic amount of ammonium sulfate (54 mg) and p-toluenesulfonic acid (52 mg). The mixture was heated under reflux for 72 hours until a clear solution was obtained. The mixture was cooled down and the solvents were evaporated *in vacuo.* The residue was adsorbed on silica and purified by silica gel column chromatography, using a 9:1 $CH_2Cl_2$/$CH_3OH$ mixture containing 1 % concentrated aqueous ammonia as eluent, affording the desired compound (305 mg, yield 46 %) which was characterized by its mass spectrum as follows : m/z (%) 785 ($[2M+H]^+$, 15), 382 ($[M+H]^+$, 100).

Example 89 synthesis of 2-amino-4-(*N*-phenoxyacetyl)-homopiperazin-1-yl)-6-(3,4-dimethoxyphenyl)pteridine

To a solution of 2-amino-4-(homopiperazin-1-yl)-6-(3,4-dimethoxyphenyl) pteridine (160 mg) in DMF (20 ml) was added triethylamine (0.55 mmole) and phenoxyacetyl chloride (0.5 mmole). The solution was stirred at room temperature for 24 hours. The solution was diluted with $CH_2Cl_2$ and extracted 3 times with water. The organic solvents were evaporated *in* vacuo. The residue was adsorbed on silica, and purified by flash chromatography (silica, the mobile phase being $CH_3OH$/$CH_2Cl_2$ mixtures (in a ratio gradually ranging from 2:98 to 5:95). This procedure provided with a yield of 67 % the title compound as a yellow powder (145 mg) which was characterized as follows:

- mass spectrum : m/z (%) 1053 ($[2M+H]^+$, 5), 516 ($[M+H]^+$, 100), and
- UV spectrum (nm) : 213, 296, 408.

Examples 90 to 98 - synthesis of 2-amino-4-[*N*-(thio)carboxy)-piperazin-1-yl]-6-(3,4-dimethoxyphenyl)pteridines

To a solution of 2-amino-4-(piperazin-1-yl)-6-(3,4-dimethoxyphenyl)pteridine (200 mg, 0.55 mmole) in DMF (20 ml) was added triethylamine (0.65 mmole, 92 µl) and a suitable chloroformate (0.71 mmole). The solution was stirred at room temperature for 2 to 24 hours, depending upon the chloroformate used, while monitoring the reaction by TLC. The solution was diluted with $CH_2Cl_2$ and extracted with water (3 times). The organic solvents were evaporated *in vacuo.* The residue was adsorbed on silica, and purified by silica gel column chromatography, the mobile phase being $CH_3OH/CH_2Cl_2$ mixtures (in a ratio gradually ranging from 2:98 to 5:95). This procedure provided with a yield ranging from 60 % to 80 %, depending on the chloroformate used, the following pure pteridine derivatives, which were characterized by their mass spectrum (MS) and their ultraviolet spectrum (UV).

- 2-amino-4-[(*N*-4-methyl-phenoxy-carbonyl)-piperazin-1-yl]-6-(3,4-dimethoxyphenyl) pteridine, obtained from *p*-tolylchloroformate (example 90) : MS : m/z (%) 502 ([M+H]$^+$, 100) ; UV (nm) : 215, 296, 412 ;
- 2-amino-4-[(*N*-4-methoxy-phenoxy-carbonyl)-piperazin-1-yl]-6-(3,4-dimethoxyphenyl)pteridine, obtained from *p*-methoxy-phenyl chloroformate (example 91) : MS : m/z (%) 518 ([M+H]$^+$, 100) ; UV (nm) : 217, 296, 412 ;
- 2-amino-4-[(*N*-4-fluoro-phenoxy-carbonyl)-piperazin-1-yl]-6-(3,4-dimethoxyphenyl) pteridine (example 92), obtained from *p*-fluoro-phenyl chloroformate : MS : m/z (%) 506 ([M+H]$^+$, 100) ; UV (nm) : 213, 296, 412 ;
- 2-amino-4-[*N*-(2-methoxy)-phenoxy-carbonyl)-piperazin-1-yl]-6-(3,4-dimethoxy-phenyl) pteridine (example 93), obtained from 2-methoxy-phenylchloroformate : MS : m/z (%) 518 ([M+H]$^+$, 100) ; UV (nm) : 215, 296, 410 ;
- 2-amino-4-[*N*-(4-chloro)-phenoxy-carbonyl)-piperazin-1-yl]-6-(3,4-dimethoxyphenyl) pteridine (example 94), obtained from *p*-chloro-phenyl chloroformate : MS : m/z (%) 523 ([M+H]$^+$, 100) ; UV (nm) : 217, 296, 412 ;
- 2-amino-4-[*N*-isobutoxy-carbonyl-piperazin-1-yl]-6-(3,4-dimethoxyphenyl)pteridine (example 95), obtained from isobutyl chloroformate : MS : m/z (%) 468 ([M+H]$^+$, 100) ; UV (nm) : 215, 297, 413 ;
- 2-amino-4-[*N*-(2-chloro)-phenoxy-carbonyl-piperazin-1-yl]-6-(3,4-dimethoxyphenyl) pteridine (example 96), obtained from 2-chloro-phenyl chloroformate : MS : m/z (%) 523 ([M+H]$^+$, 100) ; UV (nm) : 213, 296, 412 ;
- 2-amino-4-[*N*-(2-methoxy)-ethoxy-carbonyl)-piperazin-1-yl]-6-(3,4-dimethoxyphenyl) pteridine (example 97), obtained from 2-methoxy-ethyl chloroformate : MS : m/z (%) 470 ([M+H]$^+$, 100) ; UV (nm) : 212, 256, 296, 412 ; and
- 2-amino-4-[*N* (2-naphthoxy)-carbonyl)-piperazin-1-yl]-6-(3,4-dimethoxyphenyl) pteridine (example 98), obtained from 2-naphthyl chloroformate : MS : m/z (%) 538 ([M+H]$^+$, 100); UV (nm) : 222 , 296, 412.

Examples 99 to 109 - synthesis of 2-amino-4-(*N*-carbamoyl-piperazin-1-yl)-6-(3,4-dimethoxyphenyl)pteridines

To a solution of 2-amino-4-(piperazin-1-yl)-6-(3,4-dimethoxyphenyl)pteridine (0.61 mmol, 225 mg) in DMF (30 ml) was added a suitable isocyanate (0.92 mmole). The solution was stirred at room temperature for 2 to 24 hours, depending upon the isocyanate used, the reaction being monitored by TLC. The solution was diluted with $CH_2Cl_2$ and extracted 3 times with water. The organic solvents were evaporated *in vacuo.* The residue was adsorbed on silica, and purified by silica gel column chromatography, the mobile phase being $CH_3OH/CH_2Cl_2$ mixtures (in a ratio gradually ranging from 2:98 to 5:95). This procedure provided with a yield ranging from 60% to 80%, depending on the isocyanate used, the following pure pteridine derivatives, each as a yellow powder, which were characterized by their mass spectrum (MS) and their ultraviolet spectrum (UV):

- 2-amino-4-(N-phenyl-carbam oyl-piperazin-1-yl)-6-(3,4-dimethoxyphenyl)pteridine (example 99), obtained from phenyl isocyanate : MS: m/z (%) 487 ([M+H]$^+$, 100) ; UV (nm) : 239, 297, 412 ;
- 2-amino-4-[*N*-4-fluorophenyl-carbamoyl-piperazin-1-yl)]-6-(3,4-dimethoxyphenyl)pteridine (example 100), obtained from 4-fluoro-phenyl isocyanate : MS : m/z (%) 1031 ([2M+Na]$^+$, 15), 523 ([M+H]$^+$, 100) ; UV (nm) : 297, 413 ;
- 2-amino-4-(*N*-4-methylphenyl-carbamoyl-piperazin-1-yl)-6-(3,4-dimethoxyphenyl) pteridine (example 101), obtained from 4-methyl-phenyl isocyanate : MS : m/z (%) 1023 ([2M+Na]$^+$, 15), 501 ([M+H]$^+$, 100) ; UV (nm) : 241, 270, 413 ;
- 2-amino-4-(*N*-4-cyanophenylcarbamoyl-piperazin-1-yl)-6-(3,4-dimethoxyphenyl)pteridine (example 102), obtained from 4-cyano-phenyl isocyanate : MS : m/z (%) 512 ([M+H]$^+$, 100) ;
- 2-amino-4-(*N*-methylphenylcarbamoyl-piperazin-1-yl)-6-(3,4-dimethoxyphenyl)pteridine (example 103), obtained from 3-methyl-phenyl isocyanate :MS : m/z (%)501 ([M+H]$^+$, 100) ; UV (nm) : 241, 297, 412 ;
- 2-amino-4-(*N*-benzylcarbamoyl-piperazin-1-yl)-6-(3,4-dimethoxyphenyl)pteridine (example 104), obtained from benzyl isocyanate : MS : m/z (%) 501 ([M+H]$^+$, 100) ; UV (nm) : 242, 297, 413 ;
- 2-amino-4-(*N*-4-fluorobenzylcarbamoyl-piperazin-1-yl)-6-(3,4-dimethoxyphenyl)pteridine (example 105), obtained from 4-fluorobenzyl isocyanate : MS : m/z (%) 1059 ([2M+Na]$^+$, 10), 519 ([M+H]$^+$, 100) ; UV (nm) : 212, 297, 412 ;
- 2-amino-4-(*N*-3-chloro-4-fluorophenylcarbamoyl-piperazin-1-yl)-6-(3,4-dimethoxyphenyl)pteridine (example 106), obtained from 3-chloro-4-fluoro-phenyl isocyanate : MS: m/z (%) 540 ([M+H]$^+$, 100) ; UV (nm) : 212, 240, 296, 412 ;

- 2-amino-4-(*N*-3-thienylcarbamoyl-piperazin-1-yl)-6-(3,4-dimethoxyphenyl)pteridines (example 107), obtained from 3-thienyl isocyanate : MS : m/z (%) 493 ($[M+H]^+$, 100) ; UV (nm) : 216, 297, 413 ;
- 2-amino-4-[*N*-2-(2-thienyl)ethylcarbamoyl-piperazin-1-yl]-6-(3,4-dimethoxyphenyl) pteridine (example 108), obtained from 2-(2-thienyl)ethyl isocyanate ; and
- 2-amino-4-[(*N*-butyl-carbamoyl-piperazin-1-yl)]-6-(3,4-dimethoxyphenyl)pteridine (example 109), obtained from *n*-butyl isocyanate : MS : m/z (%) 467 ($[M+H]^+$, 100) ; UV (nm) : 214, 298, 413.

Examples 110 and 111 - synthesis of 2-amino-4-[*N*-(α-aminoacyl)-piperazin-1-yl]-6-(3,4-dimethoxyphenyl) pteridines

The procedure of examples 60 to 63 was repeated while starting from different *tert*-butoxycarbonyl-protected amino-acids, i.e. glycine (example 110) and L-asparagine (example 111). The procedure provided the two following pure pteridine derivatives as yellow powders which were characterized by their mass spectrum MS as follows:

- 2-amino-4-[*N*-aminoacetyl]-piperazin-1-yl]-6-(3,4-dimethoxy-phenyl) pteridine (example 110): MS m/z (%) 425 $[M+H]^+$; and
- 2-amino-4-[*N*-[2-(S),4-diamino-4-oxobutanoyl]-piperazin-1-yl]-6-(3,4-dimethoxyphenyl) pteridine (example 1-11): MS m/z (%) 482 ($[M+ H]^+$, 100).

Examples 112 to 115 - synthesis of 2-amino-4-(*N*-acyl-piperazin-1-yl)-6-(3.4-dimethoxyphenyl) pteridines

The compound of example 35 (0.367 g, 1 mmole) and a carboxylic acid or anhydride such as mono-methyl terephthalate (example 112), dimethylglycine (example 113), succinamic acid (example 114) or succinic anhydride (example 115) were suspended in dry DMF at room temperature under a nitrogen atmosphere and then diisopropylamine (0.418 ml, 2.4 mmole), followed by o-benzotriazol-1-yl-N,N, N',N'-tetramethyluronium tetrafluoroborate (0.482 mg, 1.5 mmole) were added. The mixture was stirred until completion of the reaction and then diluted with dichloromethane (50 ml). The organic layer was washed with a saturated solution of sodium hydrogenocarbonate (50 ml), dried over anhydrous sodium sulfate and evaporated to dryness. The crude residue was purified by silica gel column chromatography, the mobile phase consisting of $CH_3OH/CH_2Cl_2$ mixtures (in a ratio gradually ranging from 2:98 to 10:90), with 0.5 % concentrated ammonia or acetic acid if needed. This procedure provided, with yields ranging from 56 % to 72 % depending upon the starting carboxylic acid or anhydride, the desired compounds which were characterized by their mass spectrum MS as follows :

- 2-amino-4-[*N*-[4-(methoxycarbonyl)benzoyl]-piperazin-1-yl]-6-(3,4-dimethoxy-phenyl) pteridine (example 112): MS m/z (%) 530 ($[M+H]^+$, 100);
- 2-amino-4-[*N*-[4-(dimethylamino)acetyl]-piperazin-1-yl]-6-(3,4-dimethoxy-phenyl) pteridine (example 113): MS m/z (%) 453 ($[M+H]^+$, 100);
- 2-amino-4-[*N*-(4-amino-4-oxo-butanoyl)-piperazin-1-yl]-6-(3,4-dimethoxy-phenyl) pteridine (example 114): MS m/z (%) 467 ($[M+H]^+$, 100) ; and
- 2-amino-4-[*N*-(3-carboxypropanoyl)-piperazin-1-yl]-6-(3,4-dimethoxy-phenyl) pteridine (example 115): MS m/z (%) 468 ($[M+H]^+$, 100).

Example 116 synthesis of 2-amino-4-[*N*-[4-(carboxy)benzoyl]-piperazin-1-yl]-6-(3,4-dimethoxyphenyl) pteridine

2-amino-4-[*N*-[4-(methoxycarbonyl)benzoyl]-piperazin-1-yl]-6-(3,4-dimethoxyphenyl) pteridine (0.212 g, 1.4 mmole) was dissolved in THF (8 ml) and aqueous LiOH 0.1 N was added (8 ml). The mixture was stirred 24 hours at room temperature and the pH was adjusted at 3 with HCl 1 N. The precipitate was filtered, washed with $H_2O$, EtOAc, $Et_2O$ and dried in a vacuum dessicator over $P_2O_5$, yielding the title compound as a yellow powder which was characterized by its mass spectrum: MS m/z (%) 516 ($[M+H]^+$, 100).

Examples 117 to 119 - synthesis of 2-amino-4-[(*N*-alkyl-*N*-aryl)-carbamoyl-piperazin-1-yl)-6-(3,4-dimethoxyphenyl)pteridines

The synthesis of such compounds is achieved by the three-step procedure shown in figure 9, which is derived from the teaching of Batey et al. in Tetrahedron Lett. (1998) 39 : 6267-6270. The detailed procedure is as follows :

(a) to a suspension of carbonyl diimidazole (30.4 mmole, 4.93 g) in THF (50 ml) was added a suitable *N*-alkyl-aniline derivative (28 mmole), such as for example *N*-methylaniline (example 117), *N*-ethylaniline (example 118) or *N*-methyl-*p*-toluidine (example 119). The mixture was refluxed for 24 hours, after which an additional amount of carbonyl

diimidazole (2.24 g) was added. The reaction mixture was refluxed for another 6 hours, until the reaction reaches completion (TLC monitoring). After cooling down the reaction mixture, the solvents were evaporated *in vacuo* yielding the crude *N*-alkyl-aniline carbamoyl imidazoles which were used in the next step without any further purification.
(b) to a solution of the crude *N*-alkyl-aniline carbamoyl imidazole (32 mmole) in acetonitrile (50 ml) was added methyl iodide- (128 mmole). The mixture was stirred at room temperature for 24 hours. The solvent was evaporated *in vacuo* yielding N-alkyl-aniline carbamoyl *N*-methyl-imidazolium iodide.
(c) to a solution of 2-amino-4-(piperazin-1-yl)-6-(3,4-dimethoxy-phenyl)pteridine (224 mg, 0.61 mmole) in DMF (30 ml) was added triethylamine (111 μl, 0.80 mmole) and a suitable *N*-alkyl-aniline carbamoyl *N*-methyl-imidazolium iodide (0.92 mmole). The reaction mixture was stirred at room temperature for 24 hours. The reaction was diluted with $CH_2Cl_2$ and extracted 3 times with $H_2O$. Evaporation of the solvents *in vacuo,* followed by purification of the residue by silica gel column chromatography, the mobile phase being $CH_3OH/CH_2Cl_2$ mixtures (in a ratio gradually ranging from 2:98 to 3:97) provided each of the title compounds as a yellow powder, with a yield ranging from 65 to 80 %, depending on the *N*-alkyl-aniline derivative used.

The following compounds were synthesized following this procedure and characterised by their mass spectrum (MS) and ultraviolet spectrum (UV) as follows:

- 2-amino-4-(*N*-methyl-*N*-phenyl-carbamoyl-piperazin-1-yl)-6-(3,4-dimethoxyphenyl) pteridine (example 117) : MS : m/z (%) 501 ($[M+H]^+$, 100); UV (nm) : 213, 298, 412 ;
- 2-amino-4-(*N*-ethyl-*N*-phenyl-carbamoyl-piperazin-1-yl)-6-(3,4-dimethoxyphenyl) pteridine (example 118) : MS : m/z (%) 515 ($[M+H]^+$, 100) ; UV (nm) : 213, 298, 413 ; and
- 2-amino-4-(*N*-methyl-*N*-tolyl-carbamoyl-piperazin-1-yl)-6-(3,4-dimethoxyphenyl) pteridine (example 119) : MS : m/z (%) 515 ($[M+H]^+$, 100) ; UV (nm) : 213, 298, 412.

Example 120 - model of rheumatoid arthritis

Collagen type II (hereinafter referred as CII) induced experimental model of rheumatoid arthritis (hereinafter referred as RA) in DBA mice is widely accepted as the most relevant and predictive preclinical model for RA. In this model, DBA mice are immunized with CII, the collagen type mainly present in the joint structures, together with complete Freund Adjuvant in their tail. 2 to 3 weeks later, several of the immunized mice start to develop arthritis in the four footpaths. In order to further worsen the disease, mice are given a second CII boost at three weeks after the first immunisation, this time however in a footpath. Because the immune system is already immunised in these mice, this rapidly provokes a severe swelling of the injected footpath (named Delayed Type Hypersensitivity or DTH) which can be used as a measurement for T-cell activation. Within a few days after the booster, almost all untreated animals start developing symptoms of arthritis. RA development is scored from 0 to 16 (16 being severe clinical arthritis in all four footpaths). At the end of the study (3 weeks after the CII boost) antibody formation was determined against CII and histology performed on the footpaths.

The efficiency of the pteridine derivative of example 17 (administered in an amount of 20 mg/kg/day, started one day before the CII boost) was explored in this CII-model. All such treated animals developed significantly less severe rheumatoid arthritis (clinical scores ranging from 2 to 4), as compared to untreated control mice (clinical scores ranging from 6 to 12) and also compared to mice treated with methotrexate (clinical scores ranging from 2 to 7), the most effective compound for the treatment of RA to date.

Additionally, increasing the dose of the pteridine derivative of example 17 up to 40 mg/kg/day has no mortality or cytotoxic effect on mice *in vivo,* whereas increasing the dose of methotrexate (10 mg/kg/day, 3 times a week) leads to death of all animals.

Example 121 - model of protection against septic shock

As a control group, 4 sham treated (saline injection) C3H mice being injected intraperitoneously with 100 μg lipopolysaccharide (hereinafter LPS) per mouse, all died within 1 to 3 days after injection. However, when four C3H mice being injected intraperitoneously with 100 μg lipopolysaccharide (hereinafter LPS) per mouse were treated during 2 days with the pteridine derivative of example 17 (one first intraperitoneous injection of 20 mg/kg/day at the time of injection, and a second injection 24 hours later), all mice were protected from acute shock related mortality.

Examples 122 to 162 - synthesis of 2-amino-4-(N-substituted-piperazino)-6-(3,4-dimethoxy-phenyl) pteridines

The following procedure is similar to that of examples 64 to 66. To a suspension of the compound of example 6 (1 mmole) in dioxane (20 ml) was added a suitable N-substituted piperazine (1.5 mmole). The suspension was stirred

at room temperature for 16 hours. The solvents were evaporated in vacuo yielding crude 2-acetylamino-4-(N-substituted piperazino)-6-(3,4-dimethoxyphenyl) pteridine. Deprotection of the 2-acetylamino group was achieved by dissolving this crude compound in 20 ml of a 1:1 mixture of methanol and 20% $K_2CO_3$ in water. The solution was stirred for 16 hours at room temperature. Solvents were evaporated in vacuo and the residue was purified by preparative TLC (silica, using a $CH_3OH/CH_2Cl_2$ (5:95) mixture as an eluent), affording the following compounds as yellow powders in yields ranging from 20 to 70%:

- 2-amino-4-(1-(2-methoxyethyl)piperazino)-6-(3,4-dimethoxyphenyl) pteridine (example 122) was obtained from 1-(2-methoxyethyl) piperazine and characterised as follows: MS: m/z (%): 873 ([2M+Na]$^+$, 15), 426 ([M+H]+, 100];
- 2-amino-4-(1-cyclohexylmethyl)piperazino)-6-(3,4-dimethoxyphenyl) pteridine (example 123) was obtained from 1-(cyclohexylmethyl)piperazine and characterised as follows: MS: m/z (%): 949 ([2M+Na]$^+$, 5), 464 ([M+H]$^+$, 100];
- 2-amino-4-(1-cyclopentylpiperazino)-6-(3,4-dimethoxyphenyl) pteridine (example 124) was obtained from 1-cyclopentylpiperazine and characterised as follows: MS: m/z (%): 893 ([2M+Na]$^+$, 25), 436 ([M+H]$^+$, 100]; UV (MeOH, nm): 213, 296, 413;
- 2-amino-4-(1-butylpiperazino)-6-(3,4-dimethoxyphenyl) pteridine (example 125) was obtained from 1-(butyl)piperazine and characterised as follows: MS: m/z (%): 893 ([2M+Na]$^+$, 25), 436 ([M+H]$^+$, 100]; UV (MeOH, nm): 216, 295, 413;
- 2-amino-4-(1-isopropylpiperazino)-6-(3,4-dimethoxyphenyl) pteridine (example 126) was obtained from 1-(isopropyl) piperazine and characterised as follows: MS: m/z (%): 841 ([2M+Na]$^+$, 20), 410 ([M+H]$^+$, 100]; UV (MeOH, nm): 215, 295, 412;
- 2-amino-4-(1-(2-diethylaminoethyl)-piperazino)-6-(3,4-dimethoxyphenyl) pteridine (example 127) was obtained from 1-(2-diethylaminoethyl)-piperazine and characterised as follows: MS: m/z (%): 955 ([2M+Na]$^+$, 20), 437 ([M+H]$^+$, 100]; UV (MeOH, nm): 216, 297, 413;
- 2-amino-4-(1-(2-diisopropylaminoethyl)-piperazino)-6-(3,4-dimethoxyphenyl) pteridine (example 128) was obtained from 1-(2-diisopropylaminoethyl)-piperazine and

characterised as follows: MS: m/z (%): 495 ([M+H]$^+$, 100]; UV (MeOH, nm): 215, 297, 413;

- 2-amino-4-(1-(2-morpholino-4-yl-ethyl)-piperazino)-6-(3,4-dimethoxyphenyl) pteridine (example 129) was obtained from 1-(2-morpholino-4-yl-ethyl)-piperazine and

characterised as follows: MS m/z (%): 481 ([M+H]$^+$, 100]; UV (MeOH, nm): 217, 297, 414;

- 2-amino-4-(4-[2-(piperazin-1-yl)-acetyl]-morpholino)-6-(3,4-dimethoxy-phenyl) pteridine (example 130) was obtained from 4-[2-(piperazin-1-yl)-acetyl] morpholine and

characterised as follows: MS: m/z (%): 495 ([M+H]$^+$, 100]; UV (MeOH, nm): 219, 297, 414;

- 2-amino-4-(4-[2-(piperazin-1-yl)-acetyl]-pyrrolidino)-6-(3,4-dimethoxy-phenyl) pteridine (example 131) was obtained from 4-[2-(piperazin-1-yl)-acetyl] pyrrolidine and

characterised as follows: MS: m/z (%): 979 ([2M+Na)$^+$, 20], 479 ([M+H]$^+$, 100]; UV (MeOH, nm): 219, 307, 411;

- 2-amino-4-(2-[piperazin-1-yl]-acetic acid N-methyl N-phenyl amide)-6-(3,4-dimethoxyphenyl) pteridine (example 132) was obtained from 2-[piperazin-1-yl]-acetic acid N-methyl-N-phenylamide and characterised as follows: MS: m/z (%): 515 ([M+H]$^+$, 100]; UV (MeOH, nm): 219, 307, 411;
- 2-amino-4-(2-(piperazin-1-yl)-propionic acid ethyl ester)-6-(3,4-dimethoxyphenyl) pteridine (example 133) was obtained from 2-(piperazin-1-yl)-propionic acid ethyl ester (in order to avoid transesterification, a mixture of ethanol and sodium was used for the deprotection of the acetyl group) and characterised as follows: MS: m /z (%): 468 ([M+H]$^+$, 100]; UV (MeOH, nm) : 216, 297, 413;
- 2-amino-4-(3-(piperazin-1-yl)-propionic acid ethyl ester)-6-(3,4-dimethoxyphenyl) pteridine (example 134) was obtained from 3-(piperazin-1-yl)-propionic acid ethyl ester (in order to avoid transesterification, a mixture of ethanol and sodium (15 equivalents) was used for the deprotection of the acetyl group) and characterised as follows: MS: m/z (%): 957 ([2M+Na]$^+$, 10)], 468 ([M+H]$^+$, 100]; UV (MeOH, nm) : 216, 296, 412;
- 2-amino-4-(2-(piperazin-1-yl)-acetic acid ethyl ester)-6-(3,4-dimethoxyphenyl) pteridine (example 135) was obtained from 3-(piperazin-1-yl)-propionic acid ethyl ester (in order to avoid transesterification, a mixture of ethanol and sodium (15 equivalents) was used for the deprotection of the acetyl group) and characterised as follows: MS: m/z (%): 454 ([M+H]$^+$, 100]; UV (MeOH, nm) : 216, 297, 413;

- 2-amino-4-(1-(3-methyl-benzyl)piperazinyl)-6-(3,4-dimethoxyphenyl) pteridine (example 136) was obtained from 1-(3-methylbenzyl)-piperazine and characterised as follows: MS: *m*/*z* (%): 965 ($[2M+Na]^+$, 10), 472 ([M+H]+, 100];
- 2-amino-4-[(2,6-dichlorobenzyl)piperazin-1-yl]-6-(3,4-dimethoxyphenyl) pteridine (example 137) was obtained from 1-(2,6-dichloro-benzyl)-piperazine and characterised as follows: MS: m/z (%): 526 ($[M+H]^+$, 100);
- 2-amino-4-((4-fluorobenzyl)piperazin-1-yl)-6-(3,4-dimethoxyphenyl) pteridine (example 138) was obtained from 1-(4-fluorobenzyl)-piperazine and characterised as follows: MS: m/z (%): 476 ($[M+H]^+$, 100);
- 2-amino-4-((4-chlorobenzyl)piperazin-1-yl)-6-(3,4-dimethoxyphenyl) pteridine (example 139) was obtained from 1-(4-chloro-benzyl)-piperazine and characterised as follows: MS: *m*/*z* (%): 492 ($[M+H]^+$, 100);
- 2-amino-4-((4-methylbenzyl)piperazin-1-yl)-6-(3,4-dimethoxyphenyl) pteridine (example 140) was obtained from 1-(4-methyl-benzyl)-piperazine and characterised as follows: MS: *m*/*z* (%): 472 ($[M+H]^+$, 100);
- 2-amino-4-((2-fluorobenzyl)piperazin-1-yl)-6-(3,4-dimethoxyphenyl) pteridine (example 141) was obtained from 1-(2-fluorobenzyl)-piperazine and characterised as follows: MS: m/z (%): 476 ($[M+H]^+$, 100);
- 2-amino-4-((3,4-dichlorobenzyl)piperazin-1-yl)-6-(3,4-dimethoxyphenyl) pteridine (example 142) was obtained from 1-(3,4-dichlorobenzyl)-piperazine and characterised as follows: MS: m/z (%): 526 ($[M+H]^+$, 100);
- 2-amino-4-(piperonyl-piperazin-1-yl)-6-(3,4-dimethoxyphenyl) pteridine (example 143) was obtained from 1-piperonyl-piperazine and characterised as follows: MS: m/z (%): 502 ($[M+H]^+$, 100);
- 2-amino-4-((4-*tert*-butylbenzyl)piperazin-1-yl)-6-(3,4-dimethoxyphenyl) pteridine (example 144) was obtained from 1-(4-*tert*-butyl-benzyl) piperazine) and characterised as follows: MS: *m*/*z* (%): 514 ($[M+H]^+$, 100);
- 2-amino-4-((4-pyridyl)-piperazin-1-yl)-6-(3,4-dimethoxyphenyl) pteridine (example 145) was obtained from 1-(4-pyridyl)-piperazine and characterised as follows: MS: m/z (%): 445 ($[M+H]^+$, 100); UV (MeOH, nm): 213, 289, 411;
- 2-amino-4-((2-pyridyl-piperazin-1-yl)-6-(3,4-dimethoxyphenyl) pteridine (example 146) was obtained from 1-(2-pyridyl)-piperazine and characterised as follows: MS: *m*/z (%): 911 ($[2M+Na]^+$, 60), 889 ($[2M+H]^+$, 60), 445 ($[M+H]^+$, 100); UV (MeOH, nm): 215, 247, 297, 413;
- 2-amino-4-((2-pyrimidinyl)-piperazin-1-yl)-6-(3,4-dimethoxyphenyl) pteridine (example 147) was obtained from 1-(2-pyrimidinyl)-piperazine and characterised as follows: MS: *m*/*z* (%): 446 ($[M+H]^+$, 100); UV (MeOH, nm): 216, 244, 297, 413;
- 2-amino-4-((3-methoxyphenyl)-piperazin-1-yl)-6-(3,4-dimethoxyphenyl) pteridine (example 148) was obtained from 1-(3-methoxyphenyl)-piperazine and characterised as follows: MS: m/z (%): 969 ($[2M+Na]^+$, 15), 446 ($[M+H]^+$, 100); UV (MeOH, nm): 215, 295, 413;
- 2-amino-4-(1-(3-phenylpropyl-piperazine)-6-(3,4-dimethoxyphenyl) pteridine (example 149) was obtained from 1-(3-phenylpropyl)-piperazine and characterised as follows: MS: m/z (%): 486 ($[M+H]^+$, 100); UV (MeOH, nm): 217, 296, 413;
- 2-amino-4-((3,4-dichlorophenyl)-piperazin-1-yl)-6-(3,4-dimethoxyphenyl pteridine (example 150) was obtained from 1-(3,4-dichlorophenyl)-piperazine and characterised as follows: MS: *m*/*z* (%): 512 ($[M+H]^+$, 100); UV (MeOH, nm): 213, 263, 295, 412;
- 2-amino-4-((3-dichlorophenyl)-piperazin-1-yl)-6-(3,4-dimethoxyphenyl) pteridine (example 151) was obtained from 1-(3-dichlorophenyl)-piperazine and characterised as follows: MS: *m*/*z* (%): 478 ($[M+H]^+$, 100); UV (MeOH, nm): 213, 257, 296, 413;
- 2-amino-4-((1-phenylethyl)-piperazin-1-yl)-6-(3,4-dimethoxyphenyl) pteridine (example 152) was obtained from 1-(1-phenylethyl)-piperazine and characterised as follows: MS: *m*/*z* (%): 965 ($[2M+Na]^+$, 10), 472 ($[M+H]^+$, 100); UV (MeOH, nm): 216, 298, 413;
- 2-amino-4-((2-(1-pyrrolyl)-ethyl-piperazin-1-yl)-6-(3,4-dimethoxyphenyl) pteridine (example 153) was obtained from 1-[2-(1-(pyrrolyl)-ethyl]-piperazine and characterised as follows: MS: *m*/*z* (%): 461 ($[M+H]^+$, 100); UV (MeOH, nm): 216, 297, 413;
- 2-amino-4-((2-phenoxyethyl-piperazin-1-yl)-6-(3,4-dimethoxyphenyl) pteridine (example 154) was obtained from 1-(2-phenoxyethyl)-piperazine and characterised as follows: MS: *m*/*z* (%): 488 ($[M+H]^+$, 100); UV (MeOH, nm): 216, 297, 413;
- 2-amino-4-(1-(2-imidazol-1-yl-ethyl-piperazine)-6-(3,4-dimethoxyphenyl) pteridine (example 155) was obtained from 1-(2-imidazol-1-yl-ethyl)-piperazine and characterised as follows: MS: m/z (%): 945 ($[2M+Na]^+$, 10), 462 ($[M+H]^+$, 100); UV (MeOH, nm): 216, 297, 413;
- 2-amino-4-((3-pyridyl)-methyl)-piperazin-1-yl)-6-(3,4-dimethoxyphenyl) pteridine (example 156) was obtained from 1-(3-pyridyl)-methyl-piperazine and characterised as follows: MS: m/z (%): 939 ($[2M+Na]^+$, 15), 459 ($[M+H]^+$, 100); UV (MeOH, nm): 215, 297, 413;
- 2-amino-4-((4-pyridyl)-methyl)-piperazin-1-yl)-6-(3,4-dimethoxyphenyl) pteridine (example 157) was obtained from 1-(4-pyridyl)-methyl-piperazine and characterised as follows: MS: *m*/*z* (%): 939 ($[2M+Na]^+$, 15), 459 ($[M+H]^+$, 100); UV (MeOH, nm): 218, 297, 414;
- 2-amino-4-((1-naphtylmethyl)-piperazin-1-yl)-6-(3,4-dimethoxyphenyl) pteridine (example 158) was obtained from 1-(1-naphtylmethyl-piperazine and characterised as follows: MS: m/z (%): 508 ($[M+H]^+$, 100); UV (MeOH, nm): 223,

297, 413;

- 2-amino-4-(*N*-phenethylpiperazin-1-yl)-6-(3,4-dimethoxyphenyl) pteridine (example 159) was obtained from *N*-phenethylpiperazine and characterised as follows: MS: *m*/*z* (%): 965 ([2M+Na]$^+$, 10), 472 ([M+H]$^+$, 100); UV (MeOH, nm): 215, 297, 413;
- 2-amino-4-((2-methoxyphenyl)-piperazin-1-yl)-6-(3,4-dimethoxyphenyl) pteridine (example 160) was obtained from 1-(2-methoxyphenyl)-piperazine and characterised as follows: MS: m/z (%): 474 ([M+H]$^+$, 100); UV (MeOH, nm): 213, 295, 413;
- 2-amino-4-((4-methoxyphenyl)-piperazin-1-yl)-6-(3,4-dimethoxyphenyl) pteridine (example 161) was obtained from 1-(4-methoxyphenyl)-piperazine and characterised as follows: MS: m/z (%): 474 ([M+H]$^+$, 100); UV (MeOH, nm): 212, 296, 413; and
- 2-amino-4-((4-chlorophenyl)-piperazin-1-yl)-6-(3,4-dimethoxyphenyl) pteridine (example 162) was obtained from 1-(4-chlorophenyl)piperazine and characterised as follows: MS: m/z (%): 478 ([M+H]$^+$, 100); UV (MeOH, nm): 258, 296, 413.

Examples 163 to 180 - synthesis of 2-amino-6-(3,4-dimethoxyphenyl)-4-(substituted piperazin-1-yl) pteridines

The following procedure is in accordance with the scheme shown in figure 3. A mixture of the compound of example 4 (299 mg, 1.0 mmole), 1,1,1,3,3,3-hexamethyldisilazane (1 ml, 4.7 mmole), a N-substituted piperazine (4.0 mmole), p-toluenesulfonic acid (20 mg, 0.1 mmole) and ammonium sulfate (20 mg, 0.15 mmole) in toluene (4 ml) was refluxed for 48 hours (the reaction mixture became clear when the reaction was finished). After removing the solvents under reduced pressure, the residue was purified by flash chromatography over silica ($CH_3OH/CH_2Cl_2$ 1:20 to 1:30) reaching the desired following compounds as yellow solids in yields indicated below:

- 2-amino-6-(3,4-dimethoxyphenyl)-4-(4-(3-propionitrit)-piperazin-1-yl) pteridine (example 163) was obtained from 3-(1-piperazinyl)-propionitrile in 43 % yield and characterized as follows: Rf = 0.50 ($MeOH/CH_2Cl_2$=1/9); UV (MeOH/$H_2O$, nm): 215, 297, 412; MS (m/z): 421 ([M+H]$^+$, 100);
- 2-amino-6-(3,4-dimethoxyphenyl)-4-(4-(2-(1,3)-dioxolan-2-yl-ethyl)-piperazin-1-yl) pteridine (example 164) was obtained from 2-[2-(piperazin-1-yl)-ethyl]-1,3-dioxolane in 55 % yield and characterized as follows: Rf = 0.49 ($MeOH/CH_2Cl_2$=1/9); UV (MeOH/$H_2O$, nm): 215, 295, 412; MS (m/z): 468 ([M+H]$^+$, 100);
- 2-amino-6-(3,4-dimethoxyphenyl)4-(4-(2-ethoxyethyl)-piperazin-1-yl) pteridine (example 165) was obtained from 1-(2-ethoxyethyl)-piperazine in 35 % yield and characterized as follows: Rf = 0.33 ($MeOH/CH_2Cl_2$=1/9); UV (MeOH/$H_2O$, nm): 213, 295, 412; MS (m/z): 440 ([M+H]$^+$, 100);
- 2-amino-6-(3,4-dimethoxyphenyl)-4-(pent-3-yl-piperazin-1-yl) pteridine (example 166) was obtained from 1-(3-pentyl)-piperazine in 22 % yield and characterized as follows: Rf = 0.43 ($MeOH/CH_2Cl_2$=1/9); UV (MeOH/$H_2O$, nm): 212, 293, 412; MS (m/z): 438.2 ([M+H]$^+$,100);
- 2-amino-6-(3,4-dimethoxyphenyl)-4-(1-pentyl-piperazin-1-yl) pteridine (example 167) was obtained from 1-(1-pentyl)-piperazine in 22 % yield and characterized as follows: Rf = 0.54 ($MeOH/CH_2Cl_2$=1/9); UV (MeOH/$H_2O$, nm): 212, 294, 412; MS (m/z): 438 ([M+H], 100);
- 2-amino-6-(3,4-dimethoxyphenyl)-4-(1-isobutyl-piperazin-1-yl) pteridine (example 168) was obtained from 1-isobutylpiperazine in 26 % yield and characterized as follows: Rf = 0.42 ($MeOH/CH_2Cl_2$=1/9); UV (MeOH/$H_2O$, nm): 213, 294, 412; MS (m/z): 424 ([M+H]$^+$, 100);
- 2-amino-6-(3,4-dimethoxyphenyl)-4-((tetrahydrofurfuryl)-piperazin-1-yl) pteridine (example 169) was obtained from 1-tetrahydrofurfuryl-piperazine in 31 % yield and

characterized as follows: Rf = 0.37 ($MeOH/CH_2Cl_2$=1/9); UV (MeOH/$H_2O$, nm): 215, 295, 412; MS (m/z): 453 ([M+H]$^+$, 100);

- 2-amino-6-(3,4-dimethoxyphenyl)-4-(1,3-dioxolan-2-yl-methylpiperazin-1-yl) pteridine (example 170) was obtained from 2-(piperazin-1-yl-methyl)-1,3-dioxolane in 65 % yield and characterized as follows: Rf = 0.46 ($MeOH/CH_2Cl_2$=1/9); UV (MeOH/$H_2O$, nm): 217, 297, 413; MS (m/z): 454 ([M+H]$^+$, 100);
- 2-amino-4-((3,5-dichlorophenyl)-piperazin-1-yl)-6-(3,4-dimethoxyphenyl) pteridine (example 171) was obtained from 1-(3,5-dichlorophenyl)-piperazine in 83 % yield and

characterised as follows: Rf = 0.70 ($MeOH/CH_2Cl_2$=1/9); UV (MeOH/$H_2O$, nm): 216, 263, 296, 413; MS (m/z): 512.2 ([M+H]$^+$, 100);

- 2-amino-6-(3,4-dimethoxyphenyl)-4-((4-fluorophenyl)-piperazin-1-yl) pteridine (example 172) was obtained from 1-(4-fluororopheny)-piperazine in 20 % yield and characterised as follows: Rf = 0.53 ($MeOH/CH_2Cl_2$=1/9); UV (Me-

OH/$H_2O$, nm): 212, 296, 413; MS (m/z): 462.2 ($[M+H]^+$, 100);

- 2-amino-6-(3,4-dimethoxyphenyl)-4-((3-trifluoromethylphenyl)-piperazin-1-yl) pteridine (example 173) was obtained from 1-(3-trifluororomethylphenyl)-piperazine in 71 % yield and characterised as follows: Rf = 0.58 (MeOH/$CH_2Cl_2$=1/9); UV (MeOH/$H_2O$, nm): 212, 257, 296, 413; MS (m/z): 512.2 ($[M+H]^+$, 100);
- 2-amino-6-(3,4-dimethoxyphenyl)-4-((3,4-dimethylphenyl)-piperazin-1-yl) pteridine (example 174) was obtained from 1-(3,4-dimethylphenyl)-piperazine in 48 % yield and

characterised as follows: Rf = 0.58 (MeOH/$CH_2Cl_2$=1/9); UV (MeOH/$H_2O$, nm): 243, 296, 413; MS (m/z): 472.3 ($[M+H]^+$, 100);

- 2-amino-6-(3,4-dimethoxyphenyl)-4-(3-methylphenyl)-piperazin-1-yl) pteridine (example 175) was obtained from 1-(3-methylphenyl)-piperazine in 59 % yield and characterised as follows: Rf = 0.47 (MeOH/$CH_2Cl_2$=1/9); UV (MeOH/$H_2O$, nm): 218, 244, 297, 413; MS (m/z): 458.2 ($[M+H]^+$, 100);
- 2-amino-6-(3,4-dimethoxyphenyl)-4-((4-methylphenyl)-piperazin-1-yl) pteridine (example 176) was obtained from 1-(4-methylphenyl)-piperazine in 59 % yield and characterised as follows: Rf = 0.50 (MeOH/$CH_2Cl_2$=1/9); UV (MeOH/$H_2O$, nm): 213, 242, 296, 413; MS (m/z): 458.2 ($[M+H]^+$, 100);
- 2-amino-6-(3,4-dimethoxyphenyl)-4-((2-pyridyl)methyl-piperazin-1-yl) pteridine (example 177) was obtained from 1-((2-pyridyl)-methyl)-piperazine in 27 % yield and characterised as follows: Rf = 0.38 (MeOH/$CH_2Cl_2$=1/9); UV (MeOH/$H_2O$, nm): 216, 297, 413; MS (m/z): 459.2 ($[M+H]^+$, 100);
- 2-amino-6-(3,4-dimethoxyphenyl)-4-(thiazol-2-yl)-piperazin-1-yl) pteridine (example 178) was obtained from 1-thiazol-2-yl-piperazine in 11 % yield and characterised as follows: Rf = 0.55 (MeOH/$CH_2Cl_2$=1/9); UV (MeOH/$H_2O$, nm): 213, 294, 413; MS (m/z): 451.2 ($[M+H]^+$, 100);
- 2-amino-6-(3,4-dimethoxyphenyl)-4-(1-(1-methyl-piperidin-3-yl-metnyl)-piperazin-1-yl) pteridine (example 179) was obtained from 1-(1-methyl-piperidin-3-yl-methyl)-piperazine in 48 % yield and characterised as follows: MS (m/z): 479 ($[M+H]^+$ 100); UV (MeOH/$H_2O$, nm): 217, 266, 297, 412; and
- 2-amino-6-(3,4-dimethoxyphenyl)-4-((4-trifluoromethylphenyl)-piperazin-1-yl) pteridine (example 180) was obtained from 1-(4-trifluororomethylphenyl)-piperazine in 48 % yield and characterised as follows: Rf = 0.55 (MeOH/$CH_2Cl_2$=1/9); UV (MeOH/$H_2O$, nm): 212, 266, 294, 412; MS (m/z): 512.2 ($[M+H]^+$, 100).

Examples 181 to 184 - synthesis of 2-amino-6-(3,4-dimethoxyphenyl)-4- (substituted piperazin-1-yl) pteridine trihydrochloride salts

A mixture of the compound of example 4 (299 mg, 1.0 mmole), 1,1,1,3,3,3-hexamethyldisilazane (1 ml, 4.7 mmole), a N-substituted piperazine (4.0 mmole), p-toluenesulfonic acid (20 mg, 0.1 mmole) and ammonium sulfate (20 mg, 0.15 mmole) in toluene (4 ml) was refluxed for 48 hours (the reaction mixture became clear when the reaction was finished). After removing the solvents under reduced pressure, the residue was purified by flash chromatography over silica ($CH_3OH/CH_2Cl_2$ 1:20 to 1:30). To a solution of the resulting free pteridine base in methanol (20 ml), 1.25 M HCl in MeOH (4 ml, 5.0 mmole) was slowly added. The mixture was stirred at room temperature for one hour. The precipitate (which is the trihydrochloride salt of the free pteridine base) was filtered off and washed with methanol. Drying in the vacuum over $P_2O_5$ yielded the corresponding hydrochloride salt as a yellow solid. The following salts were made according to this procedure, with yields indicated below:

- 2-amino-6-(3,4-dimethoxyphenyl)-4-(4-(2-dimethylaminoethyl)-piperazin-1-yl)-pteridine trihydrochloride salt (example 181) obtained in 58% yield was characterised as follows: Rf = 0.20 (MeOH/$Et_3N/CH_2Cl_2$=4/2/100); UV (MeOH/$H_2O$, nm): 215, 297, 412; MS (m/z): 439 ($[M-3HCl+H]^+$, 100);
- 2-amino-6-(3,4-dimethoxyphenyl)-4-(4-(3-dimethylaminopropyl)-piperazin-1-yl) pteridine trihydrochloride salt (example 182) obtained in 57 % yield was characterised as follows: Rf = 0.25 (MeOH/$Et_3N/CH_2Cl_2$=4/2/100); UV (MeOH/$H_2O$, nm): 214, 296, 412; MS (m/z): 453 ($[M-3HCl+H]^+$, 100);
- 2-amino-6-(3,4-dimethoxyphenyl)-4-(4-(2-dipropylaminoethyl)-piperazin-1-yl) pteridine trihydrochloride salt (example 183) obtained in 52 % yield was characterised as follows: Rf = 0.40 (MeOH/$Et_3N/CH_2Cl_2$=4/2/100); UV (MeOH/$H_2O$, nm): 216, 297, 413; MS (m/z): 495 ($[M-3HCl+H]^+$, 100); and
- 2-amino-6-(3,4-dimethoxyphenyl)-4-(4-(2-piperidin-1-yl-ethyl)-piperazin-1-yl) pteridine trihydrochloride salt (example 184) obtained in 44 % yield was characterised as follows: Rf = 0.35 (MeOH/$Et_3N/CH_2Cl_2$=4/2/100); UV (MeOH/$H_2O$, nm): 216, 297, 412; MS (m/z): 479 ($[M-3HCl+H]^+$, 100).

Examples 185 to 187 - synthesis of 2-amino-4-(N-substituted-piperazino)-6-(3,4-dimethoxyphenyl) pteridines

While repeating the experimental procedure of examples 64-66 and 133-162, the three following compounds

were obtained as yellow powders:

- 2-amino-4-[4-trifluoromethyl-2-nitro-phenyl-piperazin-1-yl)-6-(3,4-dimethoxyphenyl) pteridine (example 185) was obtained from 1-(4-trifluoromethyl-2-nitro-phenyl)-piperazine and characterised as follows : MS m/z (%) 557 ($[M+H]^+$, 100) ;
- 2-amino-4-[2-trifluoromethyl-4-nitro-phenyl-piperazin-1-yl)-6-(3,4-dimethoxyphenyl) pteridine (example 186) was obtained from 1-(2-trifluoromethyl-4-nitro-phenyl)-piperazine and characterised as follows : MS m/z (%) 557 ($[M+H]^+$, 100) ; and
- 2-amino-4-[2-(piperazin-1-yl)-acetic acid N-(2-thiazolyl)-amide]-6-(3,4-dimethoxyphenyl) pteridine (example 187) was obtained from 2-(piperazin-1-yl)-acetic acid *N*-(2-thiazolyl)-amide and characterised as follows : MS m/z (%) 508 ($[M+H]^+$, 100).

Example 188 - mixed lymphocyte reaction assay

Pteridine derivatives were first dissolved (10mM) in dimethylsulfoxide (hereinafter referred as DMSO) and further diluted in culture medium before use for the following *in vitro* experiments. The commercially available culture medium consisted of RPMI-1640 + 10% foetal calf serum (FCS). Some pteridine derivatives described in the previous examples (as indicated in table 1) were tested in the following mixed lymphocyte reaction (MLR) assay.

Peripheral blood mononuclear cells (hereinafter referred as PBMC) were isolated from heparinized peripheral blood by density gradient centrifugation over Lymphoprep (Nycomed, Maorstua, Norway). Allogeneic PBMC or Eppstein-Barr Virus-transformed human B cells [commercially available under the trade name RPMI1788 (ATCC name CCL156)] which strongly express B7-1 and B7-2 antigens were used as stimulator cells after irradiation with 30 Gy. MLR was performed in triplicate wells. After 5 days incubation at 37°C, 1 µCi [$^3$H]-thymidine was added to each cup. After a further 16 hours incubation, cells were harvested and counted in a ß-counter. Inhibition of proliferation by a compound (drug) described in some of the previous examples was counted using the formula:

$$\%\ \text{inhibition} = \frac{(\text{cpm + drugs}) - (\text{cpm cult. med})}{(\text{cpm - drugs}) - (\text{OD cult. med})} \times 100$$

wherein cpm is the thymidine count per minute. The MLR assay is regarded by those skilled in the art as an *in vitro* analogue of the transplant rejection since it is based on the recognition of allogeneic major histocompatibility antigens on the stimulator leukocytes, by responding lymphocytes.

Table 1 below shows the $IC_{50}$ values for various pteridine derivatives i n the MLR test. The $IC_{50}$ value represents the lowest concentration of the pteridine derivative (expressed in µmole/l) that resulted in a 50% suppression of the MLR.

TABLE 1

| Example n° | MLR | Example n° | MLR | Example n° | MLR |
|---|---|---|---|---|---|
| 8 | 0.9 | 29 | 0.0005 | 49 | 0.4 |
| 9 | 0.5 | 30 | 0.1 | 50 | 0.065 |
| 10 | 0.1 | 31 | 3.0 | 51 | 0.3 |
| 11 | 0.4 | 32 | 0.4 | 55 | 5.1 |
| 12 | 0.3 | 36 | 0.8 | 56 | 2.8 |
| 13 | 0.1 | 37 | < 0.001 | 57 | 1.9 |
| 14 | 0.2 | 38 | 0.3 | 58 | 7.6 |
| 15 | 0.1 | 39 | 0.7 | 60 | 4.1 |
| 16 | 0.8 | 40 | 0.6 | 62 | 10 |
| 17 | 0.0038 | 41 | 0.5 | 63 | 10 |
| 18 | 0.3 | 42 | 0.4 | 64 | 0.4 |
| 19 | 2.4 | 43 | 0.2 | 65 | 0.9 |

(continued)

| Example n° | MLR | Example n° | MLR | Example n° | MLR |
|---|---|---|---|---|---|
| 20 | 0.5 | 44 | 0.2 | 66 | 0.8 |
| 21 | 6.0 | 45 | 0.09 | 79 | 4.1 |
| 26 | 0.5 | 46 | 0.6 | 80 | 4.7 |
| 27 | 0.08 | 47 | 0.3 | 82 | 3.2 |
| 28 | 0.03 | 48 | 0.3 | 87 | 8.4 |
| 89 | 0.087 | 90 | 0.058 | 91 | 0.052 |
| 92 | 0.05 | 93 | 0.07 | 94 | 0.1 |
| 95 | 0.12 | 96 | 0.15 | 97 | 0.24 |
| 98 | 0.12 | 99 | 0.0034 | 100 | 0.0074 |
| 101 | 0.0008 | 102 | 0.074 | 103 | 0.0065 |
| 104 | 0.018 | 105 | 0.037 | 106 | 0.058 |
| 107 | 0.003 | 108 | 0.034 | 109 | 0.1 |
| 111 | 8.8 | 118 | 0.9 | 119 | 0.3 |
| 122 | 0.8 | 123 | 3.9 | 124 | 0.9 |
| 125 | 0.8 | 126 | 4.9 | 127 | 9.1 |
| 128 | 7.4 | 129 | 2.5 | 130 | 1.5 |
| 131 | 0.3 | 132 | < 0.1 | 133 | 1.2 |
| 136 | 1.4 | 137 | 7.6 | 138 | 6.0 |
| 141 | 0.7 | 144 | 4.3 | 145 | 3.5 |
| 146 | 0.2 | 147 | 0.2 | 148 | 0.3 |
| 149 | 0.5 | 150 | 1.0 | 151 | 0.6 |
| 152 | 1.2 | 153 | 0.2 | 154 | 0.1 |
| 155 | 3.6 | 156 | 0.6 | 157 | 0.5 |
| 158 | 0.8 | 159 | 0.11 | 160 | 3.8 |
| 161 | 0.4 | 162 | 0.8 | 135 | 0.6 |
| 163 | 0.5 | 164 | 2.3 | 165 | 2.9 |
| 166 | 5.3 | 167 | 3.8 | 168 | 1.6 |
| 169 | 0.8 | 171 | 3.4 | 172 | 0.1 |
| 173 | 0.7 | 174 | 0.5 | 175 | 0.4 |
| 176 | 0.3 | 177 | 0.8 | 178 | 1.3 |
| 179 | 7.9 | 183 | 8.2 | 184 | 7.7 |
| 185 | 1.9 | 186 | 5.0 | 187 | 0.1 |

Example 189 - TNF-α assay

Peripheral blood mononuclear cells (herein referred as PBMC), in response to stimulation by lipopolysaccharide (hereinafter LPS), a gram-negative bacterial endotoxin, produce various chemokines, in particular human TNF-α. Inhibition of the activation of PBMC can therefore be measured by the level of suppression of the production of TNF-α by PBMC in response to stimulation by LPS.

Such inhibition measurement was performed as follows: PBMC were isolated from heparinized peripheral blood by density gradient centrifugation over Lymphoprep (commercially available from Nycomed, Norway). LPS was then

added to the PMBC suspension in complete medium ($10^6$ cells /ml) at a final concentration of 1 μg/ml. The pteridine derivative to be tested was added at different concentrations (0.1 μM, 1 μM and 10 μM) and the cells were incubated at 37°C for 72 hours in 5% $CO_2$. The supernatants were collected, then TNF-α concentrations were measured with respectively an anti-TNF-α antibody in a sandwich ELISA (Duo Set ELISA human TNFα, commercially available from R&D Systems, United Kingdom). The colorimetric reading of the ELISA was measured by a Multiskan RC plate reader (commercially available from ThermoLabsystems, Finland) at 450 nm (reference wavelength: 690 nm). Data analysis was performed with Ascent software 2.6. (also from ThermoLabsystems, Finland): a standard curve (recombinant human TNFα) was drawn and the amount (pg/ml) of each sample on the standard curve was determined.

The % suppression of human TNFα production by the pteridine derivatives of the invention (drugs) was calculated using the formula:

$$\text{\% suppression} = \frac{\text{pg/ml in drugs} - \text{pg/ml in cult. med.}}{(\text{pg/ml in cult. med.+ LPS}) - \text{pg/ml cult. med.}}$$

Table 2 below shows the $IC_{50}$ values (expressed in μM) of the tested pteridine derivatives in the TNF-α assay.

TABLE 2

| Example n° | TNF-α | Example n° | TNF-α | Example n° | TNF-α |
|---|---|---|---|---|---|
| 8 | 0.4 | 29 | 0.077 | 48 | 0.08 |
| 9 | 0.14 | 30 | 0.3 | 49 | 0.07 |
| 10 | 0.07 | 31 | 0.5 | 50 | 0.02 |
| 11 | 0.4 | 32 | 0.4 | 51 | 0.04 |
| 12 | 0.2 | 36 | 0.3 | 55 | 10 |
| 13 | 0.08 | 37 | 0.1 | 56 | 3.7 |
| 14 | 0.06 | 38 | 0.06 | 60 | 2.5 |
| 15 | 0.01 | 39 | 0.8 | 62 | 7.6 |
| 16 | 0.55 | 40 | 0.7 | 64 | 0.05 |
| 17 | 0.08 | 41 | 0.9 | 65 | 0.095 |
| 18 | 0.09 | 42 | 0.1 | 66 | 0.3 |
| 19 | 0.06 | 43 | 0.7 | 79 | 9.8 |
| 20 | 0.03 | 44 | 0.7 | 80 | 10.0 |
| 26 | 0.5 | 45 | 0.4 | 82 | 9.3 |
| 27 | 0.5 | 46 | 0.2 | 89 | 1.2 |
| 28 | 0.5 | 47 | 0.6 | 90 | 0.04 |
| 91 | 0.06 | 92 | 0.06 | 93 | 0.05 |
| 94 | 0.09 | 95 | 0.1 | 96 | 0.01 |
| 97 | 0.3 | 98 | 0.08 | 99 | 0.09 |
| 100 | 0.51 | 101 | 0.43 | 102 | 0.63 |
| 103 | 0.1 | 104 | 0.6 | 105 | 0.7 |
| 106 | 1.4 | 107 | 0.5 | 108 | 0.4 |
| 109 | 0.4 | 112 | 0.28 | 113 | 3.2 |

(continued)

| Example n° | TNF-α | Example n° | TNF-α | Example n° | TNF-α |
|---|---|---|---|---|---|
| 119 | 0.26 | 122 | 0.6 | 123 | 0.8 |
| 124 | 0.4 | 126 | 0.9 | 127 | 2.6 |
| 128 | 3.3 | 129 | 0.8 | 130 | 1.4 |
| 131 | 1.9 | 132 | 0.05 | 133 | 0.33 |
| 134 | 0.07 | 135 | 0.27 | 136 | 0.8 |
| | | 137 | 0.7 | 139 | 0.4 |
| 140 | 1.3 | 141 | 0.2 | 143 | 0.8 |
| 144 | 0.7 | 145 | 4.4 | 146 | 0.2 |
| 147 | 0.1 | 148 | 0.3 | 149 | 0.3 |
| 150 | 0.3 | 151 | 0.1 | 152 | 0.3 |
| 153 | 0.1 | 154 | 0.03 | 155 | 0.9 |
| 156 | 0.4 | 157 | 0.3 | 158 | 0.7 |
| 159 | 0.02 | 160 | 0.42 | 161 | 0.06 |
| 162 | 0.4 | 163 | 0.5 | 164 | 0.2 |
| 165 | 0.3 | 166 | 0.8 | 167 | 0.3 |
| 168 | 0.09 | 169 | 0.3 | 170 | 0.4 |
| 171 | 0.5 | 172 | 0.05 | 174 | 0.11 |
| 176 | < 0.01 | 177 | 0.09 | 178 | 0.15 |
| 179 | 1.8 | 180 | 1.4 | 181 | 8.8 |
| 182 | 4.8 | 183 | 1.2 | 184 | 0.7 |
| 185 | 0.5 | 186 | 0.4 | 187 | 0.06 |

Example 190 - protection against a lethal dose of TNF-α

A model of TNF-α induced shock in C57BU6 male mice was performed as follows. Five animals from the control group received an intravenous administration of a lethal dose of TNF-α (10 μg) in the tail. Ten animals from the test group received three intraperitoneous injections of the pteridine derivative of example 17 (20 mg/kg/day) respectively 48 hours, 24 hours and immediately before an intravenous injection of TNF-α (10 μg).

Body temperature, a clinical sign of TNF-induced shock, was followed for 40 hours in control mice and in mice receiving the pteridine derivative of example 17: the body temperature of control mice dropped significantly when compared to mice receiving the test compound of example 17.

Furthermore, all five mice from the control group died within 40 hours, the survival rate (80%) of mice that received the pteridine derivative of example 17 in addition to the TNF-α dose (10 μg) was quite substantial.

Example 191 - inhibition of the metastasis of melanoma cells in mice

C57BU6 mice were injected with $1.5 \times 10^6$ B16BL/6 melanomasarcoma cells subcutaneously in the foot path and were divided, three days later, into 4 groups:

- a control 1 group of 6 mice receiving only vehicle 3 times a week;
- a control 2 group of 5 mice receiving a lethal dose of TNF (15 μg, subcutaneously) 3 times a week;
- a group 3 receiving the pteridine derivative of example 17 alone 3 times a week at 20mg/kg
- a group 4 of 7 mice receiving the pteridine derivative of example 17 in an amount of 20 mg/kg intraperitenously on days 3, 4, and 5 and also receiving TNF (15 μg, subcutaneously) on day 5. This combined treatment with TNF and the pteridine derivative of example 17 was continued for 2 weeks.

Tumor size data (tumor size was measured as the largest diameter multiplied by the smallest diameter) show that the combined treatment in group 4 leads to a significant reduction of tumor size (120 $mm^2$) when compared to the control group 1 (tumor size: 440 $mm^2$). Reduction of tumor size is also true in mice of group 3, although to a lesser extent (198 $mm^2$).

All mice of control group 2 died within the very first days of treatment, whereas mortality was 3/7 in mice of group 4.

At the end of experiment, it was looked macroscopically at black metastasis in inguinal and/or para-aortic lymphoneuds in all tumor bearing groups of mice. The proportions of mice having metastasis were:

- 4/5 mice in control group 1,
- 0/4 mice in group 4, and
- 2/4 in group 3.

Example 192 - IL-1 β assay

Peripheral blood mononuclear cells (herein referred as PBMC), in response to stimulation by lipopolysaccharide (LPS), a gram-negative bacterial endotoxin, produce various chemokines, in particular human IL-1 β. Inhibition of the activation of PBMC can therefore be measured by the level of suppression of the production of IL-1 β by PBMC in response to stimulation by LPS.

Such inhibition measurement was performed as follows: PBMC were isolated from heparinized peripheral blood by density gradient centrifugation over Lymphoprep (commercially available from Nycomed, Norway). LPS was then added to the PMBC suspension in complete medium ($10^6$ cells /ml) at a final concentration of 1 μg/ml. The pteridine derivative to be tested was added at different concentrations (0.1 μM, 1 μM and 10 μM) and the cells were incubated at 37°C for 72 hours in 5% $CO_2$, The supernatants were collected, then IL-1 β concentrations were measured with an anti-IL-1 β antibody in a sandwich ELISA. The colorimetric reading of the ELISA was measured by a Multiskan RC plate reader (commercially available from ThermoLabsystems, Finland) at 450 nm (reference wavelength: 690 nm). Data analysis was performed with Ascent software 2.6. (also from ThermoLabsystems, Finland) : a standard curve (recombinant human IL-1 β) was drawn and the amount (pg/ml) of each sample on the standard curve was determined.

The % suppression of human IL-1 β by the pteridine derivatives (drugs) of this invention was calculated using the formula:

$$\text{\% suppression} = \frac{\text{pg/ml in drugs} - \text{pg/ml in cult. med.}}{(\text{pg/ml in cult. med.+ LPS}) - \text{pg/ml cult. med.}}$$

Table 3 below shows the $IC_{50}$ values (expressed in μM) of the tested pteridine derivatives in the IL-1 β assay.

TABLE 3

| Example n° | IL-1 β | Example n° | IL-1 β | Example n° | IL-1 β |
|---|---|---|---|---|---|
| 12 | 5.0 | 15 | 7.0 | 17 | 2.8 |
| 20 | 8.5 | 21 | 6.7 | 36 | 0.8 |
| 37 | 3.3 | 42 | 0.9 | 46 | 0.8 |
| 47 | 0.9 | 48 | 0.6 | 49 | 2.2 |
| 50 | 5.5 | 56 | 8.7 | 57 | 9.5 |
| 64 | 4.4 | 65 | 3.5 | 91 | 4.2 |
| 92 | 8.6 | 93 | 4.5 | 95 | 1.0 |
| 98 | 6.2 | 100 | 7.4 | 101 | 5.6 |
| 107 | 0.8 | 108 | 1.0 | 122 | 10 |
| 123 | 4.2 | 124 | 5.0 | 125 | 7.5 |
| 126 | 3.7 | 132 | 5.3 | 134 | 1.2 |

(continued)

| Example n° | IL-1 β | Example n° | IL-1 β | Example n° | IL-1 β |
|---|---|---|---|---|---|
| 136 | 4.7 | 139 | 1.2 | 141 | 0.6 |
| 146 | 5.0 | 147 | 4.3 | 148 | 7.2 |
| 149 | 4.9 | 150 | 3.5 | 151 | 3.7 |
| 152 | 3.6 | 153 | 2.0 | 154 | 2.2 |
| 157 | 2.3 | 159 | 5.4 | 161 | 3.2 |
| 162 | 3.8 | 166 | 4.4 | 167 | 3.7 |
| 172 | 5.1 | 173 | 4.6 | 174 | 5.0 |
| 176 | 4.9 | 178 | 4.0 | 180 | 4.4 |
| 187 | 2.4 | | | | |

## Claims

**1.** A pteridine derivative having the general formula (I):

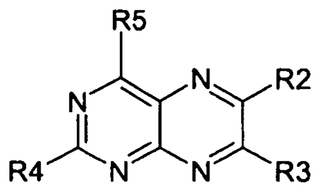

(I)

wherein:

- $R_5$ is a group represented by the general formula (II):

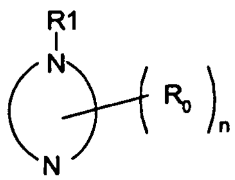

(II)

wherein:

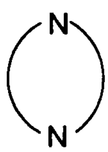

(III)

represents a piperazin-1-yl group or a homopiperazin-1-yl group, and wherein:

- each substituent $R_0$ of the heterocyclic ring (III) is a group independently selected from methyl and phenyl;
- n is 0, 1 or 2;
- $R_1$ is a substituent group selected from the group consisting of formyl, acyl, thio-acyl, amide, thioamide, sulfonyl, sulfinyl, carboxylate, thiocarboxylate, amino-substituted acyl, alkoxyalkyl, $C_{3-10}$ cycloalkyl-alkyl, $C_{3-10}$ cycloalkyl, dialkylaminoalkyl, heterocyclic-substituted alkyl, acyl-substituted alkyl, thioacyl-substituted alkyl, amido-substituted alkyl, thioamido-substituted alkyl, carboxylato-substituted alkyl, thiocarboxylato-substituted alkyl, (amino-substituted acyl)alkyl, heterocyclic, carboxylic acid ester, ω-cyanoalkyl, ω-carboxylic ester-alkyl, halo $C_{1-7}$ aikyi, $C_{2-7}$ alkenyl, $C_{2-7}$ alkynyl, arylalkenyl, aryloxyalkyl, arylalkyl and aryl, wherein the aryl moiety of each of said arylalkenyl, aryloxyalkyl, arylalkyl and aryl radicals is optionally substituted with one or more substituents independently selected from the group consisting of halogen, $C_{1-7}$ alkyl, $C_{2-7}$ alkenyl, $C_{2-7}$ alkynyl, halo $C_{1-7}$ alkyl, nitro, hydroxyl, sulfhydryl, amino, $C_{1-7}$ alkoxy, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thio-heterocyclic, arylalkylthio, heterocyclic-substituted alkylthio, formyl, sulfonamido, hydroxylamino, alkoxyamino, mercaptoamino, thioalkylamino, acylamino, thioacylamino, cyano, carboxylic acid or esters, alkylamino, cycloalkylamino, alkenylamino, cycloalkenylamino, alkynylamino, arylamino, aryl-alkylamino, hydroxyalkylamino, mercaptoalkylamino, and heterocyclic amino;
- $R_3$ is hydrogen,
- $R_4$ is amino;
- $R_2$ is a phenyl group optionally substituted with one or more substituents selected from the group consisting of halogen, $C_{1-7}$ alkyl and $C_{1-7}$ alkoxy;

and/or a pharmaceutically acceptable addition salt thereof and/or a stereoisomer thereof and/or a mono- or a di-*N*-oxide thereof and/or a solvate thereof and/or a dihydro- or tetrahydropteridine derivative thereof.

**2.** A pteridine derivative according to claim 1, wherein $R_5$ is a group selected from the group consisting of piperazin-1-yl, 2-methylpiperazin-1-yl, 2-phenylpiperazin-1-yl, homopiperazin-1-yl and 2,5-dimethylpiperazin-1-yl, the said group being substituted in its 4 position with a substituent $R_1$ which has a carbonyl (oxo) or thiocarbonyl (thioxo) or sulfonyl function.

**3.** A pteridine derivative according to claim 1 or claim 2, wherein $R_5$ is a piperazin-1-yl group substituted in its 4 position with a substituent $R_1$, wherein $R_1$ is selected from the group consisting of:

- $COR_8$ wherein $R_8$ is selected from the group consisting of hydrogen; $C_{1-7}$ alkyl; $C_{3-10}$ cycloalkyl; aryl optionally substituted with one or more substituents selected from the group consisting of halogen, $C_{1-7}$ alkyl, cyano and $C_{1-7}$ alkoxy; heterocyclic optionally substituted with one or more halogen atoms; arylalkyl; aryloxyalkyl; arylalkoxyalkyl; alkoxyalkyl; arylalkoxy; aryloxy; arylalkenyl; heterocyclic-substituted alkyl; alkylamino and arylamino,
- $CSR_9$, wherein $R_9$ is selected from the group consisting of alkylamino and aryloxy,
- $SO_2R_{10}$, wherein $R_{10}$ is selected from the group consisting of aryl and arylalkyl , and
- $R_{11}$, wherein $R_{11}$ is selected from the group consisting of aryl, arylalkyl, arylalkenyl, alkoxyalkyl, heterocyclic-substituted alkyl, $C_{3-10}$ cycloalkyl-alkyl, heterocyclic, $C_{3-10}$ cycloalkyl, dialkylaminoalkyl, aryloxyalkyl, ω-cyanoalkyl, ω-carboxylato-alkyl and carboxamidoalkyl.

**4.** A pteridine derivative according to any of claims 1 to 3, being selected from the group consisting of:

- 2-amino-4-(*N*-acetylpiperazin-1-yl)-6-(3,4-dimethoxyphenyl)pteridine;
- 2-amino-4-[(*N*-propionyl)-piperazin-1-yl]-6-(3,4-dimethoxyphenyl) pteridine;
- 2-amino-4-[(*N*-hexanoyl)-piperazin-1-yl]-6-(3,4-dimethoxyphenyl) pteridine;
- 2-amino-4-(*N*-benzoylpiperazin-1-yl)-6-(3,4-dimethoxyphenyl) pteridine;
- 2-amino-4-[*N*-(4-chlorobenzoyl)]piperazin-1-yl)-6-(3,4-dimethoxyphenyl) pteridine;
- 2-amino-4-[(*N*-2-thiophenecarbonyl)-piperazin-1-yl]-6-(3,4-dimethoxy-phenyl) pteridine;
- 2-amino-4-[(*N*-diethylcarbamoyl)-piperazin-1-yl]-6-(3,4-dimethoxy-phenyl) pteridine;
- 2-amino-4-[(*N*-hydrocinnamoyl)-piperazin-1-yl]-6-(3,4-dimethoxyphenyl) pteridine;
- 2-amino-4-[*N*-(4-cyanobenzoyl)-piperazin-1-yl]-6-(3,4-dimethoxyphenyl) pteridine;
- 2-amino-4-[(*N*-phenoxyacetyl)-piperazin-1-yl]-6-(3,4-dimethoxy-phenyl) pteridine;
- 2-amino-4-[(*N*-4-butylbenzoyl)-piperazin-1-yl]-6-(3,4-dimethoxyphenyl) pteridine;
- 2-amino-4-[(*N*-isonicotinoyl)-piperazin-1-yl]-6-(3,4-dimethoxyphenyl) pteridine;
- 2-amino-4-[(*N*-diisopropylcarbamoyl)-piperazin-1-yl]-6-(3,4-dimethoxy-phenyl) pteridine; 2-amino-4-[*N*-(4-pentoxybenzoyl)-piperazin-1-yl]-6-(3,4-dimethoxy-phenyl) pteridine;
- 2-amino-4-[*N*-(3-methoxybenzoyl)piperazin-1-yl]-6-(3,4-dimethoxy-phenyl) pteridine;
- 2-amino-4-[*N*-(2-furoyl)-piperazin-1-yl]-6-(3,4-dimethoxyphenyl) pteridine;
- 2-amino-4-[*N*-benzyloxyacetyl)-piperazin-1-yl]-6-(3,4-dimethoxyphenyl) pteridine;
- 2-amino-4-[(*N*-(p-chlorophenoxyacetyl)-piperazin-1-yl]-6-(3,4-dimethoxyphenyl) pteridine;
- 2-amino-4-[(*N*-cyclohexylcarbonyl)-piperazin-1-yl]-6-(3,4-dimethoxyphenyl) pteridine;
- 2-amino-4-[(*N*-phenylsulfonyl)-piperazin-1-yl]-6-(3,4-dimethoxyphenyl) pteridine; 2-amino-4-[(*N*-p-fluorobenzoyl)-piperazin-1-yl]-6-(3,4-dimethoxyphenyl) pteridine;
- 2-amino-4-[(*N*-2-thiophenacetyl)-piperazin-1-yl]-6-(3,4-dimethoxy-phenyl) pteridine;
- 2-amino-4-[(*N*-cinnamoyl)-piperazin-1-yl]-6-(3,4-dimethoxyphenyl) pteridine;
- 2-amino-4-[(*N*-1-pyrrolidinylcarbonyl)-piperazin-1-yl]-6-(3,4-dimethoxyphenyl) pteridine;
- 2-amino-4-[(*N*-diphenylcarbamoyl)-piperazin-1-yl]-6-(3,4-dimethoxyphenyl) pteridine;
- 2-amino-4-[*N*-(2,6-dichloro-5-fluoro-nicotinoyl)]-piperazin-1-yl]-6-(3,4-dimethoxyphenyl) pteridine;
- 2-amino-4-[(*N*-methoxyacetyl)-piperazin-1-yl]-6-(3,4-dimethoxyphenyl) pteridine;
- 2-amino-4-[*N*-(2-methoxybenzoyl)-piperazin-1-yl]-6-(3,4-dimethoxy-phenyl) pteridine;
- 2-amino-4-[(*N*-benzylsulfonyl)-piperazin-1-yl]-6-(3,4-dimethoxyphenyl) pteridine;
- 2-amino-4-[*N*-(3,4-dichlorobenzoyl)-piperazin-1-yl]-6-(3,4-dimethoxyphenyl) pteridine;
- 2-amino-4-[*N*-(4-chlorophenylacetyl)-piperazin-1-yl]-6-(3,4-dimethoxyphenyl) pteridine;
- 2-amino-4-[(*N*-(1-naphtoyl)-piperazin-1-yl]-6-(3,4-dimethoxyphenyl) pteridine;
- 2-amino-4-[*N*-(3-furoylcarbonyl)-piperazin-1-yl]-6-(3,4-dimethoxyphenyl) pteridine;
- 2-amino-4-[(*N*-benzyloxycarbonyl)-piperazin-1-yl]-6-(3,4-dimethoxyphenyl) pteridine;
- 2-amino-4-[(*N*-dimethylthiocarbamoyl)-piperazin-1-yl]-6-(3,4-dimethoxyphenyl) pteridine;
- 2-amino-4-[(*N*-phenoxycarbonyl)-piperazin-1-yl)-6-(3,4-dimethoxyphenyl) pteridine; 2-amino-4-[(*N*-phenoxythiocarbonyl)-piperazin-1-yl]-6-(3,4-dimethoxyphenyl) pteridine;
- 2-amino-4-[*N*-(2-(*S*)-amino-3-phenylpropionyl)-piperazin-1-yl]-6-(3,4-dimethoxyphenyl) pteridine;
- 2-amino-4-[*N*-[2-(*S*)-amino-3-(4-hydroxyphenyl)propionyl]-piperazin-1-yl]-6-(3,4-dimethoxyphenyl) pteridine;
- 2-amino-4-[*N*-(pyrrolidin-2-(*S*)-yl)carbonyl-piperazin-1-yl]-6-(3,4-dimethoxyphenyl) pteridine;
- 2-amino-4-[[N-2-(S)-amino-3-(indol-2-yl)propionyl]-piperazin-1-yl]-6-(3,4-dimethoxyphenyl) pteridine;
- 2-amino-4-(*N* phenoxyacetyl)-homopiperazin-1-yl*)*-6-(3,4-dimethoxyphenyl)pteridine;
- 2-amino-4-[(N-4-methyl-phenoxy-carbonyl)-piperazin-1-yl]-6-(3,4-dimethoxyphenyl) pteridine;
- 2-amino-4-[(N-4-methoxy-phenoxy-carbonyl)-piperazin-1-yl]-6-(3,4-dimethoxyphenyl)pteridine;
- 2-amino-4-[*N*-(2-methoxy)-phenoxy-carbonyl)-piperazin-1-yl]-6-(3,4-dimethoxyphenyl) pteridine;
- 2-amino-4-[*N*-(4-chloro)-phenoxy-carbonyl)-piperazin-1-yl]-6-(3,4-dimethoxyphenyl) pteridine;
- 2-amino-4-[*N*-isobutoxy-carbonyl-piperazin-1-yl]-6-(3,4-dimethoxyphenyl)pteridine;
- 2-amino-4-[*N*-(2-chloro)-phenoxy-carbonyl-piperazin-1-yl]-6-(3,4-dimethoxyphenyl) pteridine;
- 2-amino-4-[*N*-(2-methoxy)-ethoxy-carbonyl)-piperazin-1-yl]-6-(3,4-dimethoxyphenyl) pteridine;
- 2-amino-4-[*N*-(2-naphthoxy)-carbonyl)-piperazin-1-yl]-6-(3,4-dimethoxyphenyl) pteridine;
- 2-amino-4-(*N*-phenyl-carbamoyl-piperazin-1-yl)-6-(3,4-dimethoxyphenyl)pteridine;
- 2-amino-4-[*N*-4-fluorophenyl-carbamoyl-piperazin-1-yl)]-6-(3,4-dimethoxyphenyl)pteridine;
- 2-amino-4-(*N*-4-methylphenyl-carbamoyl-piperazin-1-yl)-6-(3,4-dimethoxyphenyl) pteridine;
- 2-amino-4-(N-4-cyanophenylcarbamoyf-piperazin-1-yl)-6-(3,4-dimethoxyphenyl)pteridine;
- 2-amino-4-(*N*-3-methylphenylcarbamoyl-piperazin-1-yl)-6-(3,4-dimethoxyphenyl)pteridine;
- 2-amino-4-(*N*-benzylcarbamoyl-piperazin-1-yl)-6-(3,4-dimethoxyphenyl)pteridine;
- 2-amino-4-(N-4-fluorobenzylcarbamoyl-piperazin-1-yl)-6-(3,4-dimethoxyphenyl)pteridine;
- 2-amino-4-(N-3-chloro-4-fluorophenylcarbamoyl-piperazin-1-yl)-6-(3,4-dimethoxyphenyl) pteridine;
- 2-amino-4-(*N*-3-thienylcarbamoyl-piperazin-1-yl)-6-(3,4-dimethoxyphenyl)pteridine;
- 2-amino-4-(*N*-2-(2-thienyl)ethylcarbamoyl-piperazin-1-yl]-6-(3,4-dimethoxyphenyl) pteridine;
- 2-amino-4-[(*N*-butyl-carbamoyl-piperazin-1-yl)]-6-(3,4-dimethoxyphenyl)pteridine;
- 2-amino-4-[*N*-aminoacetyl]-piperazin-1-yl]-6-(3,4-dimethoxyphenyl) pteridine;
- 2-amino-4-[*N*-[2-(S),4-diamino-4-oxobutanoyl]-piperazin-1-yl]-6-(3,4-dimethoxyphenyl) pteridine;
- 2-amino-4-[*N*-[4-(methoxycarbonyl)benzoyl)-piperazin-1-yl]-6-(3,4-dimethoxyphenyl) pteridine;
- 2-amino-4-[*N*-[4-(dimethylamino)acetyl]-piperazin-1-yl]-6-(3,4-dimethoxyphenyl) pteridine;
- 2-amino-4-[*N*-(4-amino-4-oxo-butanoyl)-piperazin-1-yl]-6-(3,4-dimethoxyphenyl) pteridine;
- 2-amino-4-[*N*-(3-carboxypropanoyl)-piperazin-1-yl]-6-(3,4-dimethoxyphenyl) pteridine;
- 2-amino-4-[*N*-[4-(carboxy)benzoyl]-piperazin-1-yl]-6-(3,4-dimethoxyphenyl) pteridine;
- 2-amino-4-(*N*-methyl-*N*-phenyl-carbamoyl-piperazin-1-yl)-6-(3,4-dimethoxyphenyl) pteridine;
- 2-amino-4-(*N*-ethyl-*N*-phenyl-carbamoyl-piperazin-1-yl)-6-(3,4-dimethoxyphenyl) pteridine;
- 2-amino-4-(*N*-methyl-*N*-tolyl-carbamoyl-piperazin-1-yl)-6-(3,4-dimethoxyphenyl) pteridine;
- 2-amino-4-(1-(2-methoxyethyl)piperazino)-6-(3,4-dimethoxyphenyl) pteridine;
- 2-amino-4-(1-cyclohexylmethyl)piperazino)-6-(3,4-dimethoxyphenyl) pteridine;
- 2-amino-4-(1-cyclopentylpiperazino)-6-(3,4-dimethoxyphenyl) pteridine;
- 2-amino-4-(1-butylpiperazino)-6-(3,4-dimethoxyphenyl) pteridine;
- 2-amino-4-(1-isopropylpiperazino)-6-(3,4-dimethoxyphenyl) pteridine;
- 2-amino-4-(1-(2-diethylaminoethyl)-piperazino)-6-(3,4-dimethoxyphenyl) pteridine;
- 2-amino-4-(1-(2-diisopropylaminoethyl)-piperazino)-6-(3,4-dimethoxyphenyl) pteridine;

- 2-amino-4-(1-(2-morpholino-4-yl-ethyl)-piperazino)-6-(3,4-dimethoxyphenyl) pteridine;
- 2-amino-4-(4-[2-(piperazin-1-yl)-acetyl]-morpholino)-6-(3,4-dimethoxy-phenyl) pteridine;
- 2-amino-4-(4-[2-(piperazin-1-yl)-acetyl]-pyrrolidino)-6-(3,4-dimethoxy-phenyl) pteridine;
- 2-amino-4-(2-[piperazin-1-yl]-acetic acid N-methyl N-phenyl amide)-6-(3,4-dimethoxyphenyl) pteridine;
- 2-amino-4-(2-(piperazin-1-yl)-propionic acid ethyl ester)-6-(3,4-dimethoxyphenyl) pteridine;
- 2-amino-4-(3-(piperazin-1-yl)-propionic acid ethyl ester)-6-(3,4-dimethoxyphenyl) pteridine;
- 2-amino-4-(2-(piperazin-1-yl)-acetic acid ethyl ester)-6-(3,4-dimethoxyphenyl) pteridine;
- 2-amino-4-(1-(3-methyl-benzyl)piperazinyl)-6-(3,4-dimethoxyphenyl) pteridine;
- 2-amino-4-[(2,6-dichlorobenzyl)piperazin-1-yl]-6-(3,4-dimethoxyphenyl) pteridine;
- 2-amino-4-((4-fluorobenzyl)piperazin-1-yl)-6-(3,4-dimethoxyphenyl) pteridine;
- 2-amino-4-((4-chlorobenzyl)piperazin-1-yl)-6-(3,4-dimethoxyphenyl) pteridine;
- 2-amino-4-((4-methylbenzyl)piperazin-1-yl)-6-(3,4-dimethoxyphenyl) pteridine;
- 2-amino-4-((2-fluorobenzyl)piperazin-1-yl)-6-(3,4-dimethoxyphenyl) pteridine;
- 2-amino-4-((3,4-dichlorobenzyl)piperazin-1-yl)-6-(3,4-dimethoxyphenyl) pteridine;
- 2-amino-4-(piperonyl-piperazin-1-yl)-6-(3,4-dimethoxyphenyl) pteridine;
- 2-amino-4-((4-*tert*-butylbenzyl)piperazin-1-yl)-6-(3,4-dimethoxyphenyl) pteridine;
- 2-amino-4-((4-pyridyl)-piperazin-1-yl)-6-(3,4-dimethoxyphenyl) pteridine;
- 2-amino-4-((2-pyridyl)-piperazin-1-yl)-6-(3,4-dimethoxyphenyl) pteridine;
- 2-amino-4-((2-pyrimidinyl)-piperazin-1-yl)-6-(3,4-dimethoxyphenyl) pteridine;
- 2-amino-4-((3-methoxyphenyl)-piperazin-1-yl)-6-(3,4-dimethoxyphenyl) pteridine;
- 2-amino-4-(1-(3-phenylpropyl-piperazine)-6-(3,4-dimethoxyphenyl) pteridine;
- 2-amino-4-((3,4-dichlorophenyl)-piperazin-1-yl)-6-(3,4-dimethoxyphenyl) pteridine;
- 2-amino-4-((3-dichlorophenyl)-piperazin-1-yl)-6-(3,4-dimethoxyphenyl) pteridine;
- 2-amino-4-((1-phenylethyl)-piperazin-1-yl)-6-(3,4-dimethoxyphenyl) pteridine;
- 2-amino-4-((2-(1-pyrrolyl)-ethyl-piperazin-1-yl)-6-(3,4-dimethoxyphenyl) pteridine;
- 2-amino-4-((2-phenoxyethyl-piperazin-1-yl)-6-(3,4-dimethoxyphenyl) pteridine;
- 2-amino-4-(1-(2-imidazol-1-yl-ethyl-piperazine)-6-(3,4-dimethoxyphenyl) pteridine;
- 2-amino-4-((3-pyridyl)-methyl)-piperazin-1-yl)-6-(3,4-dimethoxyphenyl) pteridine;
- 2-amino-4-((4-pyridyl)-methyl)-piperazin-1-yl))-6-(3,4-dimethoxyphenyl) pteridine;
- 2-amino-4-((1-naphtylmethyl)-piperazin-1-yl-6-(3,4-dimethoxyphenyl) pteridine;
- 2-amino-4-(*N*-phenethylpiperazin-1-yl)-6-(3,4-dimethoxyphenyl) pteridine;
- 2-amino-4-((2-methoxyphenyl)-piperazin-1-yl)-6-(3,4-dimethoxyphenyl) pteridine;
- 2-amino-4-((4-methoxyphenyl)-piperazin-1-yl)-6-(3,4-dimethoxyphenyl) pteridine;
- 2-amino-4-((4-chlorophenyl)-piperazin-1-yl)-6-(3,4-dimethoxyphenyl) pteridine;
- 2-amino-6-(3,4-dimethoxyphenyl)-4-(4-(3-propionitril)-piperazin-1-yl) pteridine;
- 2-amino-6-(3,4-dimethoxyphenyl)-4-(4-(2-(1,3)-dioxolan-2-yl-ethyl)-piperazin-1-yl) pteridine;
- 2-amino-6-(3,4-dimethoxyphenyl)-4-(4-(2-ethoxyethyl)-piperazin-1-yl) pteridine;
- 2-amino-6-(3,4-dimethoxyphenyl)-4-(pent-3-yl-piperazin-1-yl) pteridine;
- 2-amino-6-(3,4-dimethoxyphenyl)-4-(1-pentyl-piperazin-1-yl) pteridine;
- 2-amino-6-(3,4-dimethoxyphenyl)-4-(1-isobutyl-piperazin-1-yl) pteridine;
- 2-amino-6-(3,4-dimethoxyphenyl)-4-((tetrahydrofurfuryl)-piperazin-1-yl) pteridine;
- 2-amino-6-(3,4-dimethoxyphenyl)-4-(1,3-dioxolan-2-yl-methylpiperazin-1-yl) pteridine;
- 2-amino-4-((3,5-dichlorophenyl)-piperazin-1-yl)-6-(3,4-dimethoxyphenyl) pteridine;
- 2-amino-6-(3,4-dimethoxyphenyl)-4-((4-fluorophenyl)-piperazin-1-yl) pteridine;
- 2-amino-6-(3,4-dimethoxyphenyl)-4-((3-trifluoromethylphenyl)-piperazin-1-yl) pteridine;
- 2-amino-6-(3,4-dimethoxyphenyl)-4-((3,4-dimethylphenyl)-piperazin-1-yl) pteridine;
- 2-amino-6-(3,4-dimethoxyphenyl)-4-((3-methylphenyl)-piperazin-1-yl) pteridine;
- 2-amino-6-(3,4-dimethoxyphenyl)-4-((4-methylphenyl)-piperazin-1-yl) pteridine;
- 2-amino-6-(3,4-dimethoxyphenyl)-4-((2-pyridyl)methyl-piperazin-1-yl) pteridine;
- 2-amino-6-(3,4-dimethoxyphenyl)-4-(thiazol-2-yl)-piperazin-1-yl) pteridine;
- 2-amino-6-(3,4-dimethoxyphenyl)-4-(1-(1-methyl-piperidin-3-yl-methyl)-piperazin-1-yl) pteridine;
- 2-amino-6-(3,4-dimethoxyphenyl)-4-((4-trifluoromethylphenyl)-piperazin-1-yl) pteridine;
- 2-amino-6-(3,4-dimethoxyphenyl)-4-(4-(2-dimethylaminoethyl)-piperazin-1-yl)-pteridine trihydrochloride salt;
- 2-amino-6-(3,4-dimethoxyphenyl)-4-(4-(3-dimethylaminopropyl)-piperazin-1-yl) pteridine trihydrochloride salt;
- 2-amino-6-(3,4-dimethoxyphenyl)-4-(4-(2-dipropylaminoethyl)-piperazin-1-yl) pteridine trihydrochloride salt;
- 2-amino-6-(3,4-dimethoxyphenyl)-4-(4-(2-piperidin-1-yl-ethyl)-piperazin-1-yl) pteridine trihydrochloride salt;
- 2-amino-4-[2-trifluoromethyl-4-nitro-phenyl-piperazin-1-yl)-6-(3,4-dimethoxyphenyl) pteridine; and
- 2-amino-4-[2-(piperazin-1-yl)-acetic acid N-(2-thiazolyl)-amide]-6-(3,4-dimethoxyphenyl) pteridine.

5. A pharmaceutical composition comprising as an active principle at least one pteridine derivative according to any of claims 1 to 4.

6. A pharmaceutical composition according to claim 5, further comprising one or more biologically-active drugs selected from the group consisting of immunosuppressant and/or immunomodulator drugs, antineoplastic drugs, and antiviral agents.

7. A pharmaceutical composition according to claim 5, further comprising one or more immunosuppressant drugs selected from the group consisting of cyclosporin A; substituted xanthines; pentoxyfylline; daltroban, sirolimus, tacrolimus; rapamycin; leflunomide; mycophenolic acid and salts thereof; adrenocortical steroids; azathioprine, brequinar; gusperimus; 6-mercaptopurine; mizoribine; chloroquine; hydroxychloroquine; monoclonal antibodies with immunosuppressive properties; etanercept; infliximab; and kineret.

8. A pharmaceutical composition according to claim 5, further comprising one or more immunomodulator drugs selected from the group consisting of acemannan, amiprilose, bucillamine, ditiocarb sodium, imiquimod, inosine pranobex, interferon-β, interferon-γ, lentinan, levamisole, pidotimod, romurtide, platonin, procodazole, propagermanium, thymomodulin, thymopentin and ubenimex.

9. A pharmaceutical composition according to claim 5, further comprising one or more antineoplastic drugs selected from the group consisting of alkaloids, alkylating agents, alkyl sulfonates, aziridines, ethylenimines, methylmelamines, nitrogen mustards, nitrosoureas, antibiotics, antimetabolites, folic acid analogs, purine analogs, pyrimidine analogs, enzymes, interferon and platinum complexes.

10. A pharmaceutical composition according to claim 5, further comprising one or more antiviral agents selected from the group consisting of retroviral enzyme inhibitors, HIV-1 IN inhibitors, nucleoside reverse transcriptase inhibitors, zidovudine, lamivudine, didanosine, stavudine, zalcitabine, non-nucleoside reverse transcriptase inhibitors, nevirapine, delavirdine, foscarnet sodium, HIV-1 protease inhibitors, saquinavir, ritonavir, indinavir, nelfinavir, acyclovir, cidofovir, cytarabine, edoxudine, famciclovir, floxuridine, ganciclovir, idoxuridine, penciclovir, sorivudine, trifluridine, valaciclovir, vidarabine, kethoxal, methisazone, moroxydine, podophyllotoxin, ribavirine, rimantadine, stallimycine, statolon, tromantadine and xenazoic acid.

**Patentansprüche**

1. Pteridinderivat mit der allgemeinen Formel (I):

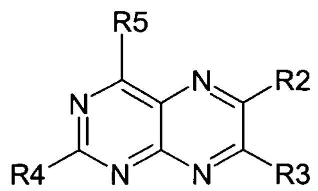

(I)

worin:

- $R_5$ eine Gruppe ist, dargestellt durch die allgemeine Formel (II):

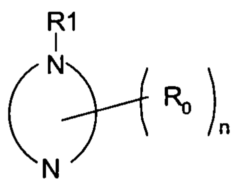

(II)

worin:

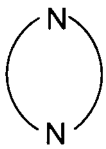

(III)

eine Piperazin-1-yl-Gruppe oder eine Homopiperazin-1-yl-Gruppe darstellt, und worin:

- jeder Substiuent $R_0$ des heterocyclischen Rings (III) eine Gruppe ist, die unabhängig voneinander ausgewählt ist aus Methyl und Phenyl;
- n 0, 1 oder 2 ist;
- $R_1$ eine Substitutionsgruppe ist, ausgewählt aus der Gruppe, bestehend aus Formyl, Acyl, Thio-acyl, Amid, Thioamid, Sulfonyl, Sulfinyl, Carboxylat, Thiocarboxylat, Amino-subsituiertem Acyl, Alkoxyalkyl, $C_{3-10}$-Cycloalkyl-alkyl, $C_{3-10}$-Cycloalkyl, Dialkylaminoalkyl, heterocyclisch-substituiertem Alkyl, Acylsubstituiertem Alkyl, Thioacyl-substituiertem Alkyl, Amido-substituiertem Alkyl, Thioamido-substituiertem Alkyl, Carboxylato-substituiertem Alkyl, Thiocarboxylato-substituiertem Alkyl, (Amino-substituiertem Acylalkyl, Heterocyclen, Carbonsäureester, ω-Cyanoalkyl, ω-Carbonsäureesteralkyl, Halogen-$C_{1-7}$-Alkyl, $C_{2-7}$-Alkenyl, $C_{2-7}$-Alkinyl, Arylalkenyl, Aryloxyalkyl, Arylalkyl und Aryl, wobei der Arylrest eines jeden Arylalkenyl-, Aryloxialkyl-, Arylalkyl- und Arylradikals wahlweise mit einem oder mehreren Substituenten substituiert ist, unabhängig voneinander ausgewählt aus der Gruppe, bestehend Halogen, $C_{1-7}$Alkyl, $C_{2-7}$-Alkenyl, $C_{2-7}$-Alkinyl, Halogen $C_{1-7}$-Alkyl, Nitro, Hydroxyl, Sulfhydryl, Amino, $C_{1-7}$-Alkoxy, $C_{3-10}$-Cycloalkoxy, Aryloxy, Arylalkyloxy, Oxyheterocyclen, heterocyclisch-substituiertem Alkyloxy, Thio-$C_{1-7}$-Alkyl, Thio-$C_{3-10}$-Cycloalkyl, Thioaryl, Thio- Heterocyclen, Arylalkylthio, heterocyclisch- substituiertem Alkylthio, Formyl, Sulfonamido, Hydroxylamino, Alkoxyamino, Mercaptoamino, Thioalkylamino, Acylamino, Thioacylamino, Cyano, Carbonsäure oder Estern, Alkylamino, Cycloalkylamino, Alkenylamino, Cycloalkenylamino, Alkinylamino, Arylamino, Aryl- alkylamino, Hydroxyalkylamino, Mercaptoalkylamino und heterocyclischem Amino;
- $R_3$ Wasserstoff ist;
- $R_4$ Amino ist;
- $R_2$ eine Phenylgruppe ist, die wahlweise mit einem oder mehreren Substituenten substituiert ist, ausgewählt aus der Gruppe, bestehend aus Halogen, $C_{1-7}$-Alkyl und $C_{1-7}$-Alkoxy;

und/oder ein pharmazeutisch annehmbares Additionssalz dessen und/oder ein Stereoisomer dessen und/oder ein Mono- oder ein Di-N-oxid dessen und/oder ein Solvat dessen und/oder ein Dihydro- oder Tetrahydropteridinderivat dessen.

**2.** Pteridinderviat gemäß Anspruch 1, wobei $R_5$ eine Gruppe ist, ausgewählt aus der Gruppe, bestehend aus Piperazin-1-yl, 2-Methylpiperazin-1-yl, 2-Phenylpiperazin-1-yl, Homopiperazin-1-yl und 2,5-Dimethylpiperazin-1-yl, wobei die Gruppe an ihrer 4-Position mit einem Subtituenten $R_1$ substituiert ist, der eine Carbonyl(oxo)- oder Thiocarbonyl (thioxo)- oder Sulfonylfunktion besitzt.

**3.** Pteridinderivat gemäß Anspruch 1 oder 2, wobei $R_5$ eine Piperazin-1-yl-Gruppe ist, die an ihrer 4-Position mit einem Substituenten $R_1$ substituiert ist, wobei $R_1$ ausgewählt ist aus der Gruppe, bestehend aus:

- $COR_8$, wobei $R_8$ ausgewählt ist aus der Gruppe, bestehend aus Wasserstoff; $C_{1-7}$-Alkyl ; $C_{3-10}$-Cycloalkyl ; Aryl, wahlweise substituiert mit einem oder mehreren Substituenten, ausgewählt aus der Gruppe, bestehend aus Halogen, $C_{1-7}$-Alkyl, Cyano und $C_{1-7}$-Alkoxy ; Heterocyclen, wahlweise substituiert mit einem oder mehreren Halogenatomen; Arylalkyl; Aryloxyalkyl; Arylalkoxyalkyl; Alkoxyalkyl; Arylalkoxy; Aryloxy; Arylalkenyl; heterocyclisch-substituiertem Alkyl; Alkylamino und Arylamino,
- $CSR_9$, wobei $R_9$ ausgewählt ist aus der Gruppe, bestehend aus Alkylamino und Aryloxy,
- $SO_2R_{10}$, wobei $R_{10}$ ausgewählt ist aus der Gruppe, bestehend aus Aryl und Arylalkyl, und
- $R_{11}$, wobei $R_{11}$ ausgewählt ist aus der Gruppe, bestehend aus Aryl, Arylalkyl, Arylalkenyl, Alkoxyalkyl, heterocyclisch-substituiertem Alkyl, $C_{3-10}$-Cycloalkyl-alkyl, Heterocyclen, $C_{3-10}$-Cycloalkyl, Dialkylaminoalkyl, Aryloxyalkyl, ω-Cyanoalkyl, ω-Carboxylato-Alkyl und Carboxamidoalkyl.

**4.** Pteridinderivat gemäß einem der Ansprüche 1 bis 3, ausgewählt aus der Gruppe, bestehend aus:

- 2-Amino-4-(N acetylpiperazin-1-yl)-6-(3,4-dimethoxyphenyl)pteridin;

- 2-Amino-4-[(N-propionyl)-piperazin-1-yl]-6-(3,4-dimethoxyphenyl)pteridin;
- 2-Amino-4-[(*N*-hexanoyl)-piperazin-1-yl]-6-(3,4-dimethoxyphenyl)pteridin;
- 2-Amino-4-(N-benzoylpiperazin-1-yl)-6-(3,4-dimethoxyphenyl)pteridin;
- 2-Amino-4-[*N*-(4-chlorbenzoyl)]piperazin-1-yl)-6-(3,4-dimethoxyphenyl)pteridin;
- 2-Amino-4-[(*N*-2-thiophencarbonyl)-piperazin-1-yl]-6-(3,4-dimethoxyphenyl)pteridin;
- 2-Amino-4-[(*N*-diethylcarbamoyl)-piperazin-1-yl]-6-(3,4-dimethoxyphenyl)pteridin;
- 2-Amino-4-[(*N*-hydrocinnamoyl)-piperazin-1-yl]-6-(3,4-dimethoxyphenyl)pteridin;
- 2-Amino-4-[*N*-(4-cyanobenzoyl)-piperazin-1-yl]-6-(3,4-dimethoxyphenyl)pteridin;
- 2-Amino-4-[(*N*-phenoxyacetyl)-piperazin-1-yl]-6-(3,4-dimethoxyphenyl)pteridin;
- 2-Amino-4-[(N-4-butylbenzoyl)-piperazin-1-yl]-6-(3,4-dimethoxyphenyl)pteridin;
- 2-Amino-4-[(N-isonicotinoyl)-piperazin-1-yl]-6-(3,4-dimethoxyphenyl)pteridin;
- 2-Amino-4-[(*N*-diisopropylcarbamoyl)-piperazin-1-yl]-6-(3,4-dimethoxyphenyl)pteridin;
- 2-Amino-4-[*N*-(4-pentoxybenzoyl)-piperazin-1-yl]-6-(3,4-dimethoxyphenyl)pteridin;
- 2-Amino-4-[*N*-(3-methoxybenzoyl)-piperazin-1-yl]-6-(3,4-dimethoxyphenyl)pteridin;
- 2-Amino-4-[*N*-(2-furoyl)-piperazin-1-yl]-6-(3,4-dimethoxyphenyl)pteridin;
- 2-Amino-4-[(*N*-benzyloxyacetyl)-piperazin-1-yl]-6-(3,4-dimethoxyphenyl)pteridin;
- 2-Amino-4-[(*N*-(p-chlorphenoxyacetyl)-piperazin-1-yl]-6-(3,4-dimethoxyphenyl)pteridin;
- 2-Amino-4-[(*N*-cyclohexylcarbonyl)-piperazin-1-yl]-6-(3,4-dimethoxyphenyl)pteridin;
- 2-Amino-4-[(*N*-phenylsulfonyl)-piperazin-1-yl]-6-(3,4-dimethoxyphenyl)pteridin;
- 2-Amino-4-[(N-p-fluorbenzoyl)-piperazin-1-yl]-6-(3,4-dimethoxyphenyl)pteridin;
- 2-Amino-4-[(N-2-thiophenacetyl)-piperazin-1-yl]-6-(3,4-dimethoxyphenyl)pteridin;
- 2-Amino-4-[(N-cinnamoyl)-piperazin-1-yl]-6-(3,4-dimethoxyphenyl)pteridin;
- 2-Amino-4-[(*N*-1-pyrrolidinylcarbonyl)-piperazin-1-yl]-6-(3,4-dimethoxyphenyl)pteridin;
- 2-Amino-4-[(N-diphenylcarbamoyl)-piperazin-1-yl]-6-(3,4-dimethoxyphenyl)pteridin;
- 2-Amino-4-[*N*-(2,6-dichlor-5-fluor-nicotinoyl)]-piperazin-1-yl]-6-(3,4-dimethoxyphenyl)pteridin;
- 2-Amino-4-[(*N*-methoxyacetyl)-piperazin-1-yl]-6-(3,4-dimethoxyphenyl)pteridin;
- 2-Amino-4-[*N*-(2-methoxybenzoyl)-piperazin-1-yl]-6-(3,4-dimethoxyphenyl)pteridin;
- 2-Amino-4-[(N-benzylsulfonyl)-piperazin-1-yl]-6-(3,4-dimethoxyphenyl)pteridin;
- 2-Amino-4-[*N*-(3,4-dichlorobenzoyl)-piperazin-1-yl]-6-(3,4-dimethoxyphenyl)pteridin;
- 2-Amino-4-[*N*-(4-chlorophenylacetyl)-piperazin-1-yl]-6-(3,4-dimethoxyphenyl)pteridin;
- 2-Amino-4-[(*N*-(1-naphtoyl)-piperazin-1-yl]-6-(3,4-dimethoxyphenyl)pteridin;
- 2-Amino-4-[*N*-(3-furoylcarbonyl)-piperazin-1-yl]-6-(3,4-dimethoxyphenyl)pteridin;
- 2-Amino-4-[(*N*-benzyloxycarbonyl)-piperazin-1-yl]-6-(3,4-dimethoxyphenyl)pteridin;
- 2-Amino-4-[(*N*-dimethylthiocarbamoyl)-piperazin-1-yl]-6-(3,4-dimethoxyphenyl)pteridin;
- 2-Amino-4-[(*N*-phenoxycarbonyl)-piperazin-1-yl]-6-(3,4-dimethoxyphenyl)pteridin;
- 2-Amino-4-[(*N*-phenoxythiocarbonyl)-piperazin-1-yl]-6-(3,4-dimethoxyphenyl)pteridin;
- 2-Amino-4-[N-(2-(S)-amino-3-phenylpropionyl)-piperazin-1-yl]-6-(3,4-dimethoxyphenyl)pteridin;
- 2-Amino-4-[*N*-[2-(S)-amino-3-(4-hydroxyphenyl)propionyl]-piperazin-1-yl]-6-(3,4-dimethoxyphenyl)pteridin;
- 2-Amino-4-[*N*-(pyrrolidin-2-(*S*)-yl)carbonyl-piperazin-1-yl]-6-(3,4-dimethoxyphenyl)pteridin;
- 2-Amino-4-[[*N*-2-(*S*)-amino-3-(indol-2-yl)propionyl]-piperazin-1-yl]-6-(3,4-dimethoxyphenyl)pteridin;
- 2-Amino-4-(*N*-phenoxyacetyl)-homopiperazin-1-yl)-6-(3,4-dimethoxyphenyl)pteridin;
- 2-Amino-4-[(*N*-4-methyl-phenoxy-carbonyl)-piperazin-1-yl]-6-(3,4-dimethoxyphenyl)pteridin;
- 2-Amino-4-[(*N*-4-methoxy-phenoxy-carbonyl)-piperazin-1-yl]-6-(3,4-dimethoxy-phenyl)pteridin;
- 2-Amino-4-[*N*-(2-methoxy)-phenoxy-carbonyl)-piperazin-1-yl]-6-(3,4-dimethoxyphenyl)pteridin;
- 2-Amino-4-[*N*-(4-chlor)-phenoxy-carbonyl)-piperazin-1-yl]-6-(3,4-dimethoxyphenyl)pteridin;
- 2-Amino-4-[*N*-isobutoxy-carbonyl-piperazin-1-yl]-6-(3,4-dimethoxyphenyl)pteridin;
- 2-Amino-4-[*N*-(2-chlor)-phenoxy-carbonyl-piperazin-1-yl]-6-(3,4-dimethoxyphenyl)pteridin;
- 2-Amino-4-[*N*-(2-methoxy)-ethoxy-carbonyl)-piperazin-1-yl] -6-(3,4-dimethoxyphenyl)pteridin;
- 2-Amino-4-[*N*-(2-naphthoxy)-carbonyl)-piperazin-1-yl]-6-(3,4-dimethoxyphenyl)pteridin;
- 2-Amino-4-(*N*-phenyl-carbamoyl-piperazin-1-yl)-6-(3,4-dimethoxyphenyl)pteridin;
- 2-Amino-4-[*N*-4-fluorophenyl-carbamoyl-piperazin-1-yl)]-6-(3,4-dimethoxyphenyl)pteridin;
- 2-Amino-4-(*N*-4-methylphenyl-carbamoyl-piperazin-1-yl)-6-(3,4-dimethoxyphenyl)pteridin;
- 2-Amino-4-(*N*-4-cyanophenylcarbamoyl-piperazin-1-yl)-6-(3,4-dimethoxyphenyl)pteridin;
- 2-Amino-4-(*N*-3-methylphenylcarbamoyl-piperazin-1-yl)-6-(3,4-dimethoxyphenyl)pteridin;
- 2-Amino-4-(*N*-benzylcarbamoyl-piperazin-1-yl)-6-(3,4-dimethoxyphenyl)pteridin;
- 2-Amino-4-(*N*-4-fluorbenzylcarbamoyl-piperazin-1-yl)-6-(3,4-dimethoxyphenyl)pteridin;
- 2-Amino-4-(*N*-3-chlor-4-fluorphenylcarbamoyl-piperazin-1-yl)-6-(3,4-dimethoxyphenyl)pteridin;
- 2-Amino-4-(*N*-3-thienylcarbamoyl-piperazin-1-yl)-6-(3,4-dimethoxyphenyl)pteridin;

- 2-Amino-4-[*N*-2-(2-thienyl)ethylcarbamoyl-piperazin-1-yl]-6-(3,4-dimethoxyphenyl)pteridin;
- 2-Amino-4-[(*N*-butyl-carbamoyl-piperazin-1-yl)]-6-(3,4-dimethoxyphenyl)pteridin;
- 2-Amino-4-[*N*-aminoacetyl]-piperazin-1-yl]-6-(3,4-dimethoxyphenyl)pteridin;
- 2-Amino-4-[*N*-[2-(*S*),4-diamino-4-oxobutanoyl]-piperazin-1-yl]-6-(3,4-dimethoxyphenyl)pteridin;
- 2-Amino-4-[*N*-[4-(methoxycarbonyl)benzoyl]-piperazin-1-yl]-6-(3,4-dimethoxyphenyl)pteridin;
- 2-Amino-4-[*N*-[4-(dimethylamino)acetyl]-piperazin-1-yl]-6-(3,4-dimethoxyphenyl)pteridin;
- 2-Amino-4-[*N*-(4-amino-4-oxo-butanoyl)-piperazin-1-yl]-6-(3,4-dimethoxyphenyl)pteridin;
- 2-Amino-4-[*N*-(3-carboxypropanoyl)-piperazin-1-yl]-6-(3,4-dimethoxyphenyl)pteridin;
- 2-Amino-4-[*N*-[4-(carboxy)benzoyl]-piperazin-1-yl]-6-(3,4-dimethoxyphenyl)pteridin;
- 2-Amino-4-(*N*-methyl-*N*-phenyl-carbamoyl-piperazin-1-yl)-6-(3,4-dimethoxyphenyl)pteridin;
- 2-Amino-4-(N-ethyl-N-phenyl-carbamoyl-piperazin-1-yl)-6-(3,4-dimethoxyphenyl)pteridin;
- 2-Amino-4-(N-methyl-N-tolyl-carbamoyl-piperazin-1-yl)-6-(3,4-dimethoxyphenyl)pteridin;
- 2-Amino-4-(1-(2-methoxyethyl)piperazino)-6-(3,4-dimethoxyphenyl)pteridin;
- 2-Amino-4-(1-cyclohexylmethyl)piperazino)-6-(3,4-dimethoxyphenyl)pteridin;
- 2-Amino-4-(1-cyclopentylpiperazino)-6-(3,4-dimethoxyphenyl)pteridin;
- 2-Amino-4-(1-butylpiperazino)-6-(3,4-dimethoxyphenyl)pteridin;
- 2-Amino-4-(1-isopropylpiperazino)-6-(3,4-dimethoxyphenyl)pteridin;
- 2-Amino-4-(1-(2-diethylaminoethyl)-piperazino)-6-(3,4-dimethoxyphenyl)pteridin;
- 2-Amino-4-(1-(2-diisopropylaminoethyl)-piperazino)-6-(3,4-dimethoxyphenyl)pteridin;
- 2-Amino-4-(1-(2-morpholino-4-yl-ethyl)-piperazino)-6-(3,4-dimethoxyphenyl)pteridin;
- 2-Amino-4-(4-[2-(piperazin-1-yl)-acetyl]-morpholino)-6-(3,4-dimethoxyphenyl)pteridin;
- 2-Amino-4-(4-[2-(piperazin-1-yl)-acetyl]-pyrrolidino)-6-(3,4-dimethoxyphenyl)pteridin;
- 2-Amino-4-(2-[piperazin-1-yl]-essigsäure-N-methyl-N-phenyl-amid)-6-(3,4-dimethoxyphenyl)pteridin;
- 2-Amino-4-(2-(piperazin-1-yl)-propionsäureethylester)-6-(3,4-dimethoxyphenyl)pteridin;
- 2-Amino-4-(3-(piperazin-1-yl)-propionsäureethylester)-6-(3,4-dimethoxyphenyl)pteridin;
- 2-Amino-4-(2-(piperazin-1-yl)-essigsäureethylester)-6-(3,4-dimethoxyphenyl)pteridin;
- 2-Amino-4-(1-(3-methyl-benzyl)piperazinyl)-6-(3,4-dimethoxyphenyl)pteridin;
- 2-Amino-4-[(2,6-dichlorbenzyl)piperazin-1-yl]-6-(3,4-dimethoxyphenyl)pteridin;
- 2-Amino-4-((4-fluorbenzyl)piperazin-1-yl)-6-(3,4-dimethoxyphenyl)pteridin;
- 2-Amino-4-((4-chlorbenzyl)piperazin-1-yl)-6-(3,4-dimethoxyphenyl)pteridin;
- 2-Amino-4-((4-methylbenzyl)piperazin-1-yl)-6-(3,4-dimethoxyphenyl)pteridin;
- 2-Amino-4-((2-fluorbenzyl)piperazin-1-yl)-6-(3,4-dimethoxyphenyl)pteridin;
- 2-Amino-4-((3,4-dichlorbenzyl)piperazin-1-yl)-6-(3,4-dimethoxyphenyl)pteridin;
- 2-Amino-4-(piperonyl-piperazin-1-yl)-6-(3,4-dimethoxyphenyl)pteridin;
- 2-Amino-4-((4-*tert*-butylbenzyl)piperazin-1-yl)-6-(3,4-dimethoxyphenyl)pteridin;
- 2-Amino-4-((4-pyridyl)-piperazin-1-yl)-6-(3,4-dimethoxyphenyl)pteridin;
- 2-Amino-4-((2-pyridyl)-piperazin-1-yl)-6-(3,4-dimethoxyphenyl)pteridin;
- 2-Amino-4-((2-pyrimidinyl)-piperazin-1-yl)-6-(3,4-dimethoxyphenyl)pteridin;
- 2-Amino-4-((3-methoxyphenyl)-piperazin-1-yl)-6-(3,4-dimethoxyphenyl)pteridin;
- 2-Amino-4-(1-(3-phenylpropyl-piperazine)-6-(3,4-dimethoxyphenyl)pteridin;
- 2-Amino-4-((3,4-dichlorphenyl)-piperazin-1-yl)-6-(3,4-dimethoxyphenyl)pteridin;
- 2-Amino-4-((3-dichlorphenyl)-piperazin-1-yl)-6-(3,4-dimethoxyphenyl)pteridin;
- 2-Amino-4-((1-phenylethyl)-piperazin-1-yl)-6-(3,4-dimethoxyphenyl)pteridin;
- 2-Amino-4-((2-(1-pyrrolyl)-ethyl-piperazin-1-yl)-6-(3,4-dimethoxyphenyl)pteridin;
- 2-Amino-4-((2-phenoxyethyl-piperazin-1-yl)-6-(3,4-dimethoxyphenyl)pteridin;
- 2-Amino-4-(1-(2-imidazol-1-yl-ethyl-piperazin)-6-(3,4-dimethoxyphenyl)pteridin;
- 2-Amino-4-((3-pyridyl)-methyl)-piperazin-1-yl)-6-(3,4-dimethoxyphenyl)pteridin;
- 2-Amino-4-((4-pyridyl)-methyl)-piperazin-1-yl)-6-(3,4-dimethoxyphenyl)pteridin;
- 2-Amino-4-((1-naphthylmethyl)-piperazin-1-yl)-6-(3,4-dimethoxyphenyl)pteridin;
- 2-Amino-4-(*N*-phenethylpiperazin-1-yl)-6-(3,4-dimethoxyphenyl)pteridin;
- 2-Amino-4-((2-methoxyphenyl)-piperazin-1-yl)-6-(3,4-dimethoxyphenyl)pteridin;
- 2-Amino-4-((4-methoxyphenyl)-piperazin-1-yl)-6-(3,4-dimethoxyphenyl)pteridin;
- 2-Amino-4-((4-chlorphenyl)-piperazin-1-yl)-6-(3,4-dimethoxyphenyl)pteridin;
- 2-Amino-6-(3,4-dimethoxyphenyl)-4-(4-(3-propionitril)-piperazin-1-yl)pteridin;
- 2-Amino-6-(3,4-dimethoxyphenyl)-4-(4-(2-(1,3)-dioxolan-2-yl-ethyl)-piperazin-1-yl)pteridin;
- 2-Amino-6-(3,4-dimethoxyphenyl)-4-(4-(2-ethoxyethyl)-piperazin-1-yl)pteridin;
- 2-Amino-6-(3,4-dimethoxyphenyl)-4-(pent-3-yl-piperazin-1-yl)pteridin;
- 2-Amino-6-(3,4-dimethoxyphenyl)-4-(1-pentyl-piperazin-1-yl)pteridin;

- 2-Amino-6-(3,4-dimethoxyphenyl)-4-(1-isobutyl-piperazin-1-yl)pteridin;
- 2-Amino-6-(3,4-dimethoxyphenyl)-4-((tetrahydrofurfuryl)-piperazin-1-yl)pteridin;
- 2-Amino-6-(3,4-dimethoxyphenyl)-4-(1,3-dioxolan-2-yl-methylpiperazin-1-yl)pteridin;
- 2-Amino-4-((3,5-dichlorphenyl)-piperazin-1-yl)-6-(3,4-dimethoxyphenyl)pteridin;
- 2-Amino-6-(3,4-dimethoxyphenyl)-4-((4-fluorphenyl)-piperazin-1-yl)pteridin ;
- 2-Amino-6-(3,4-dimethoxyphenyl)-4-((3-trifluormethylphenyl)-piperazin-1-yl)pteridin;
- 2-Amino-6-(3,4-dimethoxyphenyl)-4-((3,4-dimethylphenyl)-piperazin-1-yl)pteridin;
- 2-Amino-6-(3,4-dimethoxyphenyl)-4-((3-methylphenyl)-piperazin-1-yl)pteridin;
- 2-Amino-6-(3,4-dimethoxyphenyl)-4-((4-methylphenyl)-piperazin-1-yl)pteridin;
- 2-Amino-6-(3,4-dimethoxyphenyl)-4-((2-pyridyl)methyl-piperazin-1-yl)pteridin;
- 2-Amino-6-(3,4-dimethoxyphenyl)-4-(thiazol-2-yl)-piperazin-1-yl)pteridin;
- 2-Amino-6-(3,4-dimethoxyphenyl)-4-(1-(1-methyl-piperidin-3-yl-methyl)-piperazin-1-yl)pteridin;
- 2-Amino-6-(3,4-dimethoxyphenyl)-4-((4-trifluormethylphenyl)-piperazin-1-yl)pteridin;
- 2-Amino-6-(3,4-dimethoxyphenyl)-4-(4-(2-dimethylaminoethyl)-piperazin-1-yl)-pteridintrihydrochloridsalz;
- 2-Amino-6-(3,4-dimethoxyphenyl)-4-(4-(3-dimethylaminopropyl)-piperazin-1-yl) pteridintrihydrochloridsalz;
- 2-Amino-6-(3,4-dimethoxyphenyl)-4-(4-(2-dipropylaminoethyl)-piperazin-1-yl) pteridintrihydrochloridsalz;
- 2-Amino-6-(3,4-dimethoxyphenyl)-4-(4-(2-piperidin-1-yl-ethyl)-piperazin-1-yl) pteridintrihydrochloridsalz;
- 2-Amino-4-[2-trifluoromethyl-4-nitro-phenyl-piperazin-1-yl)-6-(3,4-dimethoxyphenyl)pteridin; und
- 2-Amino-4-[2-(piperazin-1-yl)-essigsäure-N-(2-thiazolyl)-amid]-6-(3,4-dimethoxyphenyl)pteridin.

**5.** Pharmazeutische Zusammensetzung, die als ein Wirkprinzip mindestens ein Pteridinderivat gemäß einem der Ansprüche 1 bis 4 umfasst.

**6.** Pharmazeutische Zusammensetzung gemäß Anspruch 5, die des Weiteren ein oder mehrere biologisch-aktive Arzneimittel umfasst, ausgewählt aus der Gruppe, bestehend aus Immunsuppressiva und/oder Immunmodulatorarzneimitteln, antineoplastischen Arzneimitteln und antiviralen Mitteln.

**7.** Pharmazeutische Zusammensetzung gemäß Anspruch 5, die des Weiteren ein oder mehrere immunsuppressive Arzneimittel umfasst, ausgewählt aus der Gruppe, bestehend aus Cyclosporin A; substituierten Xanthinen; Pentoxyfyllin; Daltroban, Sirolimus, Tacrolimus; Rapamycin; Leflunomid; Mycophenolsäure und deren Salze; adrenocorticalen Steroiden; Azathioprin, Brequinar; Gusperimus; 6-Mercaptopurin; Mizoribin; Chloroquin; Hydroxychloroquin; monoclonalen Antikörpern mit immunsuppressiven Eigenschaften; Etanercept; Infliximab; und Kineret.

**8.** Pharmazeutische Zusammensetzung gemäß Anspruch 5, die des Weiteren ein oder mehrere Immunmodulatorarzneimittel umfasst, augewählt aus der Gruppe, bestehend aus Acemannan, Amiprilose, Bucillamine, Natrium, Imiquimod, Inosine Pranobex, β-Interferon, γ-Interferon, Lentinan, Levamisol, Pidotimod, Romurtid, Platonin, Procodazole, Propagermanium, Thymomodulin, Thymopentin und Ubenimex.

**9.** Pharmazeutische Zusammensetzung gemäß Anspruch 5, die des Weiteren ein oder mehrere antineoplastische Arzneimittel umfasst, ausgewählt aus der Gruppe, bestehend aus Alkaloiden, Alkylierungsmitteln, Alkylsulfonaten, Aziridinen, Ethyleniminen, Methylmelaminen, Stickstoffsenfgas, Nitrosoharnstoffen, Antibiotika, Antimetaboliten, Folsäureanaloga, Purinanaloga, Pyrimidinanaloga, Enzymen, Interferon und Platinkomplexen.

**10.** Pharmazeutische Zusammensetzung gemäß Anspruch 5, die des Weiteren ein oder mehrere antivirale Mittel umfasst, ausgewählt aus der Gruppe, bestehend aus retroviralen Enzyminhibitoren, HIV-1-IN-Inhibitoren, Nucleosidreverstranscriptaseinhibitoren, Zidovudin, Lamivudin, Didanosin, Stavudin, Zalcitabin, Nicht-Nucleosidreversetranscriptaseinhibitoren, Nevirapin, Delavirdin, Natrium-Foscamet, HIV-1-Proteaseinhibitoren, Saquinavir, Ritonavir, Indinavir, Nelfinavir, Acyclovir, Cidofovir, Cytarabin, Edoxudin, Famciclovir, Floxuridin, Ganciclovir, Idoxuridin, Penciclovir, Sorivudin, Trifluridin, Valaciclovir, Vidarabin, Kethoxal, Methisazon, Moroxydin, Podophyllotoxin, Ribavirin, Rimantadin, Stallimycin, Statolon, Tromantadin und Xenazoesäure.

**Revendications**

**1.** Dérivé de la ptéridine ayant la formule générale (I) :

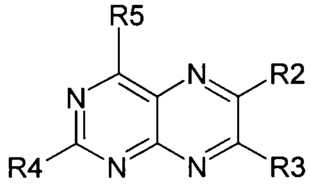

(I)

dans laquelle :

- $R_5$ est un groupe représenté par la formule générale (II):

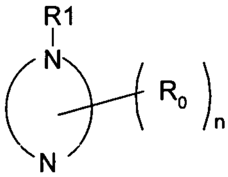

(II)

dans laquelle :

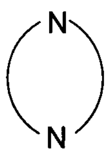

(III)

représente un groupe pipérazin-1-yl ou un groupe homopipérazin-1-yl, et dans laquelle :

- chaque substituant $R_0$ du cycle hétérocyclique (III) est un groupe choisi de manière indépendante parmi le groupe méthyle et le groupe phényle ;
- n est égal à 0, 1 ou 2 ;
- $R_1$ est un groupe de substitution choisi parmi le groupe constitué par un groupe formyle, un groupe acyle, un groupe thioacyle, un groupe amide, un groupe thioamide, un groupe sulfonyle, un groupe sulfinyle, un groupe carboxylate, un groupe thiocarboxylate, un groupe acyle substitué par un groupe amino, un groupe alcoxyalkyle, un groupe cycloalkyl-alkyle $C_{3-10}$, un groupe cycloalkyle $C_{3-10}$, un groupe dialkylaminoalkyle, un groupe alkyle substitué par un groupe hétérocyclique, un groupe alkyle substitué par un groupe acyle, un groupe alkyle substitué par un groupe thioacyle, un groupe alkyle substitué par un groupe amido, un groupe alkyle substitué par un groupe thioamido, un groupe alkyle substitué par un groupe carboxylate, un groupe alkyle substitué par un groupe thiocarboxylate, un groupe alkyl(acyle substitué par un groupe amino), un groupe hétérocyclique, un groupe ester de l'acide carboxylique, un groupe ω-cyanoalkyle, un groupe ω-ester carboxylique-alkyle, un groupe haloalkyle $C_{1-7}$, un groupe alcényle $C_{2-7}$, un groupe alcynyle $C_{2-7}$, un groupe arylalcényle, un groupe aryloxyalkyle, un groupe arylalkyle et un groupe aryle, dans lesquels le groupe caractéristique aryle de chacun desdits radicaux arylalcényle, aryloxyalkyle, arylalkyle et aryle est substitué de manière facultative par un ou plusieurs substituants choisis de manière indépendante parmi le groupe constitué par un groupe halogène, un groupe alkyle $C_{1-7}$, un groupe alcényle $C_{2-7}$, un groupe alcynyle $C_{2-7}$, un groupe haloalkyle $C_{1-7}$, un groupe nitro, un groupe hydroxyle, un groupe sulfhydryle, un groupe amino, un groupe alcoxy $C_{1-7}$, un groupe cycloalcoxy $C_{3-10}$, un groupe aryloxy, un groupe arylalkyloxy, un groupe oxy hétérocyclique, un groupe alkyloxy substitué par un groupe hétérocyclique, un groupe thioalkyle $C_{1-7}$, un groupe thiocycloalkyle $C_{3-10}$, un groupe thioaryle, un groupe thio-hétérocyclique,

un groupe arylalkylthio, un groupe alkylthio substitué par un groupe hétérocyclique, un groupe formyle, un groupe sulfonamido, un groupe hydroxylamino, un groupe alcoxyamino, un groupe mercaptoamino, un groupe thioalkylamino, un groupe acylamino, un groupe thioacylamino, un groupe cyano, un groupe acide ou esters carboxyliques, un groupe alkylamino, un groupe cycloalkylamino, un groupe alcénylamino, un groupe cycloalcénylamino, un groupe alcynylamino, un groupe arylamino, un groupe aryl-alkylamino, un groupe hydroxyalkylamino, un groupe mercaptoalkylamino et un groupe amino hétérocyclique ;
- $R_3$ est un groupe hydrogène ;
- $R_4$ est un groupe amino ;
- $R_2$ est un groupe phényle substitué de manière optionnelle par un ou plusieurs substituants choisis parmi le groupe constitué par un groupe halogène, un groupe alkyle $C_{1-7}$, ou un groupe alcoxy $C_{1-7}$;

et/ou un sel d'addition de ceux-ci acceptable d'un point de vue pharmaceutique et/ou un stéréoisomère de ceux-ci et/ou un mono- ou un di-N-oxyde de ceux-ci et/ou un solvate de ceux-ci et/ou un dérivé dihydro- ou tétrahydroptéridine de ceux-ci.

**2.** Dérivé de la ptéridine selon la revendication 1, dans lequel $R_5$ est un groupe choisi parmi le groupe constitué par un groupe pipérazin-1-yle, un groupe 2-méthylpipérazin-1-yle, un groupe 2-phénylpipérazin-1-yle, un groupe homopipérazin-1-yle et un groupe 2,5-diméthylpipérazin-1-yle, ledit groupe étant substitué en position 4 par un substituant $R_1$ qui a une fonction carbonyl(oxo) ou thiocarbonyl(thioxo) ou sulfonyle.

**3.** Dérivé de la ptéridine selon la revendication 1 ou la revendication 2, dans lequel $R_5$ est un groupe pipérazin-1-yle substitué en position 4 par un substituant $R_1$, dans lequel $R_1$ est choisi parmi le groupe constitué par :

- $COR_8$ dans lequel $R_8$ est choisi parmi le groupe constitué par un groupe hydrogène ; un groupe alkyle $C_{1-7}$ ; un groupe cycloalkyle $C_{3-10}$ ; un groupe aryle substitué de manière facultative par un ou plusieurs substituants choisis parmi le groupe constitué par un groupe halogène, un groupe alkyle $C_{1-7}$, un groupe cyanoalcoxy et un groupe alcoxy $C_{1-7}$; un groupe hétérocyclique substitué de manière facultative par un ou plusieurs atomes d'halogènes ; un groupe arylalkyle; un groupe aryloxyalkyle ; un groupe arylalcoxyalkyle ; un groupe alcoxyalkyle ; un groupe arylalcoxy ; un groupe aryloxy ; un groupe arylalcényle ; un groupe alkyle substitué par un groupe hétérocyclique ; un groupe alkylamino et un groupe arylamino,
- $CSR_9$, dans lequel $R_9$ est choisi parmi le groupe constitué par un groupe alkylamino et un groupe aryloxy,
- $SO_2R_{10}$, dans lequel $R_{10}$ est choisi parmi le groupe constitué par un groupe aryle et un groupe arylalkyle, et
- $R_{11}$, dans lequel $R_{11}$ est choisi parmi le groupe constitué par un groupe aryle, un groupe arylalkyle, un groupe arylalcényle, un groupe alcoxyalkyle, un groupe alkyle substitué par un groupe hétérocyclique, un groupe cycloalkyl-alkyle $C_{3-10}$, un groupe hétérocyclique, un groupe cycloalkyle $C_{3-10}$, un groupe dialkylaminoalkyle, groupe aryloxyalkyle, un groupe ω-cyanoalkyle, un groupe ω-carboxylato-alkyle et un groupe carboxamidoalkyle.

**4.** Dérivé de la ptéridine selon l'une quelconque des revendications 1 à 3, choisi parmi le groupe constitué par :

- la 2-amino-4-(N-acétylpipérazin-1-yl)-6-(3,4-diméthoxyphényl)ptéridine ;
- la 2-amino-4-[(N-propionyl)-pipérazin-1-yl]-6-(3,4-diméthoxyphényl)ptéridine ;
- la 2-amino-4-[(N-hexanoyl)-pipérazin-1-yl]-6-(3,4-diméthoxyphényl)ptéridine ;
- la 2-amino-4-(N-benzoylpipérazin-1-yl)-6-(3,4-diméthoxyphényl)ptéridine ;
- la 2-amino-4-[N-(4-chlorobenzoyl)]pipérazin-1-yl)-6-(3,4-diméthoxyphényl)ptéridine ;
- la 2-amino-4-[(N-2-thiophènecarbonyl)-pipérazin-1-yl]-6-(3,4-diméthoxy-phényl)ptéridine ;
- la 2-amino-4-[(N-diéthylcarbamoyl)-pipérazin-1-yl]-6-(3,4-diméthoxy-phényl)ptéridine ;
- la 2-amino-4-[(N-hydrocinnamoyl)-pipérazin-1-yl]-6-(3,4-diméthoxyphényl)ptéridine ;
- la 2-amino-4-[N-(4-cyanobenzoyl)-pipérazin-1-yl]-6-(3,4-diméthoxyphényl)ptéridine ;
- la 2-amino-4-[(N-phénoxyacétyl)-pipérazin-1-yl]-6-(3,4-diméthoxy-phényl)ptéridine ;
- la 2-amino-4-[(N-4-butylbenzoyl)-pipérazin-1-yl]-6-(3,4-diméthoxyphényl)ptéridine ;
- la 2-amino-4-[(N-isonicotinoyl)-pipérazin-1-yl]-6-(3,4-diméthoxyphényl)ptéridine ;
- la 2-amino-4-[(N-diisopropylcarbamoyl)-pipérazin-1-yl]-6-(3,4-diméthoxy-phényl)ptéridine ;
- la 2-amino-4-[N-(4-pentoxybenzoyl)-pipérazin-1-yl]-6-(3,4-diméthoxy-phényl)ptéridine ;
- la 2-amino-4-[N-(3-méthoxybenzoyl)pipérazin-1-yl]-6-(3,4-diméthoxy-phényl)ptéridine ;
- la 2-amino-4-[N-(2-furoyl)-pipérazin-1-yl]-6-(3,4-diméthoxyphényl)ptéridine ;
- la 2-amino-4-[(N-benzyloxyacétyl)-pipérazin-1-yl]-6-(3,4-diméthoxyphényl)ptéridine ;
- la 2-amino-4-[(N-(p-chlorophénoxyacétyl)-pipérazin-1-yl]-6-(3,4-diméthoxyphényl) ptéridine ;

- la 2-amino-4-[(N-cyclohexylcarbonyl)-pipérazin-1-yl]-6-(3,4-diméthoxyphényl)ptéridine ;
- la 2-amino-4-[(N-phénylsulfonyl)-pipérazin-1-yl]-6-(3,4-diméthoxyphényl)ptéridine ;
- la 2-amino-4-[(N-p-fluorobenzoyl)-pipérazin-1-yl]-6-(3,4-diméthoxyphényl)ptéridine ;
- la 2-amino-4-[(N-2-thiophénacétyl)-pipérazin-1-yl]-6-(3,4-diméthoxy-phényl)ptéridine ;
- la 2-amino-4-[(N-cinnamoyl)-pipérazin-1-yl]-6-(3,4-diméthoxyphényl)ptéridine ;
- la 2-amino-4-[(N-1-pyrrolidinylcarbonyl)-pipérazin-1-yl]-6-(3,4-diméthoxyphényl) ptéridine ;
- la 2-amino-4-[(N-diphénylcarbamoyl)-pipérazin-1-yl]-6-(3,4-diméthoxyphényl)ptéridine ;
- la 2-amino-4-[N-(2,6-dichloro-5-fluoro-nicotinoyl)]-pipérazin-1-yl]-6-(3,4- diméthoxyphényl)ptéridine ;
- la 2-amino-4-[(N-méthoxyacétyl)-pipérazin-1-yl]-6-(3,4-diméthoxyphényl)ptéridine ;
- la 2-amino-4-[N(2-méthoxybenzoyl)-pipérazin-1-yl]-6-(3,4-diméthoxy-phényl)ptéridine ;
- la 2-amino-4-[(N-benzylsulfonyl)-pipérazin-1-yl]-6-(3,4-diméthoxyphényl)ptéridine ;
- la 2-amino-4-[N-(3,4-dichlorobenzoyl)-pipérazin-1-yl]-6-(3,4-diméthoxyphényl)ptéridine ;
- la 2-amino-4-[N-(4-chlorophénylacétyl)-pipérazin-1-yl]-6-(3,4-diméthoxyphényl)ptéridine ;
- la 2-amino-4-[(N-(1-naphtoyl)-pipérazin-1-yl]-6-(3,4-diméthoxyphényl)ptéridine ;
- la 2-amino-4-[N-(3-furoylcarbonyl)-pipérazin-1-yl]-6-(3,4-diméthoxyphényl)ptéridine ;
- la 2-amino-4-[(N-benzyloxycarbonyl)-pipérazin-l-yl]-6-(3,4-diméthoxyphényl)ptéridine ;
- la 2-amino-4-[(N-diméthylthiocarbamoyl)-pipérazin-1-yl]-6-(3,4-diméthoxyphényl)ptéridine ;
- la 2-amino-4-[(N-phénoxycarbonyl)-pipérazin-1-yl]-6-(3,4-diméthoxyphényl)ptéridine ;
- la 2-amino-4-[(N-phénoxythiocarbonyl)-pipérazin-1-yl]-6-(3,4-diméthoxyphényl)ptéridine ;
- la 2-amino-4-[N-(2-(S)-amino-3-phénylpropionyl)-pipérazin-1-yl]-6-(3,4-diméthoxyphényl) ptéridine ;
- la 2-amino-4-[N-[2-(S)-amino-3-(4-hydroxyphényl)propionyl]-pipérazin-1-yl]-6-(3,4- diméthoxyphényl) ptéridine ;
- la -2-amino-4-[N-(pyrrolidin-2-(S)-yl)carbonyl-pipérazin-1-yl]-6-(3,4-diméthoxyphényl) ptéridine ;
- la 2-amino-4-[[N-2-(S)-amino-3-(indol-2-yl)propionyl]-pipérazin-1-yl]-6-(3,4- diméthoxyphényl)ptéridine ;
- la 2-amino-4-(N-phénoxyacétyl)-homopipérazin-1-yl)-6-(3,4-diméthoxyphényl)ptéridine) ;
- la 2-amino-4-[(N-4-méthyl-phénoxy-carbonyl)-pipérazin-1-yl]-6-(3,4-diméthoxyphényl) ptéridine ;
- la 2-amino-4-[(N-4-méthoxy-phénoxy-carbonyl)-pipérazin-1-yl]-6-(3,4-diméthoxyphényl) ptéridine ;
- la 2-amino-4-[N-(2-méthoxy)-phénoxy-carbonyl)-pipérazin-1-yl]-6-(3,4-diméthoxyphényl) ptéridine ;
- la 2-amino-4-[N-(4-chloro)-phénoxy-carbonyl)-pipérazin-1-yl]-6-(3,4-diméthoxyphényl) ptéridine ;
- la 2-amino-4-[N-isobutoxy-carbonyl-pipérazin-1-yl]-6-(3,4-diméthoxyphényl)ptéridine ;
- la 2-amino-4-[N-(2-chloro)-phénoxy-carbonyl-pipérazin-1-yl]-6-(3,4-diméthoxyphényl) ptéridine ;
- la 2-amino-4-[N-(2-méthoxy)-éthoxy-carbonyl)-pipérazin-1-yl]-6-(3,4-diméthoxyphényl) ptéridine ;
- la 2-amino-4-[N-(2-naphthoxy)-carbonyl)-pipérazin-1-yl]-6-(3,4-diméthoxyphényl)ptéridine ;
- la 2-amino-4-(N-phényl-carbamoyl-pipérazin-1-yl)-6-(3,4-diméthoxyphényl)ptéridine ;
- la 2-amino-4-[N-4-fluorophényl-carbamoyl-pipérazin-1-yl)]-6-(3,4-diméthoxyphényl) ptéridine ;
- la 2-amino-4-(N-4-méthylphényl-carbamoyl-pipérazin-1-yl)-6-(3,4-diméthoxyphényl) ptéridine ;
- la 2-amino-4-(N-4-cyanophénylcarbamoyl-pipérazin-1-yl)-6-(3,4-diméthoxyphényl)ptéridine ;
- la 2-amino-4-(N-3-méthylphénylcarbamoyl-pipérazin-1-yl)-6-(3,4-diméthoxyphényl)ptéridine ;
- la 2-amino-4-(N-benzylcarbamoyl-pipérazin-1-yl)-6-(3,4-diméthoxyphényl)ptéridine ;
- la 2-amino-4-(N-4-fluorobenzylcarbamoyl-pipérazin-1-yl)-6-(3,4-diméthoxyphényl)ptéridine ;
- la 2-amino-4-(N-3-chloro-4-fluorophénylcarbamoyl-pipérazin-1-yl)-6-(3,4-diméthoxyphényl) ptéridine ;
- la 2-amino-4-(N-3-thiénylcarbamoyl-pipérazin-1-yl)-6-(3,4-diméthoxyphényl)ptéridine ;
- la 2-amino-4-[N-2-(2-thiényl)éthylcarbamoyl-pipérazin-1-yl]-6-(3,4-diméthoxyphényl) ptéridine ;
- la 2-amino-4-[(N-butyl-carbamoyl-pipérazin-1-yl)]-6-(3,4-diméthoxyphényl)ptéridine ;
- la 2-amino-4-[N-aminoacétyl]-pipérazin-1-yl]-6-(3,4-diméthoxyphényl)ptéridine ;
- la 2-amino-4-[N-[2-(S),4-diamino-4-oxobutanoyl]-pipérazin-1-yl]-6-(3,4-diméthoxyphényl) ptéridine ;
- la 2-amino-4-[N-[4-(méthoxycarbonyl)benzoyl]-pipérazin-1-yl]-6-(3,4-diméthoxyphényl) ptéridine ;
- la 2-amino-4-[N-[4-(diméthylamino)acétyl]-pipérazin-1-yl]- 6-(3,4-diméthoxyphényl) ptéridine ;
- la 2-amino-4-[N-(4-amino-4-oxo-butanoyl)-pipérazin-1-yl]-6-(3,4-diméthoxyphényl) ptéridine ;
- la 2-amino-4-[N-(3-carboxypropanoyl)-pipérazin-1-yl]-6-(3,4-diméthoxyphényl)ptéridine ;
- la 2-amino-4-[N-[4-(carboxy)benzoyl]-pipérazin-1-yl]-6-(3,4-diméthoxyphényl)ptéridine ;
- la 2-amino-4-(N-méthyl-N-phényl-carbamoyl-pipérazin-1-yl)-6-(3,4-diméthoxyphényl) ptéridine ;
- la 2-amino-4-(N-éthyl-N-phényl-carbamoyl-pipérazin-1-yl)-6-(3,4-diméthoxyphényl) ptéridine ;
- la 2-amino-4-(N-méthyl-N-tolyl-carbamoyl-pipérazin-1-yl)-6-(3,4-diméthoxyphényl) ptéridine ;
- la 2-amino-4-(1-(2-méthoxyéthyl)pipérazino)-6-(3,4-diméthoxyphényl)ptéridine ;
- la 2-amino-4-(1-cyclohexylméthyl)pipérazino)-6-(3,4-diméthoxyphényl)ptéridine ;
- la 2-amino-4-(1-cyclopentylpipérazino)-6-(3,4-diméthoxyphényl)ptéridine ;
- la 2-amino-4-(1-butylpipérazino)-6-(3,4-diméthoxyphényl)ptéridine ;

- la 2-amino-4-(1-isopropylpipérazino)-6-(3,4-diméthoxyphényl)ptéridine ;
- la 2-amino-4-(1-(2-diéthylaminoéthyl)-pipérazino)-6-(3,4-diméthoxyphényl)ptéridine ;
- la 2-amino-4-(1-(2-diisopropylaminoéthyl)-pipérazino)-6-(3,4-diméthoxyphényl)ptéridine ;
- la 2-amino-4-(1-(2-morpholino-4-yl-éthyl)-pipérazino)-6-(3,4-diméthoxyphényl)ptéridine ;
- la 2-amino-4-(4-[2-(pipérazin-1-yl)-acétyl]-morpholino)-6-(3,4-diméthoxyphényl)ptéridine ;
- la 2-amino-4-(4-[2-(pipérazin-1-yl)-acétyl]-pyrrolidino)-6-(3,4-diméthoxyphényl) ptéridine;
- la 2-amino-4-(2-[pipérazin-1-yl]-acide acétique N-métyl N-phényl amide)-6-(3,4- diméthoxyphényl)ptéridine ;
- la 2-amino-4-(2-(pipérazin-1-yl)-ester méthylique de l'acide propionique)-6-(3,4- diméthoxyphényl)ptéridine ;
- la 2-amino-4-(3-pipérazin-1-yl)-ester éthylique de l'acide propionique)-6-(3,4- diméthoxyphényl)ptéridine ;
- la 2-amino-4-(2-(pipérazin-1-yl)-ester éthylique de l'acide acétique)-6-(3,4- diméthoxyphényl)ptéridine ;
- la 2-amino-4-(1-(3-méthyl-benzyl)pipérazinyl)-6-(3,4-diméthoxyphényl)ptéridine ;
- la 2-amino-4-[(2,6-dichlorobenzyl)pipérazin-1-yl)-6-(3,4-diméthoxyphényl)ptéridine ;
- la 2-amino-4-((4-fluorobenzyl)pipérazin-1-yl)-6-(3,4-diméthoxyphényl)ptéridine ;
- la 2-amino-4-((4-chlorobenzyl)pipérazin-1-yl)-6-(3,4-diméthoxyphényl)ptéridine ;
- la 2-amino-4-((4-méthylbenzyl)pipérazin-1-yl)-6-(3,4-diméthoxyphényl)ptéridine ;
- la 2-amino-4-((2-fluorobenzyl)pipérazin-1-yl)-6-(3,4-diméthoxyphényl)ptéridine ;
- la 2-amino-4-((3,4-dichlorobenzyl)pipérazin-1-yl)-6-(3,4-diméthoxyphényl)ptéridine ;
- la 2-amino-4-(pipéronyl-pipérazin-1-yl)-6-(3,4-diméthoxyphényl)ptéridine ;
- la 2-amino-4-((4-tert-butylbenzyl) pipérazin-1-yl)-6-(3,4-diméthoxyphényl)ptéridine;
- la 2-amino-4-((4-pyridyl)-pipérazin-1-yl)-6-(3,4-diméthoxyphényl)ptéridine;
- la 2-amino-4-((2-pyridiyl)-pipérazin-1-yl)-6-(3,4-diméthoxyphényl)ptéridine;
- la 2-amino-4-((2-pyrimidinyl)-pipérazin-1-yl)-6-(3,4-diméthoxyphényl) ptéridine ;
- la 2-amino-4-((3-méthoxyphényl)-pipérazin-1-yl)-6-(3,4-diméthoxyphényl)ptéridine;
- la 2-amino-4-(1-(3-phénylpropyl-pipérazine)-6-(3,4-diméthoxyphényl)ptéridine;
- la 2-amino-4-((3,4-dichlorophényl)-pipérazin-1-yl)-6-(3,4-diméthoxyphényl)ptéridine ;
- la 2-amino-4-((3-dichlorophényl)-pipérazin-1-yl)-6-(3,4-diméthoxyphényl)ptéridine ;
- la 2-amino-4-((1-phényléthyl)-pipérazin-1-yl)-6-(3,4-diméthoxyphényl)ptéridine ;
- la 2-amino-4-((2-(1-pyrrolyl)-éthyl-pipérazin-1-yl)-6-(3,4-diméthoxyphényl)ptéridine ;
- la 2-amino-4-((2-phénoxyéthyl-pipérazin-1-yl)-6-(3,4-diméthoxyphényl)ptéridine ;
- la 2-amino-4-(1-(2-imidazol-1-yl-éthyl-pipérazine)-6-(3,4-diméthoxyphényl)ptéridine ;
- la 2-amino-4-((3-pyridyl)méthyl)-pipérazin-1-yl)-6-(3,4-diméthoxyphényl)ptéridine;
- la 2-amino-4-((4-pyridyl)-méthyl)-pipérazin-1-yl)-6-(3,4-diméthoxyphényl)ptéridine ;
- la 2-amino-4-((1-naphtylméthyl)-pipérazin-1-yl)-6-(3,4-diméthoxyphényl)ptéridine ;
- la 2-amino-4-(N-phénéthylpipérazin-1-yl)-6-(3,4-diméthoxyphényl)ptéridine ;
- la 2-amino-4-((2-méthoxyphényl)-pipérazin-1-yl)-6-(3,4-diméthoxyphényl)ptéridine ;
- la 2-amino-4-((4-méthoxyphényl)-pipérazin-1-yl)-6-(3,4-diméthoxyphényl)ptéridine ;
- la 2-amino-4-((4-chlorophényl)-pipérazin-1-yl)-6-(3,4-diméthoxyphényl)ptéridine ;
- la 2-amino-6-(3,4-diméthoxyphényl)-4-(4-(3-propionitril)-pipérazin-1-yl)ptéridine ;
- la 2-amino-6-(3,4-diméthoxyphényl)-4-(4-(2-(1,3)-dioxolan-2-yl-éthyl)-pipérazin-1-yl) ptéridine ;
- la 2-amino-6-(3,4-diméthoxyphényl)-4-(4-(2-éthoxyéthyl)-pipérazin-1-yl)ptéridine ;
- la 2-amino-6-(3,4-diméthoxyphényl)-4-(pent-3-yl-pipérazin-1-yl)ptéridine ;
- la 2-amino-6-(3,4-diméthoxyphényl)-4-(1-pentyl-pipérazin-1-yl)ptéridine ;
- la 2-amino-6-(3,4-diméthoxyphényl)-4-(1-isobutyl-pipérazin-1-yl)ptéridine;
- la 2-amino-6-(3,4-diméthoxyphényl)-4-((tétrahydrofurfuryl)-pipérazin-1-yl)ptéridine ;
- la 2-amino-6-(3,4-diméthoxyphényl)-4-(1,3-dioxolan-2-yl-méthylpipérazin-1-yl)ptéridine ;
- la 2-amino-4-((3,5-dichlorophényl)-pipérazin-1-yl)-6-(3,4-diméthoxyphényl)ptéridine ;
- la 2-amino-6-(3,4-diméthoxyphényl)-4-((4-fluorophényl)-pipérazin-1-yl)ptéridine ;
- la 2-amino-6-(3,4-diméthoxyphényl)-4-((3-trifluorométhylphényl)-pipérazin-1-yl)ptéridine ;
- la 2-amino-6-(3,4-diméthoxyphényl)-4-((3,4-diméthylphényl)-pipérazin-1-yl)ptéridine ;
- la 2-amino-6-(3,4-diméthoxyphényl)-4-((3-méthylphényl)-pipérazin-1-yl)ptéridine ;
- la 2-amino-6-(3,4-diméthoxyphényl)-4-((4-méthylphényl)-pipérazin-1-yl)ptéridine ;
- la 2-amino-6-(3,4-diméthoxyphényl)-4-((2-pyridyl)méthyl-pipérazin-1-yl)ptéridine ;
- la 2-amino-6-(3,4-diméthoxphényl)-4-(thiazol-2-yl)-pipérazin-1-yl)ptéridine ;
- la 2-amino-6-(3,4-diméthoxyphényl)-4-(1-(1-méthyl-pipéridin-3-yl-méthyl)-pipérazin-1-yl) ptéridine ;
- la 2-amino-6-(3,4-diméthoxyphényl)-4-((4-trifluorométhylphényl)-pipérazin-1-yl)ptéridine ;
- le sel de trichlorhydrate de 2-amino-6-(3,4-diméthoxyphényl)-4-(4-(2-diméthylaminoéthyl)- pipérazin-1-yl)-ptéridine ;
- le sel de trichlorhydrate de 2-amino-6-(3,4-diméthoxyphényl)-4-(4-(3-diméthylaminopropyl)- pipérazin-1-yl)ptéridine ;
- le sel de trichlorhydrate de 2-amino-6-(3,4-diméthoxyphényl)-4-(4-(2-dipropylaminoéthyl)- pipérazin-1-yl) ptéridine ;
- le sel de trichlorhydrate de 2-amino-6-(3,4-diméthoxyphényl)-4-(4-(2-pipéridin-1-yl- éthyl)-pipérazin-1-yl) ptérine ;
- la 2-amino-4-[2-trifluorométhyl-4-nitro-phényl-pipérazin-1-yl)-6-(3,4-diméthoxyphényl) ptéridine ; et
- la 2-amino-4-[2-(pipérazin-1-yl)-acide acétique N-(2-thiazolyl)-amide]-6-(3,4- diméthoxyphényl)ptéridine.

**5.** Composition pharmaceutique comprenant en tant que principe actif au moins un dérivé de la ptéridine selon l'une quelconque des revendications 1 à 4.

**6.** Composition pharmaceutique selon la revendication 5, comprenant de plus un ou plusieurs médicaments actifs d'un point de vue biologique choisis parmi le groupe constitué par les médicaments immunosuppresseurs et/ou immunomodulateurs, les médicaments anticancéreux et les agents antiviraux.

**7.** Composition pharmaceutique selon la revendication 5, comprenant de plus un ou plusieurs médicaments immunosuppresseurs choisis parmi le groupe constitué par la cyclosporine A ; les xanthines substituées ; la pentoxyfylline ; le daltroban, le sirolimus, le tacrolimus ; la rapamycine; le léflunomide ; l'acide mycophénolique et ses sels ; les stéroïdes adrénocorticaux ; l'azathioprine, le brequinar ; le gusperimus ; la 6-mercaptopurine ; la mizoribine ; la chloroquine ; l'hydroxychloroquine ; les anticorps monoclonaux avec des propriétés immunosuppressives ; l'étanercept ; l'infliximab ; et le kineret.

**8.** Composition pharmaceutique selon la revendication 5, comprenant de plus un ou plusieurs médicaments immunomodulateurs choisis parmi le groupe constitué par l'acemannan, l'amiprilose, la bucillamine, le ditiocarb sodium, l'imiquimod, l'inosine, le pranobex, l'interferon-β, l'interféron-γ, le lentinan, le levamisole, le pidotimod, le romurtide, le platonin, le procodazole, le propagermanium, la thymomoduline, la thymopentine et l'ubénimex.

**9.** Composition pharmaceutique selon la revendication 5, comprenant de plus un ou plusieurs médicaments anticancéreux choisis dans le groupe constitué par les alcaloïdes, les agents alkylants, les alkylsulfonates, les aziridines, les éthylènimines, les méthylmélamines, la moutarde azotée, les nitrosourées, les antibiotiques, les antimétabolites, les analogues de l'acide folique, les analogues de la purine, les analogues de la pyrimidine, les enzymes, l'interféron et les complexes du platine.

**10.** Composition pharmaceutique selon la revendication 5, comprenant de plus un ou plusieurs agents antiviraux choisis parmi le groupe constitué par les inhibiteurs des enzymes rétrovirales, les inhibiteurs de l'IN du VIH-1, les inhibiteurs nucléosidiques de la transcriptase inverse, la zidovudine, la lamivudine, la didanosine, la stavudine, la zalcitabine, les inhibiteurs non nucléosidiques de la transcriptase inverse, la névirapine, la delavirdine, le foscarnet sodium, les inhibiteurs de la protéase du VIH-1, le saquinavir, le ritonavir, l'indinavir, le nelfinavir, l'acyclovir, le cidofovir, la cytarabine, l'édoxudine, le famciclovir, la floxuridine, le ganciclovir, l'idoxuridine, le penciclovir, la sorivudine, la trifluridine, le valaciclovir, la vidarabine, le kethoxal, la méthisazone, la moroxydine, la podophyllotoxine, la ribavirine, la rimantadine, la stallimycine, le statolon, la tromantadine et l'acide xénazoïque.

Figure 1

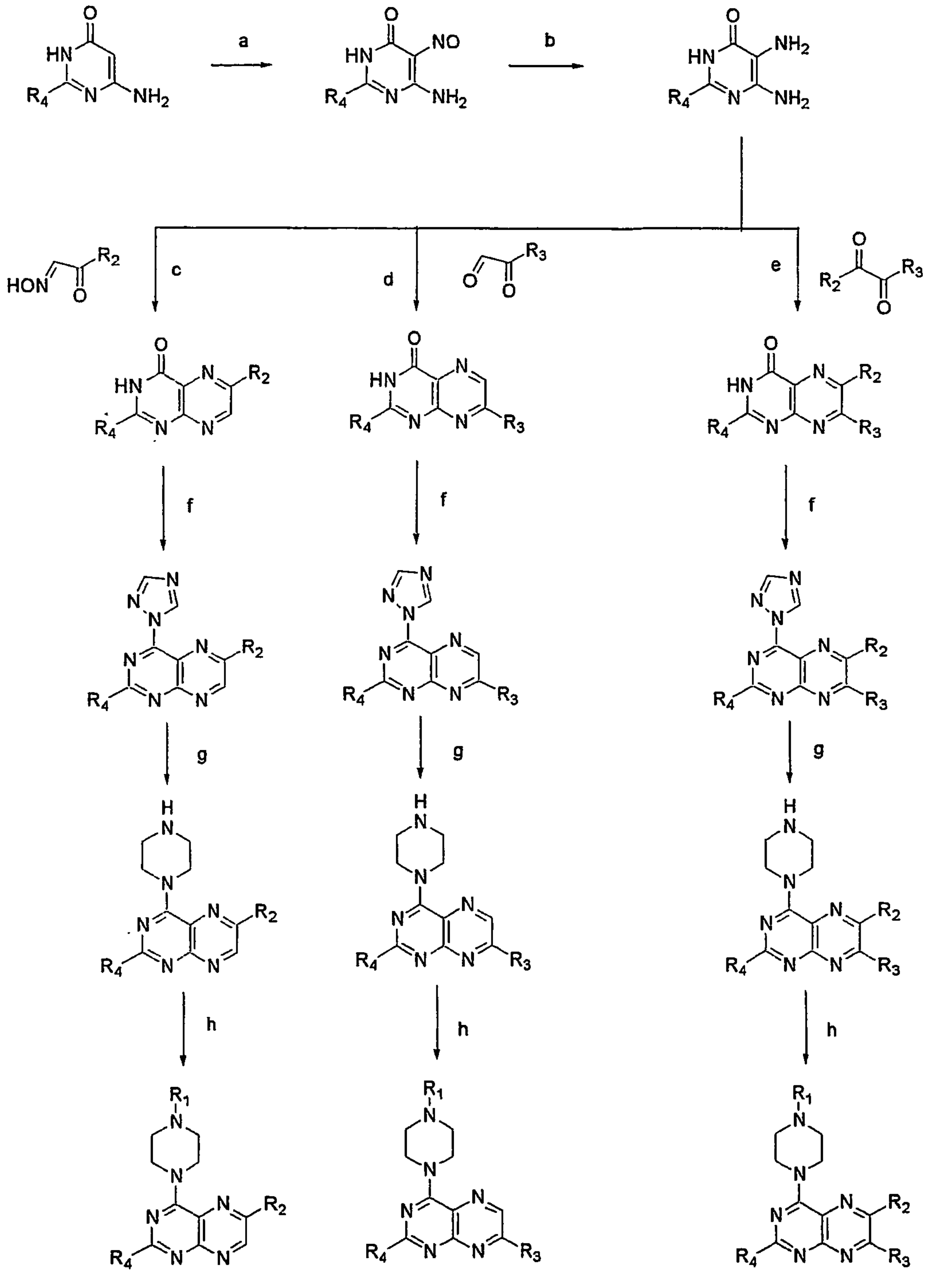

Figure 2

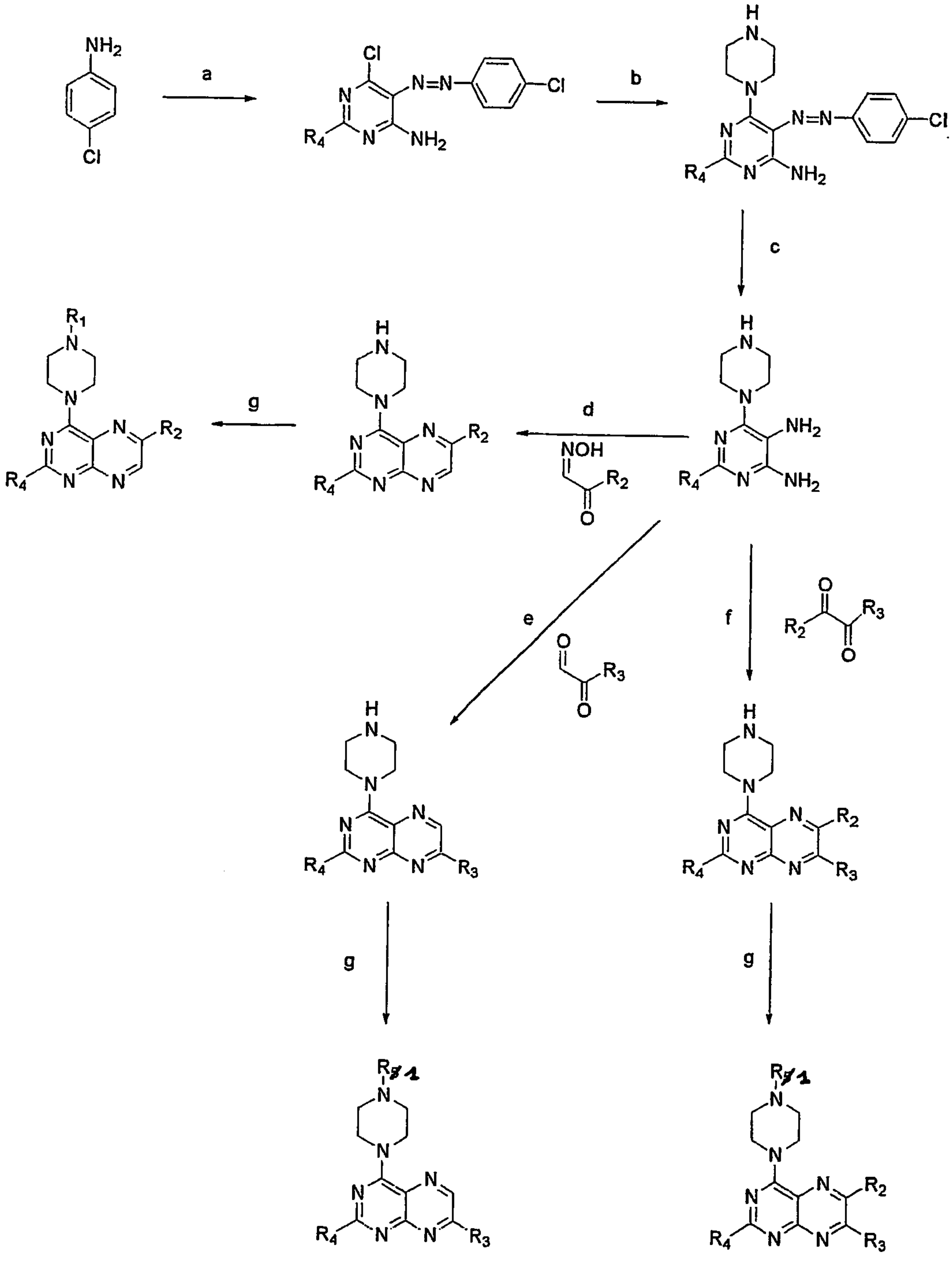

## Figure 3

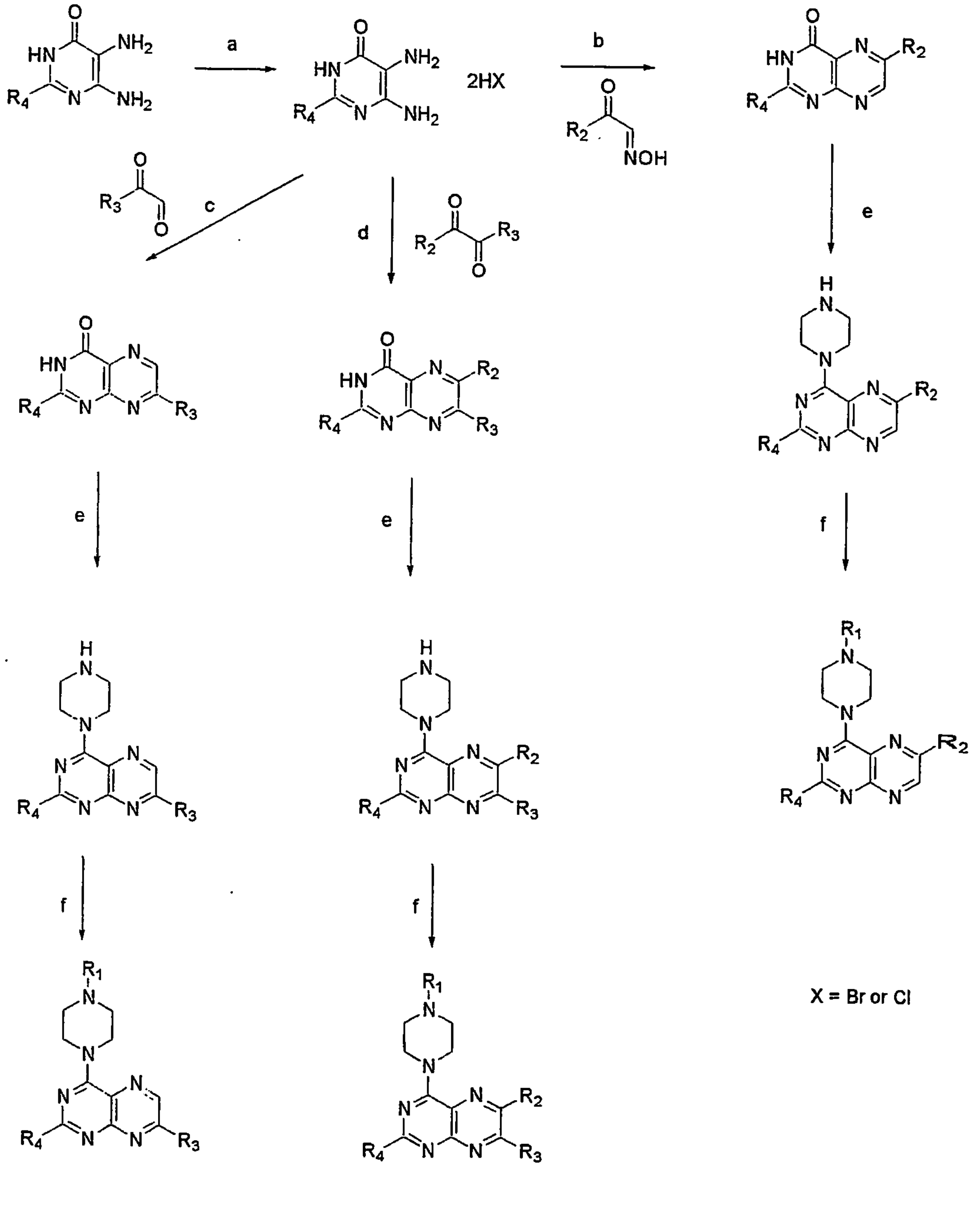

Figure 4

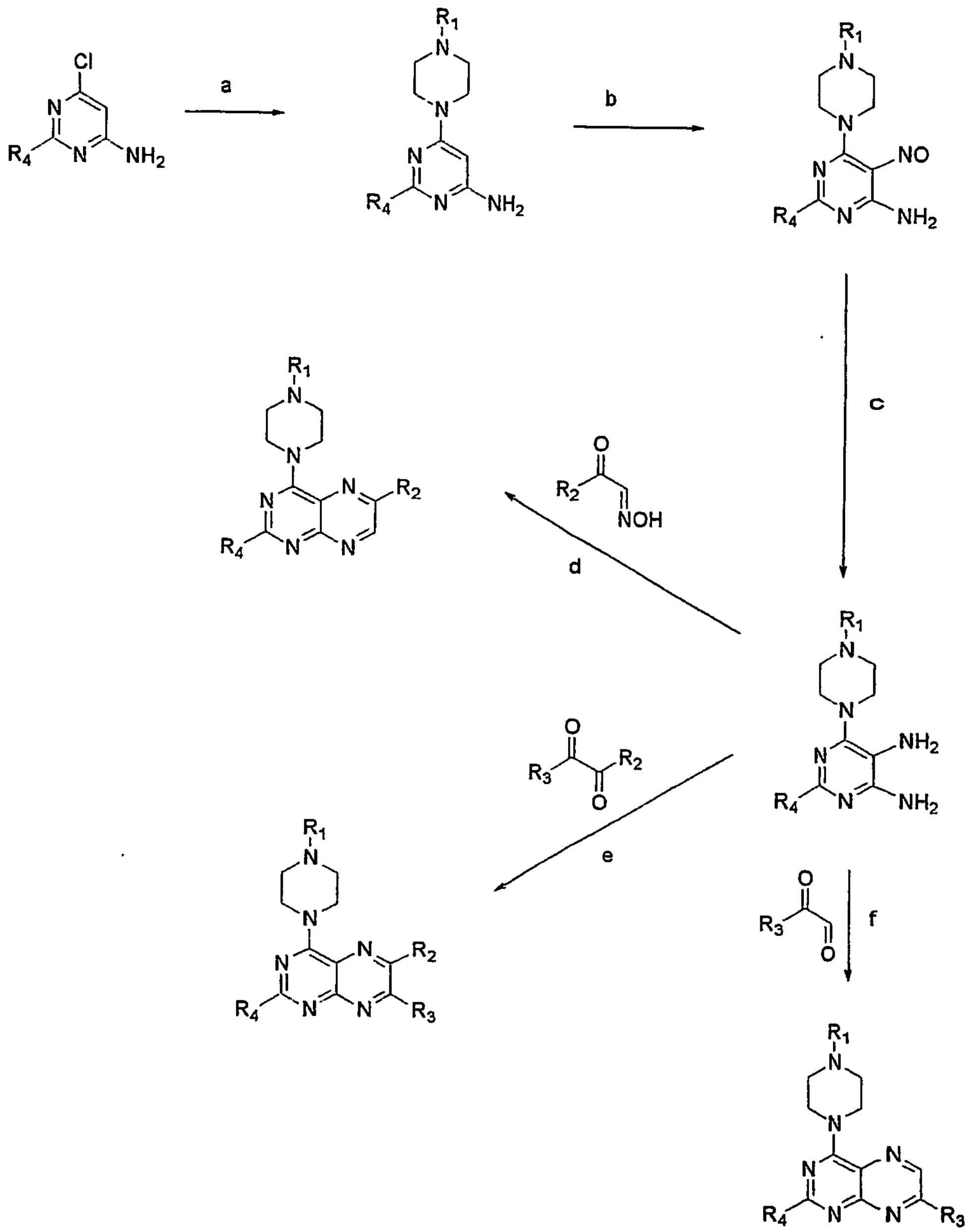

Figure 5

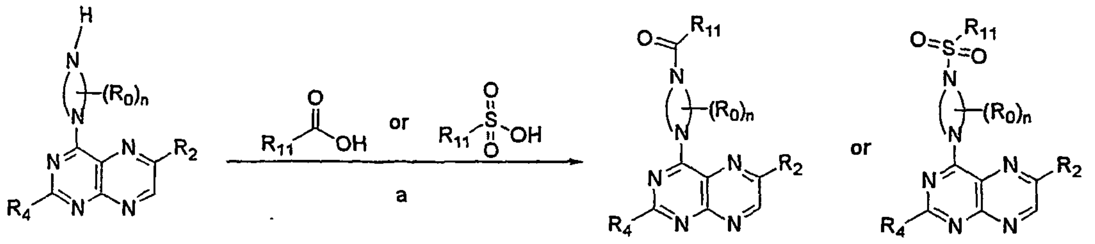


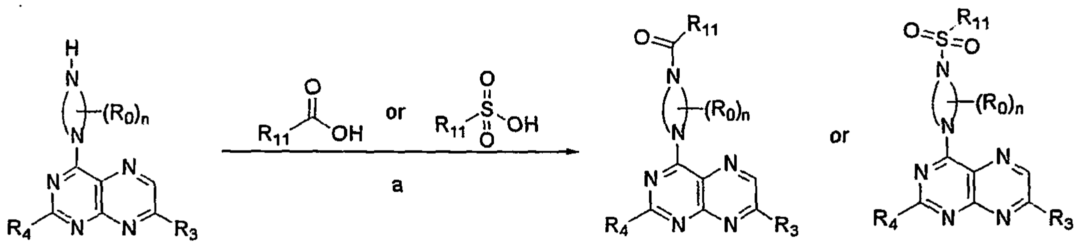


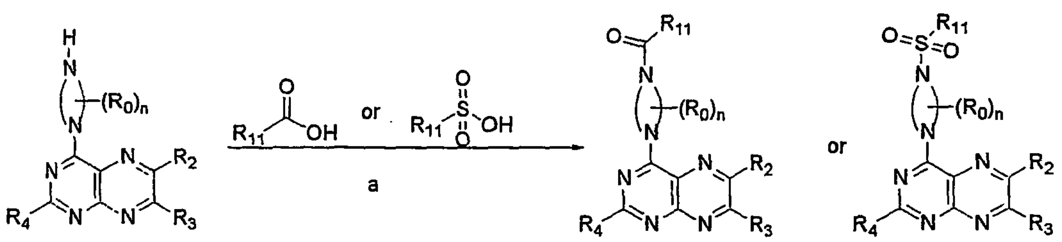

Figure 6

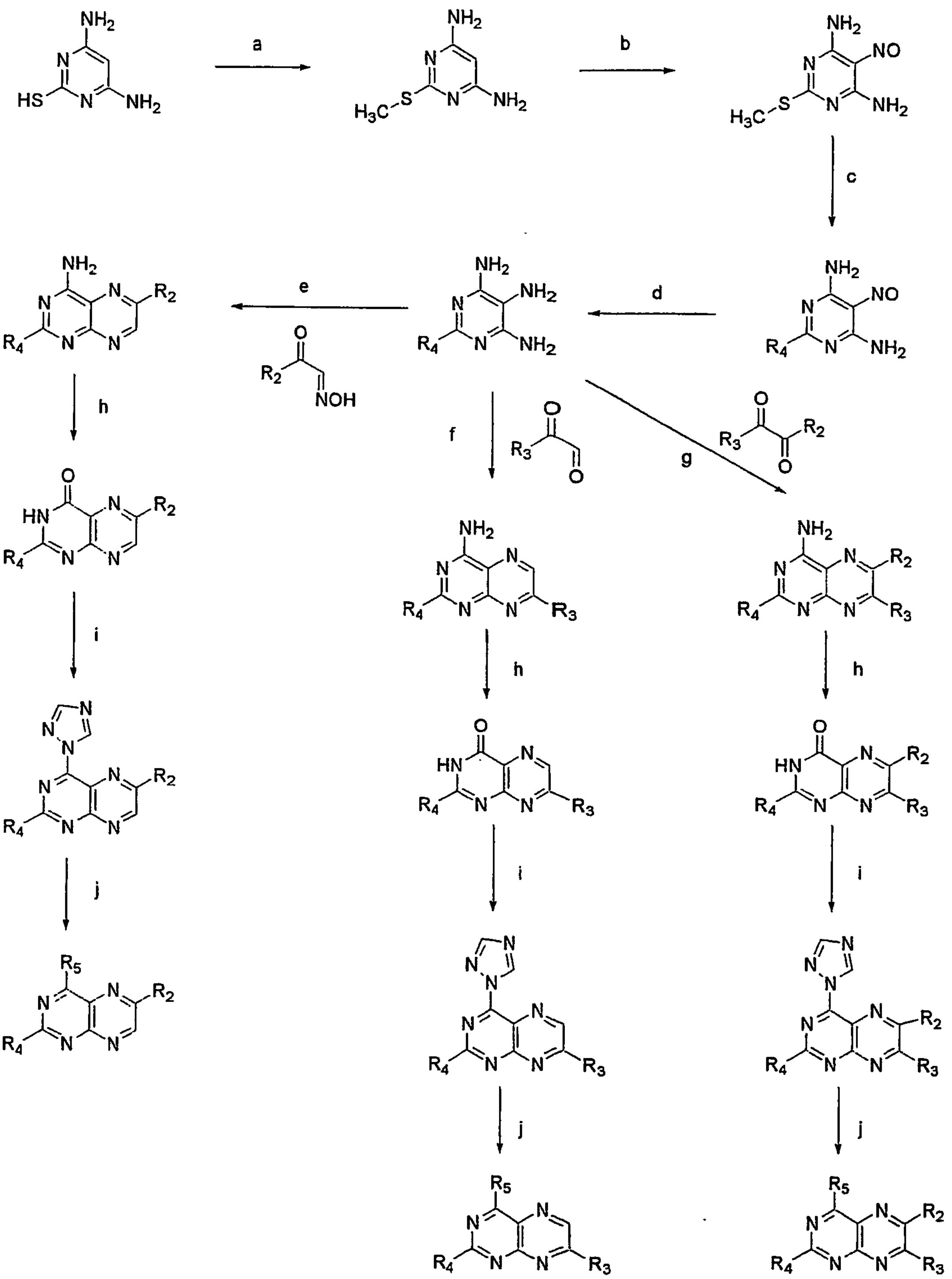

## Figure 7

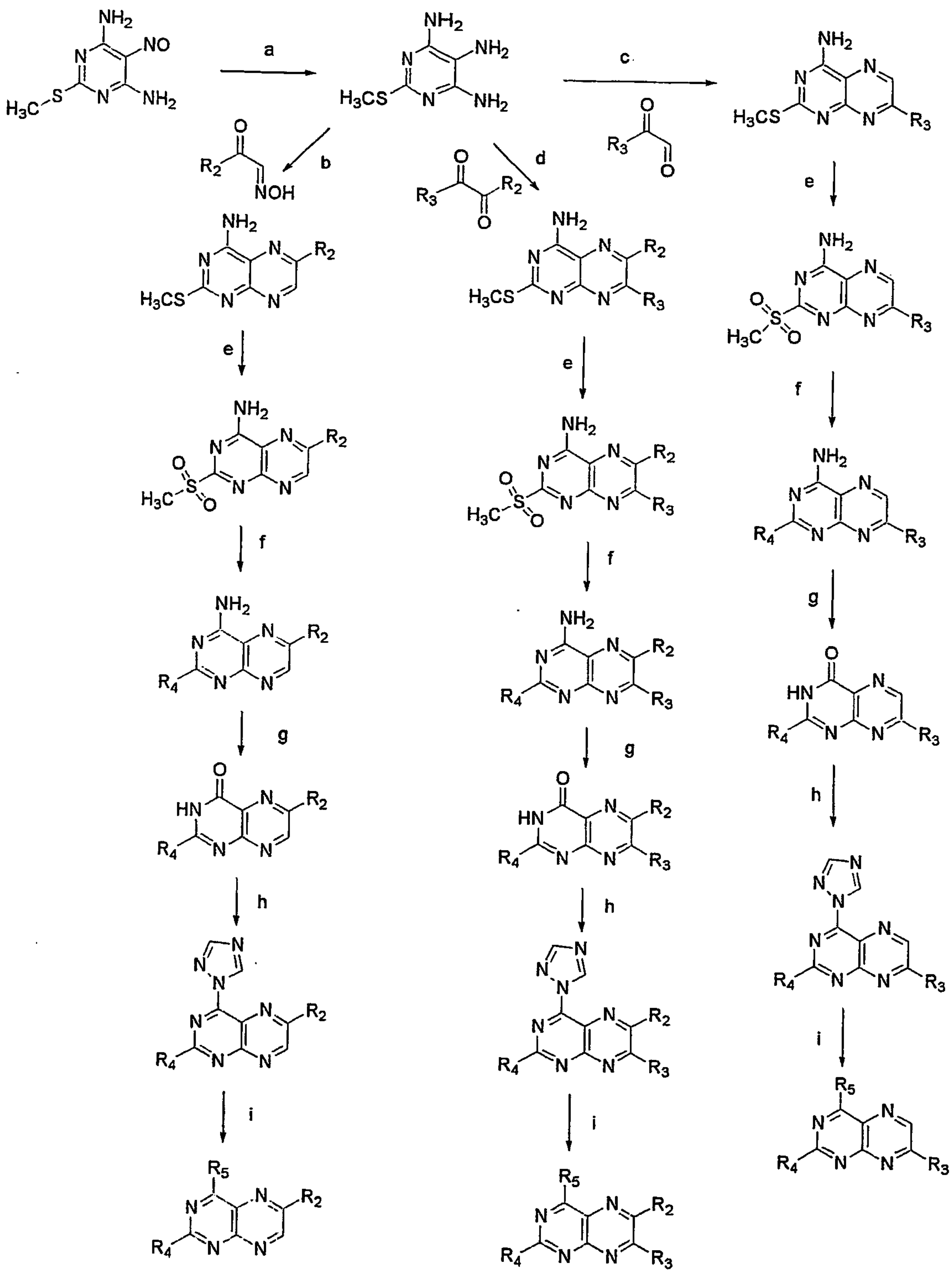

Figure 8 (reference)

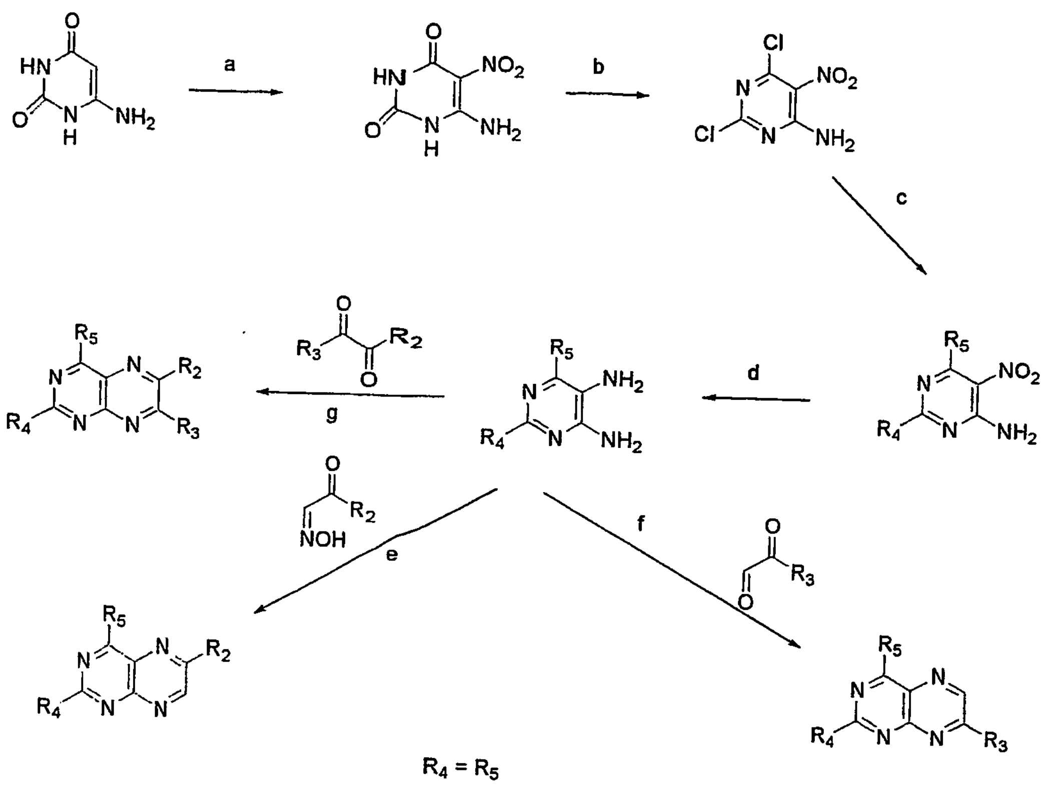


$R_4 = R_5$

Figure 9

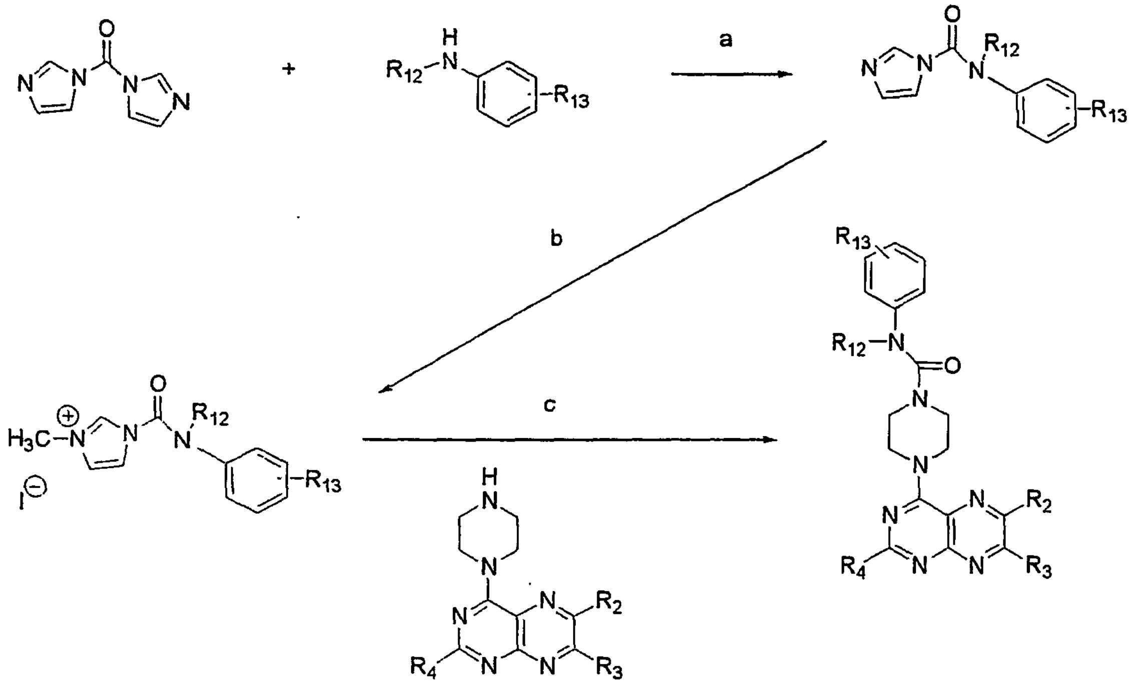

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CH 231852
- GB 763044 A
- US 2512572 A
- US 2581889 A
- US 2665275 A
- US 2667486 A
- US 2940972 A
- US 3081230 A
- US 5047405 A
- EP 362645 A
- EP 185259 A
- EP 574906 A
- WO 0232507 A
- WO 0039129 A
- WO 0121619 A
- US 5665772 A
- US 5780462 A
- US 6440991 B
- US 6331547 B


### Non-patent literature cited in the description


- **MERZ et al.** *J. Medicinal Chem.,* 1998, vol. 41, 4733-4743
- **NENOVA et al.** *Archives of Hellenic Medicine,* 2000, vol. 17, 619-621
- **SAARINEN et al.** *Cancer Research,* 1990, vol. 50, 592-595
- **RAUTONEN et al.** *AIDS,* 1991, vol. 5, 1319-1325
- **SWAPAN et al.** *Journal of Virology,* 2002, vol. 76, 11710-11714
- **JAQUES et al.** Enantiomers, Racemates and Resolution. *Wiley Interscience,* 1981
- **UN et al.** *Transplantation,* 1997, vol. 63, 1734-1738
- **ELION et al.** *J. Biol. Chem.,* 1954, vol. 208, 477-488
- **BABA et al.** *Antimicrob. Agents Chemother,* 1984, vol. 25, 515-517
- McCutcheon's Detergents and Emulsifiers Annual. Crop., Ridgewood, 1981
- Tensid-Taschenbuch. Hanser Verlag, 1981
- Encyclopaedia of Surfactants. Chemical Publishing Co, 1981
- **LANDAUER et al.** *J. Chem. Soc.,* 1953, 3721-3722
- **FRÖHLICH et al.** *J. Med. Chem.,* 1999, vol. 42, 4108-4121
- **W. PFLEIDERER.** *Chem. Ber.,* 1957, vol. 90, 2272
- **BADDILEY et al.** *J. Chem. Soc.,* 1943, 383
- **TAYLOR et al.** *J. Am. Chem. Soc.,* 1952, vol. 74, 1644-1647
- **BATEY et al.** *Tetrahedron Lett.,* 1998, vol. 39, 6267-6270